# EUROPEAN PATENT APPLICATION

(11) **EP 3 434 778 A1**
(43) Date of publication of application: **30.01.2019**
(21) Application number: 16895258.8
(22) Date of filing: 02.12.2016
(51) Int. Cl.: C12N 15/82, A01G 13/00, A01H 5/00

(54) **APPLICATION OF HERBICIDE-TOLERANT PROTEIN**

(30) Priority: 22.03.2016 CN 201610165061
(71) Applicant: Beijing Dabeinong Technology Group Co., Ltd., Beijing 100080 (CN); Beijing Dabeinong Biotechnology Co.,Ltd, Beijing 100193 (CN)
(72) Inventor: XIE, Xiangting, Beijing 100193 (CN); TAO, Qing, Beijing 100193 (CN); PANG, Jie, Beijing 100193 (CN); DING, Derong, Beijing 100193 (CN); BAO, Xiaoming, Beijing 100193 (CN)
(74) Representative: Comoglio, Elena
(86) International application number: PCT/CN2016/108409
(87) International publication number: WO 2017/161914

(57) **Abstract**

The present invention relates to the use of a herbicide-tolerant protein, wherein the method for controlling weeds comprises applying a herbicide containing an effective dose of tribenuron-methyl to a plant growth environment where at least one transgenic plant is present, wherein the transgenic plant comprises a nucleotide sequence encoding a thifensulfuron hydrolase in its genome, and compared to other plants without the nucleotide sequence encoding the hydrolase, the transgenic plant has reduced plant damage and/or an increased plant yield. The present invention discloses for the first time that a thifensulfuron hydrolase can show a high tolerance to a tribenuron-methyl herbicide, plants containing a nucleotide sequence encoding the thifensulfuron hydrolase are strongly tolerant to the tribenuron-methyl herbicide and can at least tolerate 1-fold field concentration, and thus the hydrolase has broad application prospects in plants.

## Description

### TECHNICAL FIELD

The present invention relates to the use of a herbicide-tolerant protein, in particular to the use of a thifensulfuron hydrolase to degrade a tribenuron-methyl herbicide.

### BACKGROUND ART

Weeds may exhaust valuable nutrients required by crops and other plants of interest in the soil rapidly. Currently, there are many types of herbicides used to control weeds, among which a particularly popular herbicide is glyphosate. Crops resistant to glyphosate have been developed, such as maize, soybean, cotton, sugar beet, wheat, and rice. Therefore, glyphosate can be sprayed onto a field where the glyphosate-resistant crops are planted, so as to control weeds without significant damage to the crops.

Glyphosate has been widely used in the world for more than 20 years, resulting in an over-reliance on glyphosate and glyphosate-tolerant crop technologies, as well as applying a high selection pressure on plants that are naturally more tolerant to glyphosate or have developed a glyphosate-resistant activity in wild weed species. It has been reported that a few weeds have developed resistance to glyphosate, including broad-leaved weeds and gramineous weeds, such as *Lolium rigidium, Lolium multiflorum, Eleusine indica* Gaertn, *Ambrosia artemisiifolia, Conyza canadensis, Conyza bonariensis* and *Plantago lanceolata.* Moreover, weeds that were not agricultural problems before the widespread use of glyphosate-tolerant crops have become prevalent gradually and are difficult to control with glyphosate-tolerant crops, wherein these weeds mainly appear together with (but not only with) broad-leaved weeds that are difficult to control, such as *Amaranthus, Chenopodium,* dandelion and *Commelinaceae* species.

In areas where glyphosate-resistant weeds or weed species that are difficult to control are present, growers can compensate for the weakness of glyphosate by tank mixing or switching to other herbicides that may control omitted weeds, such as sulfonylurea herbicides. Sulfonylurea herbicides have become the third herbicide after organophosphorus and acetamide herbicides with global annual sales of not less than $3 billion, and the annual application area of sulfonylurea herbicides in China has been more than 2 million hectares and still shows an expanding trend.

With the emergence of glyphosate-resistant weeds and the expanding application of sulfonylurea herbicides, there is a need to introduce sulfonylurea herbicide tolerance into plants of interest that are sensitive to sulfonylurea herbicides. Sulfonylurea herbicides can be broadly divided into ester bond-containing ones and ester bond-free ones, and there are at least ten remaining types of sulfonylurea herbicides containing ester bonds and having similar chemical structures. It has only been identified that a thifensulfuron hydrolase can degrade thifensulfuron. However, like thifensulfuron, tribenuron-methyl also belongs to a sulfonylurea herbicide containing an ester bond, and currently there is no report that thifensulfuron hydrolase is tolerant to a tribenuron-methyl herbicide.

### SUMMARY OF THE INVENTION

It is an object of the present invention to provide the use of a herbicide-tolerant protein. Provided for the first time is a method to control field weed growth by applying a herbicide containing an effective dose of tribenuron-methyl to a plant growth environment where at least one transgenic plant expressing a thifensulfuron hydrolase is present, increasing the tolerance range of the thifensulfuron hydrolase to the herbicides.

In order to achieve the object above, the present invention provides a method for controlling weeds, comprising applying a herbicide containing an effective dose of tribenuron-methyl to a plant growth environment where at least one transgenic plant is present, wherein the transgenic plant comprises a nucleotide sequence encoding a thifensulfuron hydrolase in its genome, and compared to other plants without the nucleotide sequence encoding the thifensulfuron hydrolase, the transgenic plant has reduced plant damage and/or an increased plant yield.

Further, the effective dose of tribenuron-methyl is 9-144 g ai/ha.

Furthermore, the transgenic plant is a monocotyledonous plant or a dicotyledonous plant.

Preferably, the transgenic plant is maize, soybean, *Arabidopsis thaliana,* cotton, rape, rice, sorghum, wheat, barley, millet, sugar cane or oat.

On the basis of the above technical solution, the amino acid sequence of the thifensulfuron hydrolase has an amino acid sequence shown as SEQ ID NO: 1, SEQ ID NO: 4 or SEQ ID NO: 7.

Preferably, the nucleotide sequence of the thifensulfuron hydrolase has:
(a) a nucleotide sequence encoding the amino acid sequence shown as SEQ ID NO: 1, SEQ ID NO: 4 or SEQ ID NO: 7; or
(b) a nucleotide sequence shown as SEQ ID NO: 2 or SEQ ID NO: 3; or
(c) a nucleotide sequence shown as SEQ ID NO: 5 or SEQ ID NO: 6; or
(d) a nucleotide sequence shown as SEQ ID NO: 8 or SEQ ID NO: 9.

Further, the transgenic plant may also comprise at least one second nucleotide different from the nucleotide sequence encoding the thifensulfuron hydrolase.

The second nucleotide encodes a selectable marker protein, a protein with a synthetic activity, a protein with a decomposing activity, an anti-biostress protein, an anti-nonbiostress protein, a male sterile protein, a protein affecting a plant yield and/or a protein affecting plant quality.

Specifically, the second nucleotide encodes 5-enolpyruvylshikimate-3-phosphate synthase, glyphosate oxidoreductase, glyphosate-N-acetyltransferase, glyphosate decarboxylase, glufosinate acetyltransferase, α-ketoglutarate-dependent dioxygenase, dicamba monooxygenase, 4-hydroxyphenylpyruvate dioxygenase, acetolactate synthase, cytochrome-like proteins and/or protoporphyrinogen oxidase.

Optionally, the herbicide containing an effective dose of tribenuron-methyl also includes glyphosate herbicides, glufosinate herbicides, auxin herbicides, gramineous herbicides, pre-emergence selective herbicides and/or post-emergence selective herbicides.

In order to achieve the object above, the present invention also provides a method for controlling glyphosate-tolerant weeds, comprising applying an effective dose of a tribenuron-methyl herbicide and a glyphosate herbicide to a field where at least one transgenic plant is planted, wherein the field includes glyphosate-tolerant weeds or seeds thereof, the transgenic plant comprises a nucleotide sequence encoding a thifensulfuron hydrolase and a nucleotide sequence encoding a glyphosate-tolerant protein in its genome, and compared to other plants without the nucleotide sequence encoding the thifensulfuron hydrolase and/or the nucleotide sequence encoding the glyphosate-tolerant protein, the transgenic plant has reduced plant damage and/or an increased plant yield.

Further, the effective dose of tribenuron-methyl is 9-144 g ai/ha. The effective dose of glyphosate is 200-1600 g ae/ha.

Furthermore, the transgenic plant is a monocotyledonous plant or a dicotyledonous plant.

Preferably, the transgenic plant is maize, soybean, *Arabidopsis thaliana,* cotton, rape, rice, sorghum, wheat, barley, millet, sugar cane or oat.

On the basis of the above technical solution, the amino acid sequence of the thifensulfuron hydrolase has an amino acid sequence shown as SEQ ID NO: 1, SEQ ID NO: 4 or SEQ ID NO: 7.

Preferably, the nucleotide sequence of the thifensulfuron hydrolase has:
(a) a nucleotide sequence encoding the amino acid sequence shown as SEQ ID NO: 1, SEQ ID NO: 4 or SEQ ID NO: 7; or
(b) a nucleotide sequence shown as SEQ ID NO: 2 or SEQ ID NO: 3; or
(c) a nucleotide sequence shown as SEQ ID NO: 5 or SEQ ID NO: 6; or
(d) a nucleotide sequence shown as SEQ ID NO: 8 or SEQ ID NO: 9.

Further, the glyphosate-tolerant protein includes 5-enolpyruvylshikimate-3-phosphate synthase, glyphosate oxidoreductase, glyphosate-N-acetyltransferase or glyphosate decarboxylase.

Specifically, the amino acid sequence of the glyphosate-tolerant protein has an amino acid sequence shown as SEQ ID NO: 10.

Preferably, the nucleotide sequence of the glyphosate-tolerant protein has:
(a) a nucleotide sequence encoding the amino acid sequence shown as SEQ ID NO: 10; or
(b) a nucleotide sequence shown as SEQ ID NO: 11.

In order to achieve the object above, the present invention also provides a planting system for controlling weed growth, comprising a tribenuron-methyl herbicide and a plant growth environment where at least one transgenic plant is present, by applying a herbicide containing an effective dose of tribenuron-methyl to the plant growth environment where at least one transgenic plant is present, wherein the transgenic plant comprises a nucleotide sequence encoding a thifensulfuron hydrolase in its genome, and compared to other plants without the nucleotide sequence encoding the thifensulfuron hydrolase, the transgenic plant has reduced plant damage and/or an increased plant yield.

Further, the effective dose of tribenuron-methyl is 9-144 g ai/ha.

Furthermore, the transgenic plant is a monocotyledonous plant or a dicotyledonous plant.

Preferably, the transgenic plant is maize, soybean, *Arabidopsis thaliana,* cotton, rape, rice, sorghum, wheat, barley, millet, sugar cane or oat.

On the basis of the above technical solution, the amino acid sequence of the thifensulfuron hydrolase has an amino acid sequence shown as SEQ ID NO: 1, SEQ ID NO: 4 or SEQ ID NO: 7.

Preferably, the nucleotide sequence of the thifensulfuron hydrolase has:
(a) a nucleotide sequence encoding the amino acid sequence shown as SEQ ID NO: 1, SEQ ID NO: 4 or SEQ ID NO: 7; or
(b) a nucleotide sequence shown as SEQ ID NO: 2 or SEQ ID NO: 3; or
(c) a nucleotide sequence shown as SEQ ID NO: 5 or SEQ ID NO: 6; or
(d) a nucleotide sequence shown as SEQ ID NO: 8 or SEQ ID NO: 9.

Further, the transgenic plant may also comprise at least one second nucleotide different from the nucleotide sequence encoding the thifensulfuron hydrolase.

The second nucleotide encodes a selectable marker protein, a protein with a synthetic activity, a protein with a decomposing activity, an anti-biostress protein, an anti-nonbiostress protein, a male sterile protein, a protein affecting a plant yield and/or a protein affecting plant quality.

Specifically, the second nucleotide encodes 5-enolpyruvylshikimate-3-phosphate synthase, glyphosate oxidoreductase, glyphosate-N-acetyltransferase, glyphosate decarboxylase, glufosinate acetyltransferase, α-ketoglutarate-dependent dioxygenase, 4-hydroxyphenylpyruvate dioxygenase, acetolactate synthase, cytochrome-like proteins and/or protoporphyrinogen oxidase.

Optionally, the herbicide containing a herbicidally effective dose of tribenuron-methyl also includes glyphosate herbicides, glufosinate herbicides, auxin herbicides, gramineous herbicides, pre-emergence selective herbicides and/or post-emergence selective herbicides.

In order to achieve the object above, the present invention also provides a planting system for controlling glyphosate-tolerant weeds, comprising a tribenuron-methyl herbicide, a glyphosate herbicide and a field where at least one transgenic plant is planted, by applying an effective dose of the tribenuron-methyl herbicide and the glyphosate herbicide to the field where at least one transgenic plant is planted, wherein the field includes glyphosate-tolerant weeds or seeds thereof, the transgenic plant comprises a nucleotide sequence encoding a thifensulfuron hydrolase and a nucleotide sequence encoding a glyphosate-tolerant protein in its genome, and compared to other plants without the nucleotide sequence encoding the thifensulfuron hydrolase and/or the nucleotide sequence encoding the glyphosate-tolerant protein, the transgenic plant has reduced plant damage and/or an increased plant yield.

Further, the effective dose of tribenuron-methyl is 9-144 g ai/ha. The effective dose of glyphosate is 200-1600 g ae/ha.

Furthermore, the transgenic plant is a monocotyledonous plant or a dicotyledonous plant.

Preferably, the transgenic plant is maize, soybean, *Arabidopsis thaliana,* cotton, rape, rice, sorghum, wheat, barley, millet, sugar cane or oat.

On the basis of the above technical solution, the amino acid sequence of the thifensulfuron hydrolase has an amino acid sequence shown as SEQ ID NO: 1, SEQ ID NO: 4 or SEQ ID NO: 7.

Preferably, the nucleotide sequence of the thifensulfuron hydrolase has:
(a) a nucleotide sequence encoding the amino acid sequence shown as SEQ ID NO: 1, SEQ ID NO: 4 or SEQ ID NO: 7; or
(b) a nucleotide sequence shown as SEQ ID NO: 2 or SEQ ID NO: 3; or
(c) a nucleotide sequence shown as SEQ ID NO: 5 or SEQ ID NO: 6; or
(d) a nucleotide sequence shown as SEQ ID NO: 8 or SEQ ID NO: 9.

Further, the glyphosate-tolerant protein includes 5-enolpyruvylshikimate-3-phosphate synthase, glyphosate oxidoreductase, glyphosate-N-acetyltransferase or glyphosate decarboxylase.

Specifically, the amino acid sequence of the glyphosate-tolerant protein has an amino acid sequence shown as SEQ ID NO: 10.

Preferably, the nucleotide sequence of the glyphosate-tolerant protein has:
(a) a nucleotide sequence encoding the amino acid sequence shown as SEQ ID NO: 10; or
(b) a nucleotide sequence shown as SEQ ID NO: 11.

In order to achieve the object above, the present invention also provides a method for producing a plant tolerant to a tribenuron-methyl herbicide, comprising introducing a nucleotide sequence encoding a thifensulfuron hydrolase into the genome of a plant, wherein when a herbicide containing an effective dose of tribenuron-methyl is applied to a field where at least the plant is present, the plant has reduced plant damage and/or an increased plant yield compared to other plants without the nucleotide sequence encoding the thifensulfuron hydrolase.

In order to achieve the object above, the present invention also provides a method for cultivating a plant tolerant to a tribenuron-methyl herbicide, comprising:
planting at least one plant propagule, wherein the plant propagule comprises a polynucleotide sequence encoding a thifensulfuron hydrolase in the genome;
growing the plant propagule into a plant;
and applying a herbicide containing an effective dose of tribenuron-methyl to a plant growth environment where at least the plant is included and harvesting the plant having reduced plant damage and/or an increased plant yield compared to other plants without the polynucleotide sequence encoding the thifensulfuron hydrolase.

In order to achieve the object above, the present invention also provides a method for protecting a plant from damage caused by a tribenuron-methyl herbicide, comprising applying a herbicide containing an effective dose of tribenuron-methyl to the plant growth environment where at least one transgenic plant is present, wherein the transgenic plant comprises a nucleotide sequence encoding a thifensulfuron hydrolase in its genome, and compared to other plants without the nucleotide sequence encoding the thifensulfuron hydrolase, the transgenic plant has reduced plant damage and/or an increased plant yield.

In order to achieve the object above, the present invention also provides a method for degrading a tribenuron-methyl herbicide with a thifensulfuron hydrolase, comprising applying a herbicide containing an effective dose of tribenuron-methyl to the plant growth environment where at least one transgenic plant is present, wherein the transgenic plant comprises a nucleotide sequence encoding the thifensulfuron hydrolase in its genome, and compared to other plants without the nucleotide sequence encoding the thifensulfuron hydrolase, the transgenic plant has reduced plant damage and/or an increased plant yield.

In order to achieve the object above, the present invention also provides the use of a thifensulfuron hydrolase to degrade a tribenuron-methyl herbicide.

Specifically, the use of the thifensulfuron hydrolase to degrade a tribenuron-methyl herbicide comprises applying a herbicide containing an effective dose of tribenuron-methyl to the plant growth environment where at least one transgenic plant is present, wherein the transgenic plant comprises a nucleotide sequence encoding the thifensulfuron hydrolase in its genome, and compared to other plants without the nucleotide sequence encoding the thifensulfuron hydrolase, the transgenic plant has reduced plant damage and/or an increased plant yield.

On the basis of the above technical solution, the amino acid sequence of the thifensulfuron hydrolase has an amino acid sequence shown as SEQ ID NO: 1, SEQ ID NO: 4 or SEQ ID NO: 7.

Preferably, the nucleotide sequence of the thifensulfuron hydrolase has:
(a) a nucleotide sequence encoding the amino acid sequence shown as SEQ ID NO: 1, SEQ ID NO: 4 or SEQ ID NO: 7; or
(b) a nucleotide sequence shown as SEQ ID NO: 2 or SEQ ID NO: 3; or
(c) a nucleotide sequence shown as SEQ ID NO: 5 or SEQ ID NO: 6; or
(d) a nucleotide sequence shown as SEQ ID NO: 8 or SEQ ID NO: 9.

The transgenic plant in the present invention is planted in the soil of the plant growth environment within 21 days after applying the herbicide. Optionally, the herbicide can be applied before, simultaneously with or after planting the transgenic plant. Specifically, the transgenic plant is planted in the soil 12, 10, 7 or 3 days before applying the herbicide; or the transgenic plant is planted in the soil 12, 10, 7 or 3 days after applying the herbicide. A second treatment can be further performed on the transgenic plant with the herbicide, wherein the second treatment may be between the V1-V2 stage and the V3-V4 stage, before flowering, at the flowering time, after flowering or at the seeding time.

The tribenuron-methyl in the present invention refers to methyl 2-[N-(4-methoxy-6-methyl-1,3,5-triazin-2-yl)-N-methylaminoformamidosulfonyl]benzoate as a white solid. Commonly used dosage forms are 10% tribenuron-methyl wettable powders and 75% tribenuron-methyl water dispersible granules (also referred to as dried suspension concentrates or dry suspension concentrates). Commercial preparations of tribenuron-methyl include, but are not limited to, Giant Star and Broadleaf Free.

The effective dose of tribenuron-methyl in the present invention refers to a usage amount of 9-144 g ai/ha, including 15-120 g ai/ha, 30-110 g ai/ha, 40-100 g ai/ha, 50-90 g ai/ha, 60-80 g ai/ha or 65-75 g ai/ha.

The dicotyledonous plant in the present invention includes, but is not limited to, alfalfa, bean, cauliflower, cabbage, carrot, celery, cotton, cucumber, eggplant, lettuce, melon, pea, pepper, zucchini, radish, rape, spinach, soybean, pumpkin, tomato, *Arabidopsis thaliana* or watermelon. Preferably, the dicotyledonous plant refers to soybean, *Arabidopsis thaliana,* cotton or rape.

The monocotyledonous plant in the present invention includes, but is not limited to, maize, rice, sorghum, wheat, barley, rye, millet, sugar cane, oat or turfgrass. Preferably, the monocotyledonous plant refers to maize, rice, sorghum, wheat, barley, millet, sugar cane or oat.

In the present invention, the herbicide-tolerant protein is a thifensulfuron hydrolase, such as shown as SEQ ID NO: 1, SEQ ID NO: 4 and SEQ ID NO: 7 in the sequence listing. The herbicide-tolerant gene is a nucleotide sequence encoding the thifensulfuron hydrolase, such as shown as SEQ ID NO: 2, SEQ ID NO: 3, SEQ ID NO: 5, SEQ ID NO: 6, SEQ ID NO: 8 and SEQ ID NO: 9 in the sequence listing. For use in a plant, in addition to the coding region for the thifensulfuron hydrolase, the herbicide-tolerant gene may comprise other elements, e.g., ones encoding a selectable marker protein, a protein with a synthetic activity, a protein with a decomposing activity, an anti-biostress protein, an anti-nonbiostress protein, a male sterile protein, a protein affecting plant yield and/or a protein affecting plant quality, thus obtaining a transgenic plant having a herbicide-tolerant activity and other traits.

The anti-biostress protein in the present invention refers to a protein resistant to stresses imposed by other organisms, such as an insect-resistant protein and a (virus, bacterium, fungus and nematode) disease-resistant protein.

The anti-nonbiostress protein in the present invention refers to a protein resistant to stresses imposed by the external environment, such as proteins tolerant to a herbicide, drought, heat, cold, freezing, salt stress, oxidative stress, etc.

The protein affecting plant quality in the present invention refers to a protein affecting a plant output trait, such as a protein improving the quality and content of starch, oil, vitamins and the like, and a protein improving fiber quality.

In addition, an expression cassette comprising the nucleotide sequence encoding the thifensulfuron hydrolase may further be expressed together with at least one protein encoding a herbicide-tolerant gene in a plant, wherein the herbicide-tolerant gene includes, but is not limited to, 5-enolpyruvylshikimate-3-phosphate synthase (EPSPS), glyphosate oxidoreductase (GOX), glyphosate-N-acetyltransferase (GAT), glyphosate decarboxylase, glufosinate acetyltransferase (PAT), α-ketoglutarate-dependent dioxygenase (AAD), dicamba monooxygenase (DMO), 4-hydroxyphenylpyruvate dioxygenase (HPPD), acetolactate synthase (ALS), cytochrome-like proteins (P450) and/or protoporphyrinogen oxidase (Protox).

The "glyphosate" in the present invention refers to N-phosphonomethylglycine and salts thereof. Treating with a "glyphosate herbicide" refers to performing treatment using any glyphosate-containing herbicide preparation. Commercial preparations of glyphosate include, but are not limited to, ROUNDUP® (as an isopropylamine salt of glyphosate), ROUNDUP®WEATHERMAX (as a potassium salt of glyphosate), ROUNDUP®DRY and RIVAL® (as an amine salt of glyphosate), ROUNDUP®GEOFORCE (as a sodium salt of glyphosate) and TOUCHDOWN® (as a trimethylsulfonium salt of glyphosate).

The effective dose of glyphosate in the present invention refers to a usage amount of 200-1600 g ae/ha, including 250-1600 g ae/ha, 300-1600 g ae/ha, 500-1600 g ae/ha, 800-1500 g ae/ha, 1000-1500 g ae/ha or 1200-1500 g ae/ha.

The "glufosinate" (also known as phosphinothricin) in the present invention refers to ammonium 2-amino-4-[hydroxy(methyl)phosphonyl]butyrate. Treating with a "glufosinate herbicide" refers to performing treatment using any glufosinate-containing herbicide preparation.

The auxin herbicides in the present invention simulate natural plant growth regulators called auxin or act as the regulators, wherein the herbicides affect cell wall plasticity and nucleic acid metabolism, resulting in uncontrolled cell division and growth. Damage symptoms caused by the auxin herbicides include epinastic bending or twisting of stems and petioles, cup-shaped or curled leaves and abnormal leaf shapes and veins. The auxin herbicides include, but are not limited to, phenoxycarboxylic acid compounds, benzoic acid compounds, pyridinecarboxylic acid compounds, quinolinecarboxylic acid compounds or benazolin-ethyl compounds. Typically, the auxin herbicides are dicamba, 2,4-dichlorophenoxy acetic acid (2,4-D), (4-chloro-2-methylphenoxy)acetic acid (MCPA) and/or 4-(2,4-dichlorophenoxy)butyric acid (2,4-DB).

The "dicamba" in the present invention refers to 3,6-dichloro-o-anisic acid or 3,6-dichloro-2-methoxybenzoic acid and acids and salts thereof, in which the salts thereof include isopropylamine salt, diglycolamine salt, dimethylamine salt, potassium salt and sodium salt. Commercial preparations of dicamba include, but are not limited to, Banvel® (as a DMA salt), Clarity® (BASF, as a DGA salt), VEL-58-CS-11™ and Vanquish® (BASF, as a DGA salt).

The gramineous herbicides in the present invention are not used in maize unless maize is already tolerant thereto, and such tolerance may be provided via α-ketoglutarate-dependent dioxygenase (e.g., the AAD gene), wherein the gramineous herbicides include, but are not limited to, fluazifop-p-butyl.

The pre-emergence selective herbicides in the present invention include, but are not limited to, acetanilide, acetochlor, acetolactate synthase inhibitors, dinitroaniline or protoporphyrinogen oxidase inhibitors.

The post-emergence selective herbicides in the present invention include, but are not limited to, nicosulfuron, rimsulfuron, 2,4-D, dicamba, fluoroglycofen-ethyl and quizalofop-p-ethyl.

The application amount of the herbicide in the present invention varies depending on the soil structure, the pH value, the organic content, the tillage system and the weed size, and is determined by viewing the suitable herbicide application amount on a herbicide label.

Weeds that can be controlled by the tribenuron-methyl herbicide in the present invention include, but are not limited to, *Amaranthus retroflexus, Amaranthus lividus, Solanum nigrum, Abutilon theophrasti, Polygonum bungeanum, Polygonum lapathifolium, Polygonum orientale, Fallopia convolvulus, Polygonum nodosum pers, Chenopodium album, Chenopodium serotinum, Polygonum aviculare, Stellaria media, Bidens tripartita, Bidens pilosa, Commelina communis, Chorispora tenella, Myosotis sylvatica, Elsholtzia ciliata, Equisetum arvense, Amethystea caerulea, Descurainia sophia, Descurainia pinnata, Descurainia lebbeck, Matricaria, Lactuca chaetophyllous,* sunflower, *Galeopsis bifida, Galium aparine, Kochia scoparia, Stellaria alsine, Lithospermum arvense, Vaccaria segetalis, Camelina sativa, Erysimum sinuatum, Brassica kaber wheeler, Sinapis alba, Ottelia alismoides, Capsella bursa-pastoris, Thlaspi arvense, Salsola collina, Rorippa globosa, Vicia sativa, Sonchus arvensis,* etc.

Weeds that can be controlled by the glyphosate herbicide in the present invention include, but are not limited to, *Alopecurus myosuroides, Avena fatua, Bromus japonicus, Aponogeton madagascarirnsi, Echinochloa crus-galli, Poa annua, Setaria viridis, Digitaria sanguinalis, Portulaca oleracea, Chenopodium album, Xanthium strumarium, Abutilon theophrasti, Polygonum, Plantago asiatica, Stellaria media, Galium aparine,* sedges, etc.

The planting system in the present invention refers to a combination of a plant and any herbicide tolerance shown thereby and/or an available herbicide treatment in different plant developmental stages, producing a high-yielding and/or damage-reduced plant.

Glyphosate is widely used, as it controls a very broad spectrum of broad-leaved and gramineous weed species. However, reusing glyphosate in glyphosate-tolerant crops and non-crop applications has been (and still continues to be) chosen to make weeds evolve into naturally more tolerant species or glyphosate-resistant biotypes. Most herbicide resistance management strategies suggest using an effective amount of various herbicides as a means of delaying the emergence of resistant weeds, wherein the various herbicides provide control of the same species, but have different modes of action. Superposing the thifensulfuron hydrolase gene with a glyphosate tolerance trait (and/or another herbicide tolerance trait) can achieve control of glyphosate-resistant weed species (broad-leaved weed species controlled by the tribenuron-methyl herbicide) in glyphosate-tolerant crops by allowing for selective use of glyphosate and tribenuron-methyl on the same crop. The application of these herbicides can be performed by using simultaneously in a tank mixture containing two or more herbicides with different modes of action, or using a single herbicide composition alone in continuous use (e.g., before planting, before or after emergence) (with an interval time range used being from 2 hours to 3 months), or alternatively, can be performed by using a combination of any number of herbicides representative of each applicable compound category at any time (from 7 months after planting a crop to the time when the crop is harvested (or the pre-harvest interval for a single herbicide, taking the shortest)).

A herbicide preparation (e.g., an ester, acid or salt-formulated or soluble concentrate, emulsifying concentrate or soluble liquid) and a tank mix additive (e.g., an adjuvant or compatilizer) can significantly affect weed control of a given herbicide or a combination of one or more herbicides. Any chemical combination of any of the foregoing herbicides is within the scope of the present invention.

In the present invention, weeds refer to plants competing with the cultivated plants in the plant growth environment.

The term "control" and/or "prevention" in the present invention refers to at least direct application of (e.g., by spraying) an effective dose of a tribenuron-methyl herbicide to the plant growth environment, so as to minimize weed development and/or stop weeds from growing. At the same time, the cultivated plants should be morphologically normal and can be cultivated under conventional methods for product consumption and/or production; and preferably, compared to non-transgenic wild-type plants, the cultivated plants have reduced plant damage and/or an increased plant yield. Specific performances of reduced plant damage include, but are not limited to, an improved stem resistance and/or an increased grain weight, etc. The "control" and/or "prevention" effect of the thifensulfuron hydrolase on weeds can exist independently, and will not be diminished and/or lost due to the presence of other substances that can "control" and/or "prevent" the weeds. Specifically, if any tissue of a transgenic plant (containing the polynucleotide sequence encoding the thifensulfuron hydrolase) has and/or produces the thifensulfuron hydrolase and/or another substance that can control weeds simultaneously and/or asynchronously, then the presence of the another substance will neither affect the "control" and/or "prevention" effect of the thifensulfuron hydrolase on the weeds, nor result in the "control" and/or "prevention" effect being completely and/or partially achieved by the another substance regardless of the thifensulfuron hydrolase.

In the present invention, expression of the thifensulfuron hydrolase in a transgenic plant can be accompanied by the expression of one or more other herbicide-tolerant proteins. This co-expression of more than one herbicide-tolerant protein in the same transgenic plant can be achieved by allowing the plant to comprise and express a desired gene through genetic engineering. In addition, a plant (a first parent) can express the thifensulfuron hydrolase through genetic engineering manipulation, and a second plant (a second parent) can express other herbicide-tolerant proteins through genetic engineering manipulation. Progeny plants expressing all the genes introduced into the first parent and the second parent are obtained by hybridizing the first parent with the second parent.

The genome of a plant, plant tissue or plant cell in the present invention refers to any genetic material within the plant, plant tissue or plant cell, and includes nuclear, plastid and mitochondrial genomes.

The "plant propagule" in the present invention includes, but is not limited to, plant sexual propagules and plant vegetative propagules. The plant sexual propagules include, but are not limited to, plant seeds; and the plant vegetative propagules refer to vegetative organs or a specific tissue of a plant, which can generate a new plant under *ex vivo* conditions, wherein the vegetative organs or the specific tissue include, but are not limited to, roots, stems and leaves, for example: plants with roots as the vegetative propagules include strawberry, sweet potato and the like; plants with stems as the vegetative propagules include sugar cane, potato (tuber) and the like; and plants with leaves as the vegetative propagules include aloe, begonia and the like.

The "resistance" in the present invention is heritable, and allows a plant to grow and propagate in the case where an effective treatment by a general herbicide is performed on a given plant. As recognized by a person skilled in the art, even if a certain degree of damage of a plant treated with a herbicide is apparent, the plant can still be considered "resistant". The term "tolerant" or "tolerance" in the present invention is more extensive than the term "resistance", and includes "resistance" and an improved ability of a particular plant to resist various degrees of damage induced by a herbicide, and generally damage to a wild-type plant with the same genotype can be caused at the same herbicide dose.

The polynucleotide and/or nucleotide in the present invention forms a complete "gene", which encodes a protein or a polypeptide in a desired host cell. A person skilled in the art will readily appreciate that the polynucleotide and/or nucleotide in the present invention can be placed under the control of a regulatory sequence in a host of interest.

As well known to a person skilled in the art, DNA is typically present in a double-stranded form. In this arrangement, one strand is complementary to the other, and vice versa. The other complementary strand of DNA is produced since DNA is replicated in a plant. As such, the present invention includes the use of the polynucleotides and complementary strands thereof exemplified in the sequence listing. "Coding strand" commonly used in the art refers to a strand bound to an anti-sense strand. In order to express a protein *in vivo,* one strand of DNA is typically transcribed to one mRNA complementary strand, which acts as a template to translate the protein. Actually, mRNA is transcribed from the "anti-sense" strand of DNA. The "sense" or "coding" strand has a series of codons (a codon is composed of three nucleotides, and a specific amino acid can be produced by reading three codons at a time), which can be read as an open reading frame (ORF) to form a protein or peptide of interest. The present invention also includes RNA with an equivalent function to the exemplary DNA.

The nucleic acid molecule or a fragment thereof in the present invention hybridizes with the herbicide-tolerant gene of the present invention under stringent conditions. Any conventional nucleic acid hybridization or amplification method can be used to identify the presence of the herbicide-tolerant gene of the present invention. A nucleic acid molecule or a fragment thereof is capable of specifically hybridizing with other nucleic acid molecules under certain circumstances. In the present invention, if two nucleic acid molecules can form an anti-parallel double-stranded nucleic acid structure, then it can be considered that these two nucleic acid molecules can be specifically hybridized with each other. If two nucleic acid molecules exhibit a complete complementarity, then one nucleic acid molecule of the two is said to be the "complement" of the other nucleic acid molecule. In the present invention, when each nucleotide of a nucleic acid molecule is complementary to the corresponding nucleotide of another nucleic acid molecule, then these two nucleic acid molecules are said to exhibit a "complete complementarity". If two nucleic acid molecules can be hybridized with each other with a sufficient stability to allow them to anneal and bind with each other at least under conventional "low stringency" conditions, then these two nucleic acid molecules are said to be "minimally complementary". Similarly, if two nucleic acid molecules can be hybridized with each other with a sufficient stability to allow them to anneal and bind with each other under conventional "high stringency" conditions, then these two nucleic acid molecules are said to be "complementary". Deviation from a complete complementarity is permissible, as long as this deviation does not completely prevent two molecules from forming a double-stranded structure. In order to enable a nucleic acid molecule to act as a primer or probe, it is only guaranteed that the molecule has a sufficient complementarity in its sequence to allow a stable double-stranded structure to be formed at the particular solvent and salt concentration employed.

In the present invention, a substantially homologous sequence is a segment of a nucleic acid molecule, wherein the nucleic acid molecule can be specifically hybridized with the complementary strand of another segment of a matched nucleic acid molecule under high stringency conditions. Suitable stringent conditions that promote DNA hybridization are for example treating with 6.0× sodium chloride/sodium citrate (SSC) under the condition of approximately 45°C, and then washing with 2.0× SSC under the condition of 50°C, which conditions are well known to a person skilled in the art. For example, the salt concentration in the washing step can be selected from the low stringency condition of about 2.0× SSC, 50°C to the high stringency condition of about 0.2× SSC, 50°C. In addition, the temperature condition in the washing step can rise from the low stringency condition of room temperature (about 22°C) to the high stringency condition of about 65°C. The temperature condition and the salt concentration can both vary, and it is also possible that one of the two remains unchanged while the other variable varies. Preferably, the stringent conditions in the present invention can be specifically hybridizing a sequence with the nucleotide sequence of the thifensulfuron hydrolase in the present invention in a 6× SSC, 0.5% SDS solution at 65°C, and then washing the membrane once with 2× SSC, 0.1% SDS and 1× SSC, 0.1% SDS respectively.

Consequently, sequences which have herbicide tolerance activity and are hybridized with the nucleotide sequence of the thifensulfuron hydrolase in the present invention under stringent conditions are included in the present invention. These sequences are at least approximately 40%-50% homologous, approximately 60%, 65% or 70% homologous to the sequence of the present invention, and even have a sequence homology of at least approximately 75%, 80%, 85%, 90%, 91%, 92%, 93%, 94%, 95%, 96%, 97%, 98%, 99% or more with the sequence of the present invention.

The present invention provides a functional protein. In the present invention, "functional activity" (or "activity") means that the protein/enzyme used in the present invention (alone or in combination with other proteins) has the ability to degrade a herbicide or diminish the herbicide activity. A plant producing the protein of the present invention preferably produces an "effective amount" of the protein, so that when treating the plant with a herbicide, the protein expression level is sufficient to confer the plant a complete or partial resistance or tolerance to the herbicide (unless otherwise specified, a general amount). The herbicide can be used in an amount usually killing a target plant or a normal field amount and concentration. Preferably, the plant cell and plant of the present invention are protected from growth inhibition or damage caused by treatment with the herbicide. The transformed plant and plant cell of the present invention are preferably tolerant or resistant to a tribenuron-methyl herbicide, that is, the transformed plant and plant cell are able to grow in the presence of an effective amount of the tribenuron-methyl herbicide.

The gene and protein in the present invention not only comprise a specific exemplary sequence, but also comprise a portion and/or a fragment (including an internal deletion and/or terminal deletion compared to the full-length protein), a variant, a mutant, a substitute (a protein having substituted amino acids), a chimera and a fusion protein which retain the herbicide tolerance activity characteristic of the specific exemplary protein. The "variant" or "variation" refers to a nucleotide sequence that encodes the same protein or encodes an equivalent protein having a herbicide resistance activity. The "equivalent protein" refers to a protein having the same or substantially the same herbicide tolerance bioactivity as the claimed protein.

The "fragment" or "truncation" of a DNA molecule or protein sequence in the present invention refers to a portion of the original DNA or protein sequence (nucleotides or amino acids) involved or an artificially modified form thereof (e.g., a sequence suitable for plant expression), wherein the length of the foregoing sequences may vary, but the length is sufficient to ensure that the (encoded) protein is a herbicide-tolerant protein.

Owing to the degeneracy of the genetic codon, a variety of different DNA sequences may encode the same amino acid sequence. It is within the skill of a person skilled in the art to produce these alternative DNA sequences encoding the same or substantially the same protein. These different DNA sequences are included in the scope of the present invention. A "substantially the same" sequence refers to a sequence with an amino acid substitution, deletion, addition or insertion that does not substantively affect the herbicide tolerance activity, wherein a fragment retaining the herbicide tolerance activity is also included.

The substitution, deletion or addition of an amino acid sequence in the present invention is a conventional technique in the art, and preferably, this amino acid change is: a small characteristic change, that is a conservative amino acid substitution that does not significantly affect the folding and/or activity of a protein; a small deletion, typically a deletion of about 1-30 amino acids; a small amino or carboxyl terminal extension, e.g., a methionine residue extending at the amino terminus; or a small linker peptide, e.g., about 20-25 residues in length.

Examples of conservative substitutions are substitutions occurring within the following amino acid groups: basic amino acids (e.g., arginine, lysine and histidine), acidic amino acids (e.g., glutamic acid and aspartic acid), polar amino acids (e.g., glutamine and asparagine), hydrophobic amino acids (e.g., leucine, isoleucine and valine), aromatic amino acids (e.g., phenylalanine, tryptophan and tyrosine) and small molecule amino acids (e.g., glycine, alanine, serine, threonine and methionine). Those amino acid substitutions that generally do not alter the specific activity are well known in the art, and have been described, for example, by N. Neurath and R. L. Hill in Protein published by Academic Press, New York, 1979. The most common substitutions are Ala/Ser, Val/Ile, Asp/Glu, Thu/Ser, Ala/Thr, Ser/Asn, Ala/Val, Ser/Gly, Tyr/Phe, Ala/Pro, Lys/Arg, Asp/Asn, Leu/Ile, Leu/Val, Ala/Glu and Asp/Gly, as well as reverse substitutions thereof.

As will be apparent to a person skilled in the art, this substitution can occur outside the region that is important for molecular function, and still produces an active polypeptide. Amino acid residues that are essential for the activity of the polypeptide of the present invention and thus are chosen not to be substituted can be identified according to methods known in the art, such as site-directed mutagenesis or alanine-scanning mutagenesis (see for reference, Cunningham and Wells, 1989, Science 244: 1081-1085). The latter technique is to introduce a mutation to each positively charged residue in a molecule and detect the herbicide resistance activity of the resulting mutant molecule to determine amino acid residues that are important for the molecular activity. Substrate-enzyme interaction sites can also be determined by analyzing the three-dimensional structure thereof, wherein this three-dimensional structure can be determined by nuclear magnetic resonance analysis, crystallography, photoaffinity labelling and other techniques (see, e.g., de Vos et al., 1992, Science 255: 306-312; Smith et al., 1992, J. Mol. Biol 224:899-904; and Wlodaver et al., 1992, FEBS Letters 309: 59-64).

In the present invention, the amino acid sequence encoding the thifensulfuron hydrolase includes, but is not limited to, the sequences involved in the sequence listing of the present invention, and amino acid sequences with a certain degree of homology thereto are also included in the present invention. The similarity/identity of these sequences to the sequence of the present invention is typically greater than 60%, preferably greater than 75%, more preferably greater than 80%, even more preferably greater than 90%, and may be greater than 95%. Preferred polynucleotides and proteins of the present invention can also be defined according to a more specific range of identity and/or similarity. For example, these sequences have an identity and/or similarity of 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% to the exemplary sequence of the present invention.

The regulatory sequence in the present invention includes, but is not limited to, a promoter, a transit peptide, a terminator, an enhancer, a leader sequence, an intron and other regulatory sequences operably linked to the thifensulfuron hydrolase gene.

The promoter is a plant expressible promoter. The "plant expressible promoter" refers to a promoter that ensures the expression of the coding sequence linked thereto in a plant cell. The plant expressible promoter can be a constitutive promoter. Examples of the promoters directing the constitutive expression in plants include, but are not limited to, 35S promoter derived from cauliflower mosaic virus, maize Ubi promoters, rice GOS2 gene promoters, and the like. Alternatively, the plant expressible promoter can be a tissue specific promoter, i.e. the promoter directs the expression of a coding sequence in several tissues such as green tissues at a level higher than in other tissues of the plant (which can be measured through conventional RNA trials), such as a PEP carboxylase promoter. Alternatively, the plant expressible promoter can be a wound-inducible promoter. The wound-inducible promoter or a promoter directing a wound-induced expression pattern means that when a plant suffers from wound caused by a mechanical factor or gnawing of insects, the expression of the coding sequence under the regulation of the promoter is significantly improved compared with under normal growth conditions. Examples of the wound-inducible promoters include, but are not limited to, promoters of potato and tomato protease inhibitor genes (pinI and pinII) and maize protease inhibitor gene (MPI).

The transit peptide (also known as secretion signal sequence or targeting sequence) directs a transgenic product to a specific organelle or cell compartment. For a receptor protein, the transit peptide may be heterologous, for example, targeting the chloroplast using a sequence encoding the chloroplast transit peptide, or targeting the endoplasmic reticulum using a 'KDEL' retention sequence, or targeting the vacuole using CTPP of the barley phytolectin gene.

The leader sequence includes, but is not limited to, a small RNA virus leader sequence, such as EMCV leader sequence (5' non-coding region of encephlomyocarditis virus); a potato virus Y group leader sequence, such as MDMV (Maize Dwarf Mosaic Virus) leader sequence; human immunoglobulin heavy chain binding protein (BiP); an untranslated leader sequence of the coat protein mRNA of alfalfa mosaic virus (AMV RNA4); and a tobacco mosaic virus (TMV) leader sequence.

The enhancer includes, but is not limited to, cauliflower mosaic virus (CaMV) enhancer, figwort mosaic virus (FMV) enhancer, carnation etched ring virus (CERV) enhancer, cassava vein mosaic virus (CsVMV) enhancer, mirabilis mosaic virus (MMV) enhancer, cestrum yellow leaf curling virus (CmYLCV) enhancer, cotton leaf curl Multan virus (CLCuMV) enhancer, commelina yellow mottle virus (CoYMV) enhancer and peanut chlorotic streak caulimovirus (PCLSV) enhancer.

For use in a monocotyledonous plant, the intron includes, but is not limited to, maize hsp70 intron, maize ubiquitin intron, Adh intron 1, sucrose synthase intron or rice Act1 intron. For use in a dicotyledonous plant, the intron includes, but is not limited to, CAT-1 intron, pKANNIBAL intron, PIV2 intron and "super ubiquitin" intron.

The terminator can be a suitable polyadenylation signal sequence that functions in a plant, including, but not limited to, a polyadenylation signal sequence derived from the *Agrobacterium tumefaciens* nopaline synthetase (NOS) gene, a polyadenylation signal sequence derived from the protease inhibitor II (pinII) gene, a polyadenylation signal sequence derived from the pea ssRUBISCO E9 gene and a polyadenylation signal sequence derived from the α-tubulin gene.

The "effectively linking" in the present invention indicates binding of a nucleic acid sequence, wherein the binding enables a sequence to provide a function required for the linked sequence. The "effectively linking" in the present invention can link a promoter to a sequence of interest, so that the transcription of the sequence of interest is controlled and regulated by the promoter. When a sequence of interest encodes a protein and the expression of the protein is desired, "effectively linking" means that: a promoter is linked to the sequence in such a manner that the resulting transcript is efficiently translated. If the linking of a promoter to a coding sequence is transcript fusion and expression of the encoded protein is intended to be achieved, such linking is created that the first translation initiation codon in the resulting transcript is the initiation codon in the coding sequence. Alternatively, if the linking of a promoter to a coding sequence is translation fusion and expression of the encoded protein is intended to be achieved, such a linking is created that the first translation initiation codon contained in the 5' untranslated sequence is linked to the promoter in such a manner that the relationship of the resulting translation product with the translation open reading frame encoding the desired protein is in-frame. Nucleic acid sequences that can be "effectively linked" include, but are not limited to: sequences providing gene expression functions (i.e., gene expression elements, such as promoters, 5' untranslated regions, introns, protein coding regions, 3' untranslated regions, polyadenylation sites and/or transcription terminators), sequences providing DNA transfer and/or integration functions (i.e., T-DNA boundary sequences, site-specific recombinase recognition sites and integrase recognition sites), sequences providing selective functions (i.e., antibiotic resistance markers and biosynthesis genes), sequences providing marker scoring functions, sequences assisting in sequence manipulation *in vitro* or *in vivo* (i.e., polylinker sequences and site-specific recombination sequences) and sequences providing replication functions (i.e., bacterial origins of replication, autonomously replicating sequences and centromeric sequences).

The present invention may confer a new herbicide resistance trait to a plant, and no adverse effects on phenotypes, including yields, are observed. The plant in the present invention can tolerate, for example, 2×, 3×, 4× or 5× the general application level of at least one herbicide tested. The improvement of these levels of tolerance is within the scope of the present invention. For example, foreseeable optimization and further development can be performed on various techniques known in the art, to increase the expression of a given gene.

The thifensulfuron hydrolase in the present invention is tolerant to a tribenuron-methyl herbicide. The plant in the present invention contains an exogenous DNA in its genome, wherein the exogenous DNA comprises a nucleotide sequence encoding the thifensulfuron hydrolase, and the plant is protected from the threat of a herbicide by expressing an effective amount of the protein. The effective amount refers to a dose causing no or minor damage. At the same time, the plant should be morphologically normal and can be cultivated under conventional methods for product consumption and/or production.

The expression level of the herbicide-tolerant protein in a plant material can be detected by a variety of methods described in the art, for example, quantifying the mRNA encoding the herbicide-tolerant protein produced in a tissue by applying specific primers, or specifically detecting the amount of herbicide-tolerant protein produced directly.

In the present invention, an exogenous DNA is introduced into a plant, for example introducing a gene or expression cassette or recombinant vector encoding the thifensulfuron hydrolase into a plant cell. Conventional transformation methods include, but are not limited to, *Agrobacterium-mediated* transformation, microprojectile bombardment, directly uptaking DNA into the protoplast, electroporation or silicon whisker-mediated DNA introduction.

The present invention provides the use of a herbicide-tolerant protein, having the following advantages:
1. Having a broad herbicide tolerance. The present invention discloses for the first time that a thifensulfuron hydrolase can show a high tolerance to a tribenuron-methyl herbicide, thus having broad application prospects in plants.
2. Having a strong herbicide tolerance. The thifensulfuron hydrolase of the present invention is strongly tolerant to a tribenuron-methyl herbicide and can at least tolerate 1-fold field concentration.

The technical solution of the present invention is further described in detail through drawings and examples below.

### DESCRIPTION OF THE DRAWINGS

Figure 1 is a construction flow chart of a recombinant cloning vector DBN01-T containing an ALT nucleotide sequence for the use of the herbicide-tolerant protein of the present invention;
Figure 2 is a construction flow chart of a recombinant expression vector DBN100632 containing an ALT nucleotide sequence for the use of the herbicide-tolerant protein of the present invention;
Figure 3 is a schematic structural diagram of a recombinant expression vector DBN100631 containing an ALT nucleotide sequence for the use of the herbicide-tolerant protein of the present invention;
Figure 4 is an effect diagram of the tolerance of a transgenic *Arabidopsis thaliana* T₁ plant to a tribenuron-methyl herbicide for the use of the herbicide-tolerant protein of the present invention;
Figure 5 is a construction flow chart of a recombinant expression vector DBN100828 containing an ALT nucleotide sequence for the use of the herbicide-tolerant protein of the present invention;
Figure 6 is a schematic structural diagram of a recombinant expression vector DBN100827 containing an ALT nucleotide sequence for the use of the herbicide-tolerant protein of the present invention;
Figure 7 is a construction flow chart of a recombinant cloning vector DBN05-T containing an ALT nucleotide sequence for the use of the herbicide-tolerant protein of the present invention;
Figure 8 is a construction flow chart of a recombinant expression vector DBN100830 containing an ALT nucleotide sequence for the use of the herbicide-tolerant protein of the present invention;
Figure 9 is a schematic structural diagram of a recombinant expression vector DBN100829 containing an ALT nucleotide sequence for the use of the herbicide-tolerant protein of the present invention;
Figure 10 is an effect diagram of the tolerance of a transgenic maize T₁ plant to a tribenuron-methyl herbicide for the use of the herbicide-tolerant protein of the present invention;
and Figure 11 is an effect diagram of the tolerance of a transgenic soybean T₁ plant to a tribenuron-methyl herbicide for the use of the herbicide-tolerant protein of the present invention.

### PARTICULAR EMBODIMENTS

The technical solution of the use of the herbicide-tolerant protein of the present invention is further described through specific examples below.

### Example 1. Acquisition and synthesis of an ALT gene sequence

### 1. Acquisition of an ALT gene sequence

The amino acid sequence (398 amino acids) of thifensulfuron hydrolase-1 (ALT-1) is shown as SEQ ID NO: 1 in the sequence listing; the ALT-1-01 nucleotide sequence (1197 nucleotides) encoding the corresponding ALT-1 amino acid sequence is shown as SEQ ID NO: 2 in the sequence listing; and the ALT-1-02 nucleotide sequence (1197 nucleotides) encoding the corresponding ALT-1 amino acid sequence is shown as SEQ ID NO: 3 in the sequence listing.

The amino acid sequence (369 amino acids) of thifensulfuron hydrolase-2 (ALT-2) is shown as SEQ ID NO: 4 in the sequence listing; the ALT-2-01 nucleotide sequence (1110 nucleotides) encoding the corresponding ALT-2 amino acid sequence is shown as SEQ ID NO: 5 in the sequence listing; and the ALT-2-02 nucleotide sequence (1110 nucleotides) encoding the corresponding ALT-2 amino acid sequence is shown as SEQ ID NO: 6 in the sequence listing.

The amino acid sequence (362 amino acids) of thifensulfuron hydrolase-3 (ALT-3) is shown as SEQ ID NO: 7 in the sequence listing; the ALT-3-01 nucleotide sequence (1089 nucleotides) encoding the corresponding ALT-3 amino acid sequence is shown as SEQ ID NO: 8 in the sequence listing; and the ALT-3-02 nucleotide sequence (1089 nucleotides) encoding the corresponding ALT-3 amino acid sequence is shown as SEQ ID NO: 9 in the sequence listing.

### 2. Acquisition of an EPSPS gene sequence

The amino acid sequence (455 amino acids) of a glyphosate-tolerant protein is shown as SEQ ID NO: 10 in the sequence listing; and the EPSPS nucleotide sequence (1368 nucleotides) encoding the amino acid sequence of the corresponding glyphosate-tolerant protein is shown as SEQ ID NO: 11 in the sequence listing.

### 3. Synthesis of the above-mentioned nucleotide sequences

The ALT-1-01 nucleotide sequence (shown as SEQ ID NO: 2 in the sequence listing), the ALT-1-02 nucleotide sequence (shown as SEQ ID NO: 3 in the sequence listing), the ALT-2-01 nucleotide sequence (shown as SEQ ID NO: 5 in the sequence listing), the ALT-2-02 nucleotide sequence (shown as SEQ ID NO: 6 in the sequence listing), the ALT-3-01 nucleotide sequence (shown as SEQ ID NO: 8 in the sequence listing), the ALT-3-02 nucleotide sequence (shown as SEQ ID NO: 9 in the sequence listing) and the EPSPS nucleotide sequence (shown as SEQ ID NO: 11 in the sequence listing) were synthesized by Nanjing Genscript Biotechnology Co., Ltd.; the synthetic ALT-1-01 nucleotide sequence (SEQ ID NO: 2) is further connected with a *SpeI* restriction site at the 5' end, and the ALT-1-01 nucleotide sequence (SEQ ID NO: 2) is further connected with a *Kas*I restriction site at the 3' end; the synthetic ALT-1-02 nucleotide sequence (SEQ ID NO: 3) is further connected with a *Spe*I restriction site at the 5' end, and the ALT-1-02 nucleotide sequence (SEQ ID NO: 3) is further connected with a *Kas*I restriction site at the 3' end; the synthetic ALT-2-01 nucleotide sequence (SEQ ID NO: 5) is further connected with a *Spe*I restriction site at the 5' end, and the ALT-2-01 nucleotide sequence (SEQ ID NO: 5) is further connected with a *Kas*I restriction site at the 3' end; the synthetic ALT-2-02 nucleotide sequence (SEQ ID NO: 6) is further connected with a *Spe*I restriction site at the 5' end, and the ALT-2-02 nucleotide sequence (SEQ ID NO: 6) is further connected with a *Kas*I restriction site at the 3' end; the synthetic ALT-3-01 nucleotide sequence (SEQ ID NO: 8) is further connected with a *Spe*I restriction site at the 5' end, and the ALT-3-01 nucleotide sequence (SEQ ID NO: 8) is further connected with a *Kas*I restriction site at the 3' end; the synthetic ALT-3-02 nucleotide sequence (SEQ ID NO: 9) is further connected with a *Spe*I restriction site at the 5' end, and the ALT-3-02 nucleotide sequence (SEQ ID NO: 9) is further connected with a *Kas*I restriction site at the 3' end; and the synthetic EPSPS nucleotide sequence (SEQ ID NO: 11) is further connected with a *Nco*I restriction site at the 5' end, and the EPSPS nucleotide sequence (SEQ ID NO: 11) is further connected with a *Fsp*I restriction site at the 3' end.

### Example 2. Construction of Arabidopsis thaliana recombinant expression vectors

### 1. Construction of Arabidopsis thaliana and soybean recombinant cloning vectors containing ALT nucleotide sequences

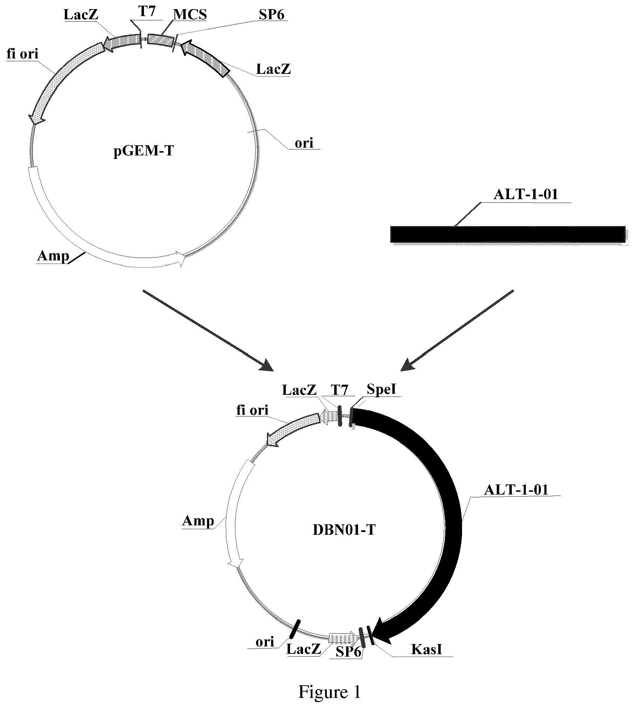

The synthetic ALT-1-01 nucleotide sequence was ligated into cloning vector pGEM-T (Promega, Madison, USA, CAT: A3600), and the operational procedure was carried out according to Promega's pGEM-T vector product instructions, obtaining a recombinant cloning vector DBN01-T, the construction process of which is as shown in Figure 1 (wherein Amp means the ampicillin resistance gene; f1 means the origin of replication of phage f1; LacZ is LacZ initiation codon; SP6 is SP6 RNA polymerase promoter; T7 is T7 RNA polymerase promoter; ALT-1-01 is the ALT-1-01 nucleotide sequence (SEQ ID NO: 2); and MCS is a multiple cloning site).

Then, *Escherichia coli* T1 competent cells (Transgen, Beijing, China, CAT: CD501) were transformed with the recombinant cloning vector DBN01-T using the heat shock method with the following heat shock conditions: water bathing 50 µL *Escherichia coli* T1 competent cells and 10 µL plasmid DNA (recombinant cloning vector DBN01-T) at 42°C for 30 seconds; shake culturing at 37°C for 1 hour (using a shaker at a rotation speed of 100 rpm for shaking); and growing on an LB plate (10 g/L of tryptone, 5 g/L of yeast extract, 10 g/L of NaCl, and 15 g/L of agar, adjusting the pH to 7.5 with NaOH) of ampicillin (100 mg/L) having its surface coated with IPTG (isopropylthio-β-D-galactoside) and X-gal (5-bromo-4-chloro-3-indole-β-D-galactoside) overnight. White colonies were picked out and cultured in an LB liquid culture medium (10 g/L of tryptone, 5 g/L of yeast extract, 10 g/L of NaCl, and 100 mg/L of ampicillin, adjusting the pH to 7.5 with NaOH) at a temperature of 37°C overnight. The plasmids in the cells were extracted through an alkaline method: centrifuging the bacteria solution at a rotation speed of 12000 rpm for 1 min, removing the supernatant, and suspending the precipitated thalli with 100 µL ice pre-cooled solution I (25 mM Tris-HCl, 10 mM EDTA (ethylenediaminetetraacetic acid), and 50 mM glucose, pH 8.0); adding 200 µL newly formulated solution II (0.2M NaOH, 1% SDS (sodium dodecyl sulfate)), inverting the tube 4 times, mixing and placing on ice for 3-5 min; adding 150 µL ice-cold solution III (3 M potassium acetate, 5 M acetic acid), mixing uniformly immediately and placing on ice for 5-10 min; centrifuging under the conditions of a temperature of 4°C and a rotation speed of 12000 rpm for 5 min, adding 2 volumes of anhydrous ethanol to the supernatant and placing at room temperature for 5 min after mixing uniformly; centrifuging under the conditions of a temperature of 4°C and a rotation speed of 12000 rpm for 5 min, discarding the supernatant, and air drying the precipitate after washing with ethanol with a concentration of 70% (V/V); adding 30 µL TE (10 mM Tris-HCl, and 1 mM EDTA, pH 8.0) containing RNase (20 µg/mL) to dissolve the precipitate; water bathing at a temperature of 37°C for 30 min to digest RNA; and storing at a temperature of -20°C for use.

After identifying the extracted plasmid by *SpeI* and *Kas*I digestion, positive clones were verified by sequencing. The results showed that the inserted ALT-1-01 nucleotide sequence in the recombinant cloning vector DBN01-T was the nucleotide sequence shown as SEQ ID NO: 2 in the sequence listing, that is, the ALT-1-01 nucleotide sequence was inserted correctly.

According to the above-mentioned method for constructing the recombinant cloning vector DBN01-T, the synthetic ALT-2-01 nucleotide sequence was ligated into a cloning vector pGEM-T, obtaining a recombinant cloning vector DBN02-T, wherein ALT-2-01 is the ALT-2-01 nucleotide sequence (SEQ ID NO: 5). Enzyme digestion and sequencing verified that the ALT-2-01 nucleotide sequence was correctly inserted into the recombinant cloning vector DBN02-T.

According to the above-mentioned method for constructing the recombinant cloning vector DBN01-T, the synthetic ALT-3-01 nucleotide sequence was ligated into a cloning vector pGEM-T, obtaining a recombinant cloning vector DBN03-T, wherein ALT-3-01 is the ALT-3-01 nucleotide sequence (SEQ ID NO: 8). Enzyme digestion and sequencing verified that the ALT-3-01 nucleotide sequence was correctly inserted into the recombinant cloning vector DBN03-T.

At the same time, according to the above-mentioned method for constructing the recombinant cloning vector DBN01-T, the synthetic EPSPS nucleotide sequence was ligated into a cloning vector pGEM-T, obtaining a recombinant cloning vector DBN04-T, wherein EPSPS is the EPSPS nucleotide sequence (SEQ ID NO: 11). Enzyme digestion and sequencing verified that the EPSPS nucleotide sequence was correctly inserted into the recombinant cloning vector DBN04-T.

### 2. Construction of Arabidopsis thaliana recombinant expression vectors containing ALT nucleotide sequences

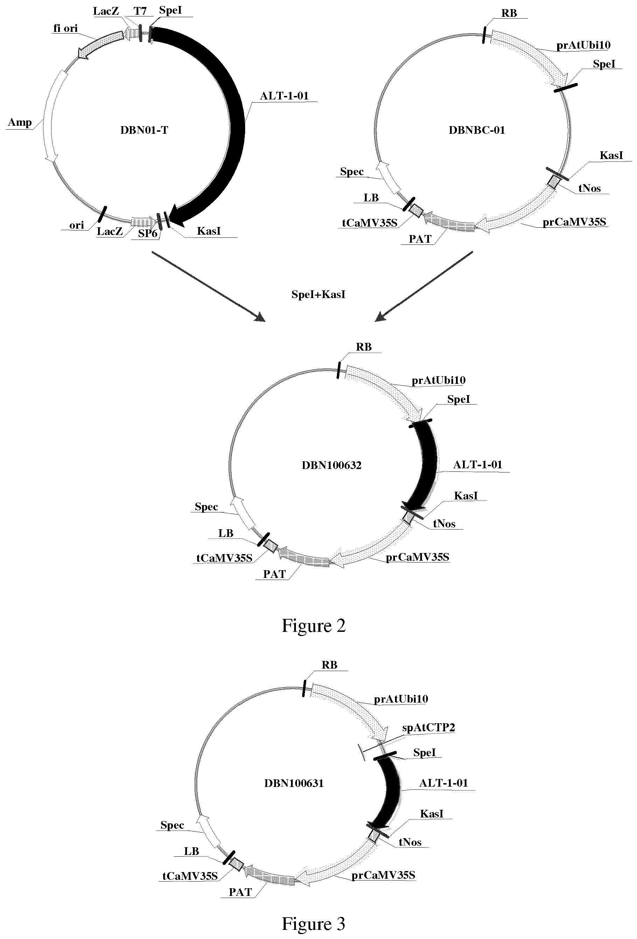

The recombinant cloning vector DBN01-T and an expression vector DBNBC-01 (vector backbone: pCAMBIA2301 (which can be provided by the CAMBIA institution)) were digested with restriction enzymes *Spe*I and *Kas*I*,* respectively; the excised ALT-1-01 nucleotide sequence fragment was inserted between the *Spe*I and *Kas*I sites in the expression vector DBNBC-01; and it is well known to a person skilled in the art to construct a vector using conventional enzyme digestion methods, a recombinant expression vector DBN100632 was constructed (located in the cytoplasm), and the construction process of which was shown as Figure 2 (Spec: the spectinomycin gene; RB: the right boundary; prAtUbi10: the *Arabidopsis thaliana* Ubiquitin 10 gene promoter (SEQ ID NO: 12); ALT-1-01: the ALT-1-01 nucleotide sequence (SEQ ID NO: 2); tNos: the terminator of nopaline synthase gene (SEQ ID NO:13); prCaMV35S: the cauliflower mosaic virus 35S promoter (SEQ ID NO: 14); PAT: the glufosinate acetyltransferase gene (SEQ ID NO: 15); tCaMV35S: the cauliflower mosaic virus 35S terminator (SEQ ID NO: 16); LB: the left boundary).

*Escherichia coli* T1 competent cells were transformed with the recombinant expression vector DBN100632 by a heat shock method with the following heat shock conditions: water bathing 50 µL *Escherichia coli* T1 competent cells and 10 µL plasmid DNA (recombinant expression vector DBN100632) at 42°C for 30 seconds; shake culturing at 37°C for 1 hour (using a shaker at a rotation speed of 100 rpm for shaking); then culturing under the condition of a temperature of 37°C on an LB solid plate containing 50 mg/L of spectinomycin (10 g/L of tryptone, 5 g/L of yeast extract, 10 g/L of NaCl, and 15 g/L of agar, adjusted to a pH of 7.5 with NaOH) for 12 hours, picking white colonies, and culturing under the condition of a temperature of 37°C overnight in an LB liquid culture medium (10 g/L of tryptone, 5 g/L of yeast extract, 10 g/L of NaCl, and 50 mg/L of spectinomycin, adjusted to a pH of 7.5 with NaOH). The plasmids in the cells were extracted through an alkaline method. The extracted plasmid was identified after digesting with restriction enzymes *SpeI* and *Kas*I*,* and positive clones were identified by sequencing. The results showed that the nucleotide sequence between the *SpeI* and *Kas*I sites in the recombinant expression vector DBN100632 was the nucleotide sequence shown as SEQ ID NO: 2 in the sequence listing, i.e., the ALT-1-01 nucleotide sequence.

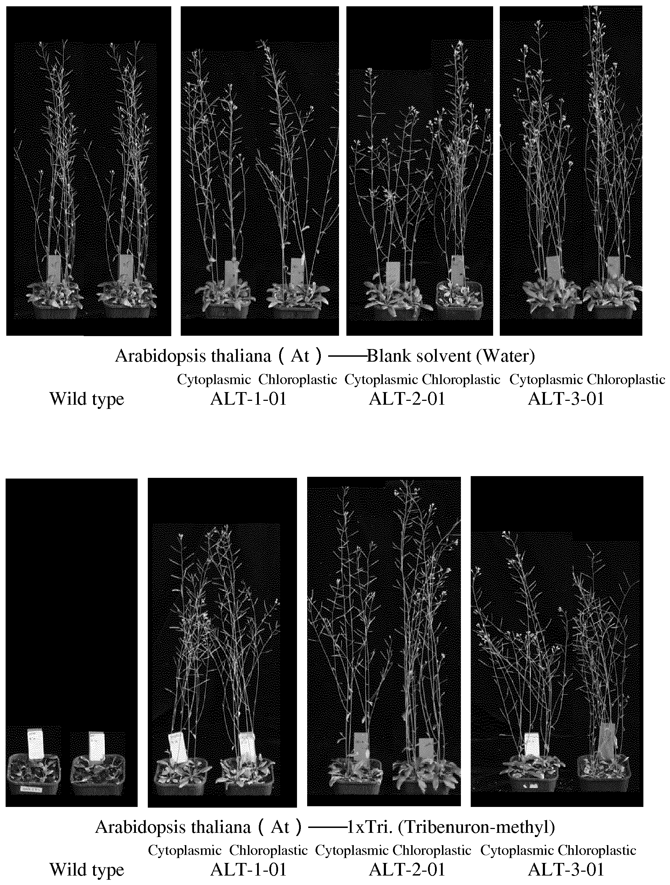

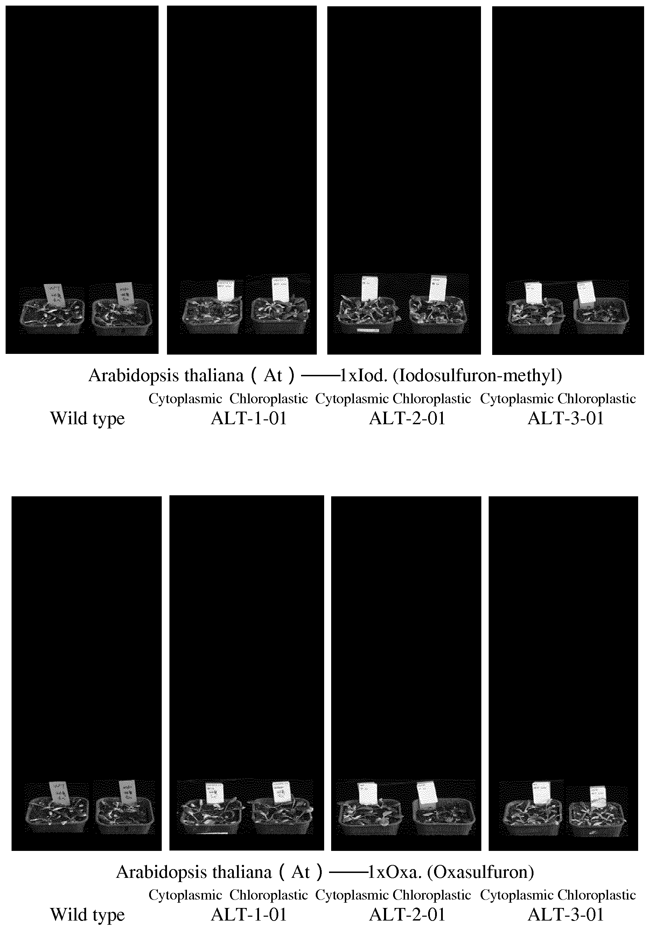

According to the above-mentioned method for constructing the recombinant expression vector DBN100632, a recombinant expression vector DBN100631 (located in the chloroplast) containing the ALT-1-01 nucleotide sequence was constructed, the vector structure of which was shown as Figure 3 (vector backbone: pCAMBIA2301 (which can be provided by the CAMBIA institution); Spec: the spectinomycin gene; RB: the right boundary; prAtUbi10: the *Arabidopsis thaliana* Ubiquitin 10 gene promoter (SEQ ID NO: 12); spAtCTP2: the *Arabidopsis thaliana* chloroplast transit peptide (SEQ ID NO: 17); ALT-1-01: the ALT-1-01 nucleotide sequence (SEQ ID NO: 2); tNos: the terminator of nopaline synthase gene (SEQ ID NO:13); prCaMV35S: the cauliflower mosaic virus 35S promoter (SEQ ID NO: 14); PAT: the glufosinate acetyltransferase gene (SEQ ID NO: 15); tCaMV35S: the cauliflower mosaic virus 35S terminator (SEQ ID NO: 16); LB: the left boundary). Positive clones were verified by sequencing. The results showed that the inserted ALT-1-01 nucleotide sequence in the recombinant expression vector DBN100631 was the nucleotide sequence shown as SEQ ID NO: 2 in the sequence listing, that is, the ALT-1-01 nucleotide sequence was inserted correctly.

According to the above-mentioned method for constructing the recombinant expression vector DBN100632, the ALT-2-01 nucleotide sequence excised by *SpeI* and *Kas*I digested recombinant cloning vector DBN02-T was inserted into the expression vector DBNBC-01, obtaining a recombinant expression vector DBN100634. Enzyme digestion and sequencing verified that the nucleotide sequence in the recombinant expression vector DBN100634 contained the nucleotide sequence shown as SEQ ID NO: 5 in the sequence listing, that is, the ALT-2-01 nucleotide sequence was inserted correctly.

According to the above-mentioned method for constructing the recombinant expression vector DBN100631, the ALT-2-01 nucleotide sequence excised by *SpeI* and *Kas*I digested recombinant cloning vector DBN02-T was inserted into the expression vector DBNBC-01, obtaining a recombinant expression vector DBN100633 (containing spAtCTP2, located in the chloroplast). Enzyme digestion and sequencing verified that the nucleotide sequence in the recombinant expression vector DBN100633 contained the nucleotide sequence shown as SEQ ID NO: 5 in the sequence listing, that is, the ALT-2-01 nucleotide sequence was inserted correctly.

According to the above-mentioned method for constructing the recombinant expression vector DBN100632, the ALT-3-01 nucleotide sequence excised by *SpeI* and *Kas*I digested recombinant cloning vector DBN03-T was inserted into the expression vector DBNBC-01, obtaining a recombinant expression vector DBN100636. Enzyme digestion and sequencing verified that the nucleotide sequence in the recombinant expression vector DBN100636 contained the nucleotide sequence shown as SEQ ID NO: 8 in the sequence listing, that is, the ALT-3-01 nucleotide sequence was inserted correctly.

According to the above-mentioned method for constructing the recombinant expression vector DBN100631, the ALT-3-01 nucleotide sequence excised by *SpeI* and *Kas*I digested recombinant cloning vector DBN03-T was inserted into the expression vector DBNBC-01, obtaining a recombinant expression vector DBN100635 (containing spAtCTP2, located in the chloroplast). Enzyme digestion and sequencing verified that the nucleotide sequence in the recombinant expression vector DBN100635 contained the nucleotide sequence shown as SEQ ID NO: 8 in the sequence listing, that is, the ALT-3-01 nucleotide sequence was inserted correctly.

### Example 3. Acquisition of Arabidopsis thaliana plants having an ALT nucleotide sequence introduced

### 1. Transformation of Agrobacterium with the recombinant expression vectors

The *Agrobacterium* GV3101 was transformed with the recombinant expression vectors DBN100632, DBN100631, DBN100634, DBN100633, DBN100636 and DBN100635 which had been correctly constructed using the liquid nitrogen method with the following transformation conditions: placing 100 µL of *Agrobacterium* GV3101, and 3 µL of plasmid DNA (recombinant expression vector) in liquid nitrogen for 10 minutes, warm water bathing at 37°C for 10 minutes; inoculating the transformed *Agrobacterium* GV3101 into an LB tube, culturing under the conditions of a temperature of 28°C and a rotation speed of 200 rpm for 2 hours, spreading on an LB plate containing 50 mg/L of rifampicin and 50 mg/L of spectinomycin until positive single clones were grown, picking out single clones for culturing and extracting the plasmids thereof, and performing enzyme digestion verification using restriction enzymes. The results showed that the structures of the recombinant expression vectors DBN100632, DBN100631, DBN100634, DBN100633, DBN100636 and DBN100635 were completely correct.

### 2. Acquisition of transgenic Arabidopsis thaliana plants

Seeds of wild-type *Arabidopsis thaliana* were suspended in a 0.1% (w/v) agarose solution. The suspended seeds were stored at 4°C for 2 days to complete the need for dormancy, in order to ensure synchronous seed germination. Vermiculite was mixed with horse manure soil, the mixture was sub-irrigated with water to wet, and the soil mixture was allowed to drain the water away for 24 hours. The pretreated seeds were sowed in the soil mixture and covered with a moisturizing cover for 7 days. The seeds were germinated and the plants were cultivated in a greenhouse under long day conditions (16 hours of light/8 hours of dark) of a constant temperature (22°C) and a constant humidity (40-50%) with a light intensity of 120-150 µmol/(m²·sec). The plants were initially irrigated with the Hoagland's nutrient solution, followed by deionized water, keeping the soil moist but not wet through.

*Arabidopsis thaliana* was transformed using the flower soaking method. One or more 15-30 mL of precultures of YEP culture solution (containing spectinomycin (50 mg/L) and rifampicin (10 mg/L)) were inoculated with the selected *Agrobacterium* colonies. The cultures were incubated at 28°C and 220 rpm with shaking at a constant speed overnight. Each preculture was used to inoculate two 500 mL of cultures of YEP culture solution (containing spectinomycin (50 mg/L) and rifampicin (10 mg/L)), and the cultures were incubated at 28°C with continuous shaking overnight. Cells were precipitated by centrifuging at about 8700 x g at room temperature for 10 minutes, and the resulting supernatant was discarded. The cell precipitate was gently re-suspended in 500 mL osmotic medium which contained 1/2x MS salt /B5 vitamin, 10% (w/v) sucrose, 0.044 µM benzylaminopurine (10 µL/L (1 mg/mL, a stock solution in DMSO)) and 300 µL/L of Silvet L-77. About 1-month-old plants were soaked in a culture medium for 15 seconds to ensure immersion of the latest inflorescence. Then, the plants were reclined laterally and covered (transparently or opaquely) for 24 hours, then washed with water, and placed vertically. The plants were cultivated with a photoperiod of 16 hours of light/8 hours of dark at 22°C. Seeds were harvested after soaking for about 4 weeks.

The newly harvested (ALT nucleotide sequence) T₁ seeds were dried at room temperature for 7 days. The seeds were sowed in 26.5 × 51 cm germination disks, and 200 mg T₁ seeds (about 10000 seeds) were accepted per disk, wherein the seeds had been previously suspended in 40 mL of 0.1% (w/v) agarose solution and stored at 4°C for 2 days to complete the need for dormancy, in order to ensure synchronous seed germination.

Vermiculite was mixed with horse manure soil, the mixture was sub-irrigated with water to wet, and water was drained through gravity. The pretreated seeds (each 40 mL) were sowed evenly in the soil mixture using a pipette, and covered with a moisturizing cover for 4-5 days. The cover was removed 1 day before performing initial transformant selection by spraying glufosinate (used to select the co-transformed PAT gene) post emergence.

The T₁ plants were sprayed with a 0.2% solution of a Liberty herbicide (200 g ai/L of glufosinate) reusing a DeVilbiss compressed air nozzle at a spray volume of 10 mL/disc (703 L/ha) 7 days after planting (DAP) and 11 DAP (the cotyledon stage and 2-4 leaf stage, respectively), to provide an effective amount of glufosinate of 280 g ai/ha per application. Surviving plants (actively growing plants) were identified 4-7 days after the final spraying, and transplanted to 7 cm x 7 cm square pots prepared with horse manure soil and vermiculite (3-5 plants/disc), respectively. The transplanted plants were covered with a moisturizing cover for 3-4 days, and placed in a 22°C culture chamber or directly transferred into a greenhouse as previously. Then, the cover was removed, and at least 1 day before testing the ability of the ALT gene to provide tribenuron-methyl herbicide resistance, the plants were planted into a greenhouse (22 ± 5°C, 50 ± 30% RH, 14 hours of light: 10 hours of dark, a minimum of 500 µE/m²s¹ natural + supplemental light).

### Example 4. Detection of herbicide tolerance effects of the transgenic Arabidopsis thaliana plants

T₁ transformants were initially selected from the background of untransformed seeds using a glufosinate selection scheme. About 40000 T₁ seeds were screened, and 380 T₁ positive transformants (PAT gene) were identified with a transformation efficiency of about 0.95%. The plants that were transformed with the recombinant expression vector DBN100632 were *Arabidopsis thaliana* plants having an ALT-1-01 nucleotide sequence located in the cytoplasm introduced (At cytoplasmic ALT-1-01), and the plants that were transformed with the recombinant expression vector DBN100631 were *Arabidopsis thaliana* plants having an ALT-1-01 nucleotide sequence located in the chloroplast introduced (At chloroplastic ALT-1-01); the plants that were transformed with the recombinant expression vector DBN100634 were *Arabidopsis thaliana* plants having an ALT-2-01 nucleotide sequence located in the cytoplasm introduced (At cytoplasmic ALT-2-01), and the plants that were transformed with the recombinant expression vector DBN100633 were *Arabidopsis thaliana* plants having an ALT-2-01 nucleotide sequence located in the chloroplast introduced (At chloroplastic ALT-2-01); and the plants that were transformed with the recombinant expression vector DBN100636 were *Arabidopsis thaliana* plants having an ALT-3-01 nucleotide sequence located in the cytoplasm introduced (At cytoplasmic ALT-3-01), and the plants that were transformed with the recombinant expression vector DBN100635 were *Arabidopsis thaliana* plants having an ALT-3-01 nucleotide sequence located in the chloroplast introduced (At chloroplastic ALT-3-01). The herbicide tolerance effects of At cytoplasmic ALT-1-01 T₁ plants, At chloroplastic ALT-1-01 T₁ plants, At cytoplasmic ALT-2-01 T₁ plants, At chloroplastic ALT-2-01 T₁ plants, At cytoplasmic ALT-3-01 T₁ plants, At chloroplastic ALT-3-01 T₁ plants and wild-type *Arabidopsis thaliana* plants on tribenuron-methyl were detected (14 days after sowing), respectively.

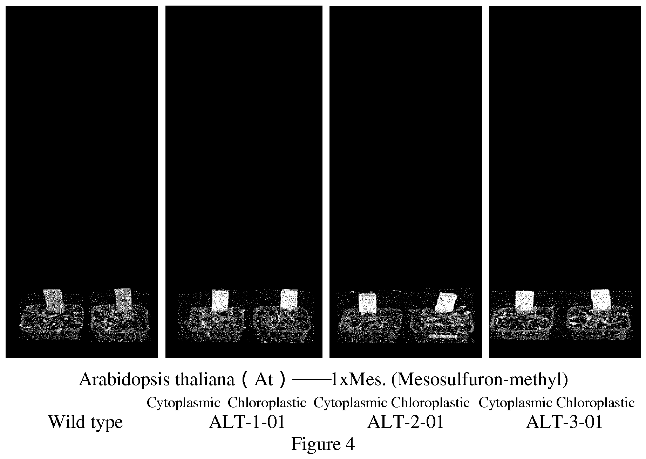

At cytoplasmic ALT-1-01 T₁ plants, At chloroplastic ALT-1-01 T₁ plants, At cytoplasmic ALT-2-01 T₁ plants, At chloroplastic ALT-2-01 T₁ plants, At cytoplasmic ALT-3-01 T₁ plants, At chloroplastic ALT-3-01 T₁ plants and wild-type *Arabidopsis thaliana* plants were sprayed with tribenuron-methyl (18 g ai/ha, 1-fold field concentration) and a blank solvent (water), respectively. Plants were counted for the resistance situations 14 days after spraying: those having a consistent growth status with the blank solvent (water) group after 14 days were classified as highly resistant plants, those having a bolting height less than 1/2 of that of the blank solvent (water) group after 14 days were classified as moderately resistant plants, those still not capable of bolting after 14 days were classified as poorly resistant plants, and those dead after 14 days were classified as non-resistant plants. Since each *Arabidopsis thaliana* T₁ plant was an independent transformation event, a significant difference in individual T1 responses could be expected at a given dose. The results are as shown in Table 1 and Figure 4.

**Table 1. Experimental results of the tolerance of transgenic Arabidopsis thaliana T₁ plants to a tribenuron-methyl herbicide**

| **Treatment** | ***Arabidopsis thaliana* genotypes** | **Highly resistant** | **Moderately resistant** | **Poorly resistant** | **Non-resistant** | **Total** |
|---|---|---|---|---|---|---|
| **Blank solvent (water)** | At cytoplasmic ALT-1-01 | 30 | 0 | 0 | 0 | 30 |
| | At chloroplastic ALT-1-01 | 28 | 0 | 0 | 0 | 28 |
| | At cytoplasmic ALT-2-01 | 31 | 0 | 0 | 0 | 31 |
| | At chloroplastic ALT-2-01 | 25 | 0 | 0 | 0 | 25 |
| | At cytoplasmic ALT-3-01 | 27 | 0 | 0 | 0 | 27 |
| | At chloroplastic ALT-3-01 | 27 | 0 | 0 | 0 | 27 |
| | wild-type | 30 | 0 | 0 | 0 | 30 |
| **18 g ai/ha tribenuron-methyl (1x Tri.)** | At cytoplasmic ALT-1-01 | 24 | 2 | 1 | 1 | 28 |
| | At chloroplastic ALT-1-01 | 28 | 0 | 0 | 2 | 30 |
| | At cytoplasmic ALT-2-01 | 25 | 1 | 1 | 3 | 30 |
| | At chloroplastic ALT-2-01 | 29 | 0 | 1 | 1 | 31 |
| | At cytoplasmic ALT-3-01 | 22 | 1 | 1 | 3 | 27 |
| | At chloroplastic ALT-3-01 | 27 | 0 | 0 | 2 | 29 |
| | wild-type | 0 | 0 | 0 | 32 | 32 |

For *Arabidopsis thaliana,* 18 g ai/ha tribenuron-methyl herbicide is an effective dose distinguishing sensitive plants from plants having an average level of resistance. The results of Table 1 and Figure 4 show that: the thifensulfuron hydrolase (ALT-1, ALT-2 and ALT-3) conferred tribenuron-methyl herbicide tolerance to individual *Arabidopsis thaliana* plants (the reason why individual plants were not tolerant was that the insertion site in the T₁ plants was random, the expression levels of the tolerance gene were different, showing a difference in tolerance level); compared to At cytoplasmic ALT-1-01 T₁ plants, At cytoplasmic ALT-2-01 T₁ plants and At cytoplasmic ALT-3-01 T₁ plants, At chloroplastic ALT-1-01 T₁ plants, At chloroplastic ALT-2-01 T₁ plants and At chloroplastic ALT-3-01 T₁ plants were able to produce a higher tribenuron-methyl herbicide tolerance, suggesting that the thifensulfuron hydrolase (ALT-1, ALT-2 and ALT-3) gene may enhance the tolerance of *Arabidopsis thaliana* plants to the tribenuron-methyl herbicide when located in the chloroplast for expression; while none of the wild-type *Arabidopsis thaliana* plants was tolerant to the tribenuron-methyl herbicide.

### Example 5. Having an unexpected technical effect on different sulfonylurea herbicides

The thifensulfuron hydrolase, which can also be known as sulfonylurea herbicide de-esterase, degrades ester bond-containing sulfonylurea herbicides (e.g., thifensulfuron, etc.) into herbicidally inactive mother acids by hydrolyzing the ester bond, and therefore it cannot degrade ester bond-free sulfonylurea herbicides (e.g., nicosulfuron, chlorsulfuron, etc.). In the prior art, there are many sulfonylurea herbicides containing ester bonds and having similar structures, such as tribenuron-methyl, iodosulfuron-methyl, oxasulfuron, mesosulfuron (mesosulfuron-methyl), pyrazosulfuron-ethyl, sulfometuron-methyl, and halosulfuron-methyl.

At cytoplasmic ALT-1-01 T₁ plants, At chloroplastic ALT-1-01 T₁ plants, At cytoplasmic ALT-2-01 T₁ plants, At chloroplastic ALT-2-01 T₁ plants, At cytoplasmic ALT-3-01 T₁ plants, At chloroplastic ALT-3-01 T₁ plants and wild-type *Arabidopsis thaliana* plants in Example 4 were sprayed with iodosulfuron-methyl (10 g ai/ha, 1-fold field concentration), mesosulfuron (14 g ai/ha, 1-fold field concentration) and oxasulfuron (60 g ai/ha, 1-fold field concentration), respectively, in addition to tribenuron-methyl (18 g ai/ha, 1-fold field concentration) and the blank solvent (water). Plants were counted for the resistance situation 14 days after spraying: those having a consistent growth status with the blank solvent (water) group after 14 days were classified as highly resistant plants, those having a bolting height less than 1/2 of that of the blank solvent (water) group after 14 days were classified as moderately resistant plants, those still not capable of bolting after 14 days were classified as poorly resistant plants, and those dead after 14 days were classified as non-resistant plants. Since each *Arabidopsis thaliana* T₁ plant was an independent transformation event, a significant difference in individual T₁ responses could be expected at a given dose. The results are as shown in Table 2 and Figure 4.

**Table 2. Experimental results of the tolerance of transgenic Arabidopsis thaliana T₁ plants to sulfonylurea herbicides**

| **Treatment** | ***Arabidopsis thaliana* genotypes** | **Highly resistant** | **Moderately resistant** | **Poorly resistant** | **Non-resistant** | **Total** |
|---|---|---|---|---|---|---|
| **Blank solvent (water)** | At cytoplasmic ALT-1-01 | 30 | 0 | 0 | 0 | 30 |
| | At chloroplastic ALT-1-01 | 28 | 0 | 0 | 0 | 28 |
| | At cytoplasmic ALT-2-01 | 31 | 0 | 0 | 0 | 31 |
| | At chloroplastic ALT-2-01 | 25 | 0 | 0 | 0 | 25 |
| | At cytoplasmic ALT-3-01 | 27 | 0 | 0 | 0 | 27 |
| | At chloroplastic ALT-3-01 | 27 | 0 | 0 | 0 | 27 |
| | wild-type | 30 | 0 | 0 | 0 | 30 |
| **10 g ai/ha iodosulfuron-methyl (1x Iod.)** | At cytoplasmic ALT-1-01 | 0 | 0 | 0 | 29 | 29 |
| | At chloroplastic ALT-1-01 | 0 | 0 | 0 | 30 | 30 |
| | At cytoplasmic ALT-2-01 | 0 | 0 | 0 | 30 | 30 |
| | At chloroplastic ALT-2-01 | 0 | 0 | 0 | 31 | 31 |
| | At cytoplasmic ALT-3-01 | 0 | 0 | 0 | 30 | 30 |
| | At chloroplastic ALT-3-01 | 0 | 0 | 0 | 32 | 32 |
| | wild-type | 0 | 0 | 0 | 29 | 29 |
| **14 g ai/ha mesosulfuron (1x Mes.)** | At cytoplasmic ALT-1-01 | 0 | 0 | 0 | 29 | 29 |
| | At chloroplastic ALT-1-01 | 0 | 0 | 0 | 32 | 32 |
| | At cytoplasmic ALT-2-01 | 0 | 0 | 0 | 32 | 32 |
| | At chloroplastic ALT-2-01 | 0 | 0 | 0 | 30 | 30 |
| | At cytoplasmic ALT-3-01 | 0 | 0 | 0 | 30 | 30 |
| | At chloroplastic ALT-3-01 | 0 | 0 | 0 | 32 | 32 |
| | wild-type | 0 | 0 | 0 | 28 | 28 |
| **60 g ai/ha oxasulfuron (1x Oxa.)** | At cytoplasmic ALT-1-01 | 0 | 0 | 0 | 28 | 28 |
| | At chloroplastic ALT-1-01 | 0 | 0 | 0 | 30 | 30 |
| | At cytoplasmic ALT-2-01 | 0 | 0 | 0 | 30 | 30 |
| | At chloroplastic ALT-2-01 | 0 | 0 | 0 | 30 | 30 |
| | At cytoplasmic ALT-3-01 | 0 | 0 | 0 | 30 | 30 |
| | At chloroplastic ALT-3-01 | 0 | 0 | 0 | 31 | 31 |
| | wild-type | 0 | 0 | 0 | 28 | 28 |

The responses of inputting the thifensulfuron hydrolase activity to *Arabidopsis thaliana* T₁ plants by ALT-1, ALT-2 and ALT-3 were compared in Table 2. The thifensulfuron hydrolase activity was conferred to all the transformed *Arabidopsis thaliana* T₁ plants; however, in the given treatments (iodosulfuron-methyl, mesosulfuron and oxasulfuron), all the transformed *Arabidopsis thaliana* T₁ plants did not exhibit the ability to degrade the above-mentioned sulfonylurea herbicides, and there was no difference between all the transformed *Arabidopsis thaliana* T₁ plants (ALT-1, ALT-2 and ALT-3) and the wild-type *Arabidopsis thaliana* plants in the degree of damage.

Table 2 fully illustrated that the results of Table 1 were unexpected. Although tribenuron-methyl as well as thifensulfuron, iodosulfuron-methyl, mesosulfuron and oxasulfuron are all sulfonylurea herbicides containing ester bonds and having similar chemical structures, the given treatments were also comparable (1-fold field concentration) and at the same time, the thifensulfuron hydrolase (ALT-1, ALT-2 and ALT-3) had been input and expressed at an expected level in the plant individuals, plants expressing the thifensulfuron hydrolase neither had the ability to degrade iodosulfuron-methyl, mesosulfuron and oxasulfuron, nor could protect themselves from damage from the above-mentioned sulfonylurea herbicides, and showed no difference from the wild-type plants in performance, wherein these data are sufficient to confirm that the tribenuron-methyl herbicide tolerance conferred by the thifensulfuron hydrolase (ALT-1, ALT-2 and ALT-3) on the plants was difficult to predict.

### Example 6. Construction of soybean recombinant expression vectors and transformation of Agrobacterium with the recombinant expression vectors

### 1. Construction of soybean recombinant expression vectors containing ALT nucleotide sequences

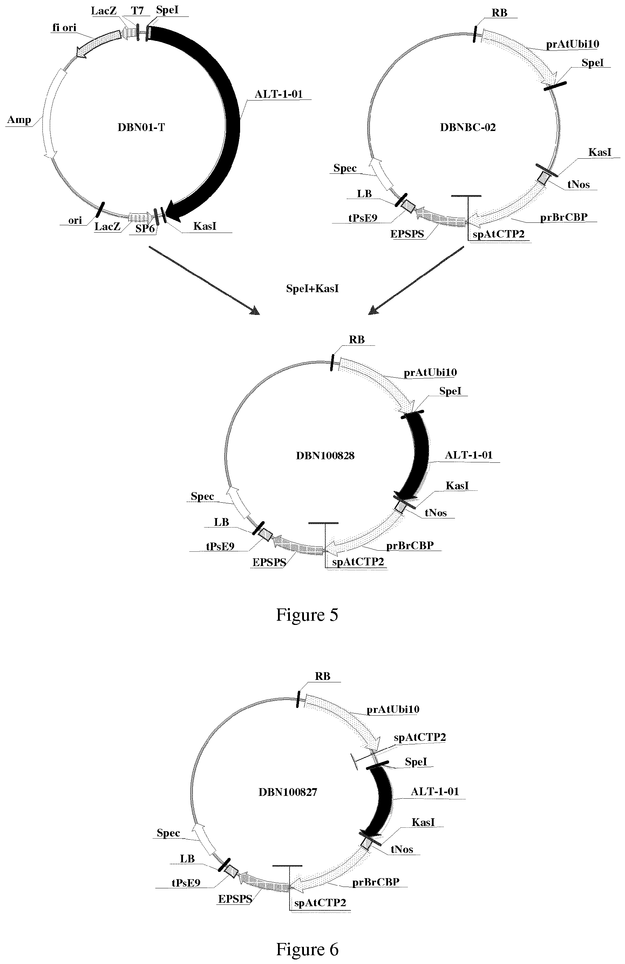

The recombinant cloning vectors DBN01-T and DBN04-T as well as an expression vector DBNBC-02 (vector backbone: pCAMBIA2301 (which can be provided by the CAMBIA institution)) were digested with restriction enzymes *SpeI* and *Kas*I as well as *Nco*I and *Fsp*I*,* respectively; the excised ALT-1-01 nucleotide sequence and EPSPS nucleotide sequence fragments were inserted between the *Spe*I and *Kas*I as well as *Nco*I and *Fsp*I sites in the expression vector DBNBC-02, respectively; and it is well known to a person skilled in the art to construct a vector using conventional enzyme digestion methods, a recombinant expression vector DBN100828 was constructed (located in the cytoplasm), the construction process of which is as shown in Figure 5 (Spec: the spectinomycin gene; RB: the right boundary; prAtUbi10: the *Arabidopsis thaliana* Ubiquitin 10 gene promoter (SEQ ID NO: 12); ALT-1-01: the ALT-1-01 nucleotide sequence (SEQ ID NO: 2); tNos: the terminator of nopaline synthase gene (SEQ ID NO:13); prBrCBP: the rape eukaryotic elongation factor gene 1α (Tsf1) promoter (SEQ ID NO: 18); spAtCTP2: the *Arabidopsis thaliana* chloroplast transit peptide (SEQ ID NO: 17); EPSPS: the 5-enolpyruvylshikimate-3-phosphate synthase gene (SEQ ID NO: 11); tPsE9: the pea RbcS gene terminator (SEQ ID NO: 19); LB: the left boundary).

According to the method in point 2 of Example 2, *Escherichia coli* T1 competent cells were transformed with the recombinant expression vector DBN100828 using the heat shock method, and the plasmids in the cells were extracted through the alkaline method. The extracted plasmid was identified after digesting with restriction enzymes *Spe*I and *Kas*I*,* and positive clones were identified by sequencing. The results showed that the nucleotide sequence between the *Spe*I and *Kas*I sites in the recombinant expression vector DBN100828 was the nucleotide sequence shown as SEQ ID NO: 2 in the sequence listing, i.e., the ALT-1-01 nucleotide sequence.

According to the above-mentioned method for constructing the recombinant expression vector DBN100828, a recombinant expression vector DBN100827 (located in the chloroplast) containing the ALT-1-01 nucleotide sequence was constructed, the vector structure of which was shown as Figure 6 (vector backbone: pCAMBIA2301 (which can be provided by the CAMBIA institution); Spec: the spectinomycin gene; RB: the right boundary; prAtUbi10: the *Arabidopsis thaliana* Ubiquitin 10 gene promoter (SEQ ID NO: 12); spAtCTP2: the *Arabidopsis thaliana* chloroplast transit peptide (SEQ ID NO: 17); ALT-1-01: the ALT-1-01 nucleotide sequence (SEQ ID NO: 2); tNos: the terminator of nopaline synthase gene (SEQ ID NO:13); prBrCBP: the rape eukaryotic elongation factor gene 1α (Tsf1) promoter (SEQ ID NO: 18); spAtCTP2: the *Arabidopsis thaliana* chloroplast transit peptide (SEQ ID NO: 17); EPSPS: the 5-enolpyruvylshikimate-3-phosphate synthase gene (SEQ ID NO: 11); tPsE9: the pea RbcS gene terminator (SEQ ID NO: 19); LB: the left boundary). Positive clones were verified by sequencing. The results showed that the inserted ALT-1-01 nucleotide sequence in the recombinant expression vector DBN100827 was the nucleotide sequence shown as SEQ ID NO: 2 in the sequence listing, that is, the ALT-1-01 nucleotide sequence was inserted correctly.

According to the above-mentioned method for constructing the recombinant expression vector DBN100828, the ALT-2-01 nucleotide sequence and the EPSPS nucleotide sequence excised by *SpeI* and *Kas*I as well as *Nco*I and *Fsp*I from digested recombinant cloning vectors DBN02-T and DBN04-T were inserted into the expression vector DBNBC-02, obtaining a recombinant expression vector DBN100826. Enzyme digestion and sequencing verified that the nucleotide sequences in the recombinant expression vector DBN100826 contained the nucleotide sequences shown as SEQ ID NO: 5 and SEQ ID NO: 11 in the sequence listing, that is, the ALT-2-01 nucleotide sequence and the EPSPS nucleotide sequence were inserted correctly.

According to the above-mentioned method for constructing the recombinant expression vector DBN100827, the ALT-2-01 nucleotide sequence and the EPSPS nucleotide sequence excised by *Spe*I and *Kas*I as well as *Nco*I and *Fsp*I from digested recombinant cloning vectors DBN02-T and DBN04-T were inserted into the expression vector DBNBC-02, obtaining a recombinant expression vector DBN100825 (containing spAtCTP2, located in the chloroplast). Enzyme digestion and sequencing verified that the nucleotide sequences in the recombinant expression vector DBN100825 contained the nucleotide sequences shown as SEQ ID NO: 5 and SEQ ID NO: 11 in the sequence listing, that is, the ALT-2-01 nucleotide sequence and the EPSPS nucleotide sequence were inserted correctly.

According to the above-mentioned method for constructing the recombinant expression vector DBN100828, the ALT-3-01 nucleotide sequence and the EPSPS nucleotide sequence excised by *Spe*I and *Kas*I as well as *Nco*I and *Fsp*I from digested recombinant cloning vectors DBN03-T and DBN04-T were inserted into the expression vector DBNBC-02, obtaining a recombinant expression vector DBN100824. Enzyme digestion and sequencing verified that the nucleotide sequences in the recombinant expression vector DBN100824 contained the nucleotide sequences shown as SEQ ID NO: 8 and SEQ ID NO: 11 in the sequence listing, that is, the ALT-3-01 nucleotide sequence and the EPSPS nucleotide sequence were inserted correctly.

According to the above-mentioned method for constructing the recombinant expression vector DBN100827, the ALT-3-01 nucleotide sequence and the EPSPS nucleotide sequence excised by *Spe*I and *Kas*I as well as *Nco*I and *Fsp*I from digested recombinant cloning vectors DBN03-T and DBN04-T were inserted into the expression vector DBNBC-02, obtaining a recombinant expression vector DBN100823 (containing spAtCTP2, located in the chloroplast). Enzyme digestion and sequencing verified that the nucleotide sequences in the recombinant expression vector DBN100823 contained the nucleotide sequences shown as SEQ ID NO: 8 and SEQ ID NO: 11 in the sequence listing, that is, the ALT-3-01 nucleotide sequence and the EPSPS nucleotide sequence were inserted correctly.

2. Transformation of *Agrobacterium* with the recombinant expression vectors *Agrobacterium* LBA4404 (Invitrogen, Chicago, USA, CAT: 18313-015) was transformed with the recombinant expression vectors DBN100828, DBN100827, DBN100826, DBN100825, DBN100824 and DBN100823 which had been correctly constructed using a liquid nitrogen method, with the following transformation conditions: placing 100 µL of *Agrobacterium* LBA4404, and 3 µL of plasmid DNA (recombinant expression vector) in liquid nitrogen for 10 minutes, warm water bathing at 37°C for 10 minutes; inoculating the transformed *Agrobacterium* LBA4404 into an LB tube, culturing under the conditions of a temperature of 28°C and a rotation speed of 200 rpm for 2 hours, spreading on an LB plate containing 50 mg/L of rifampicin and 50 mg/L of spectinomycin until positive single clones were grown, picking out single clones for culturing and extracting the plasmids thereof, and performing enzyme digestion verification using restriction enzymes. The results showed that the structures of the recombinant expression vectors DBN100828, DBN100827, DBN100826, DBN100825, DBN100824 and DBN100823 were completely correct.

### Example 7. Acquisition and verification of transgenic soybean plants

### 1. Acquisition of transgenic soybean plants

According to the *Agrobacterium* infection method conventionally used, the cotyledonary node tissue of a sterile culture of soybean variety Zhonghuang13 was co-cultured with the *Agrobacterium* in point 2 of Example 6, so as to introduce T-DNA (comprising the *Arabidopsis thaliana* Ubiquitin10 gene promoter sequence, an ALT-1-01 nucleotide sequence, an ALT-2-01 nucleotide sequence, an ALT-3-01 nucleotide sequence, the tNos terminator, the rape eukaryotic elongation factor gene 1α promoter, the *Arabidopsis thaliana* chloroplast transit peptide, 5-enolpyruvylshikimate-3-phosphate synthase gene and the pea RbcS gene terminator) in the recombinant expression vectors DBN100828, DBN100827, DBN100826, DBN100825, DBN100824 and DBN100823 constructed in Example 6.1 into the soybean chromosomes, obtaining soybean plants that were transformed with the recombinant expression vector DBN100828 and had an ALT-1-01 nucleotide sequence located in the cytoplasm introduced (Gm cytoplasmic ALT-1-01) and soybean plants that were transformed with the recombinant expression vector DBN100827 and had an ALT-1-01 nucleotide sequence located in the chloroplast introduced (Gm chloroplastic ALT-1-01); soybean plants that were transformed with the recombinant expression vector DBN100826 and had an ALT-2-01 nucleotide sequence located in the cytoplasm introduced (Gm cytoplasmic ALT-2-01) and soybean plants that were transformed with the recombinant expression vector DBN100825 and had an ALT-2-01 nucleotide sequence located in the chloroplast introduced (Gm chloroplastic ALT-2-01); and soybean plants that were transformed with the recombinant expression vector DBN100824 and had an ALT-3-01 nucleotide sequence located in the cytoplasm introduced (Gm cytoplasmic ALT-3-01) and soybean plants that were transformed with the recombinant expression vector DBN100823 and had an ALT-3-01 nucleotide sequence located in the chloroplast introduced (Gm chloroplastic ALT-3-01); meanwhile, wild type soybean plants were used as the control.

As regards the *Agrobacterium-mediated* soybean transformation, briefly, mature soybean seeds were germinated in a soybean germination culture medium (3.1 g/L of B5 salt, B5 vitamin, 20 g/L of sucrose, and 8 g/L of agar, pH 5.6), and the seeds were inoculated in a germination culture medium and cultured under the conditions of a temperature of 25 ± 1°C; and a photoperiod (light/dark) of 16 h/8 h. After 4-6 days of germination, soybean sterile seedlings swelled at bright green cotyledonary nodes were taken, hypocotyledonary axes were cut off 3-4 millimeters below the cotyledonary nodes, the cotyledons were cut longitudinally, and apical buds, lateral bud and seminal roots were removed. A wound was made at a cotyledonary node using the knife back of a scalpel, and the wounded cotyledonary node tissue was contacted with an *Agrobacterium* suspension, wherein the *Agrobacterium* can transfer the ALT-1-01 nucleotide sequence, the ALT-2-01 nucleotide sequence and the ALT-3-01 nucleotide sequence to the wounded cotyledonary node tissue (step 1: infection step). In this step, the cotyledonary node tissues were preferably immersed in the *Agrobacterium* suspension (OD₆₆₀ = 0.5-0.8, an infection culture medium (2.15 g/L of MS salt, B5 vitamin, 20 g/L of sucrose, 10 g/L of glucose, 40 mg/L of acetosyringone (AS), 4 g/L of 2-morpholine ethanesulfonic acid (MES), and 2 mg/L of zeatin (ZT), pH 5.3) to initiate the inoculation. The cotyledonary node tissues were co-cultured with *Agrobacterium* for a period of time (3 days) (step 2: co-culturing step). Preferably, the cotyledonary node tissues were cultured in a solid culture medium (4.3 g/L of MS salt, B5 vitamin, 20 g/L of sucrose, 10 g/L of glucose, 4 g/L of 2-morpholine ethanesulfonic acid (MES), 2 mg/L of zeatin, and 8 g/L of agar, pH 5.6) after the infection step. After this co-culturing stage, there can be an optional "recovery" step. In the "recovery" step, there may be at least one antibiotic (cephalosporin) known to inhibit the growth of *Agrobacterium* in a recovery culture medium (3.1 g/L of B5 salt, B5 vitamin, 1 g/L of 2-morpholine ethanesulfonic acid (MES), 30 g/L of sucrose, 2 mg/L of zeatin (ZT), 8 g/L of agar, 150 mg/L of cephalosporin, 100 mg/L of glutamic acid, and 100 mg/L of aspartic acid, pH 5.6), without the addition of a selective agent for a plant transformant (step 3: recovery step). Preferably, tissue blocks regenerated from the cotyledonary nodes were cultured in a solid culture medium with an antibiotic but without a selective agent, to eliminate *Agrobacterium* and provide a recovery stage for the infected cells. Subsequently, the tissue blocks regenerated from the cotyledonary nodes were cultured in a culture medium containing a selective agent (glyphosate), and growing transformed calli were selected (step 4: selection step). Preferably, the tissue blocks regenerated from the cotyledonary nodes were cultured in a screening solid culture medium (3.1 g/L of B5 salt, B5 vitamin, 1 g/L of 2-morpholine ethanesulfonic acid (MES), 30 g/L of sucrose, 1 mg/L of 6-benzyladenine (6-BAP), 8 g/L of agar, 150 mg/L of cephalosporin, 100 mg/L of glutamic acid, 100 mg/L of aspartic acid, and 0.25 mol/L of N-(phosphonomethyl)glycine, pH 5.6) containing a selective agent, resulting in selective growth of the transformed cells. Then, plants were regenerated from the transformed cells (step 5: regeneration step). Preferably, the tissue blocks regenerated from the cotyledonary nodes grown in a culture medium containing a selective agent were cultured in solid culture media (B5 differentiation culture medium and B5 rooting culture medium) to regenerate plants.

The screened out resistant tissues were transferred onto the B5 differentiation culture medium (3.1 g/L of B5 salt, B5 vitamin, 1 g/L of 2-morpholine ethanesulfonic acid (MES), 30 g/L of sucrose, 1 mg/L of zeatin (ZT), 8 g/L of agar, 150 mg/L of cephalosporin, 50 mg/L of glutamic acid, 50 mg/L of aspartic acid, 1 mg/L of gibberellin, 1 mg/L of auxin, and 0.25 mol/L of N-(phosphonomethyl)glycine, pH 5.6), and cultured at 25°C for differentiation. The differentiated seedlings were transferred onto the B5 rooting culture medium (3.1 g/L of B5 salt, B5 vitamin, 1 g/L of 2-morpholine ethanesulfonic acid (MES), 30 g/L of sucrose, 8 g/L of agar, 150 mg/L of cephalosporin, and 1 mg/L of indole-3-butyric acid (IBA)), cultured in the rooting culture medium to be a height of about 10 cm at 25°C, and transferred to a glasshouse for culturing until fruiting. In the greenhouse, the plants were cultured at 26 °C for 16 hours, and then cultured at 20°C for 8 hours every day.

### 2. Verification of the transgenic soybean plants using TaqMan

Leaves of about 100 mg from Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants and Gm chloroplastic ALT-3-01 soybean plants were respectively taken as samples, genomic DNAs thereof were extracted with a DNeasy Plant Maxi Kit (Qiagen), and the copy number of the *EPSPS* gene was detected by the Taqman probe fluorescence quantitative PCR method so as to determine the copy number of the ALT gene. At the same time, wild type soybean plants were used as controls, and detected and analyzed according to the above-mentioned method. Triple repeats were set for the experiments, and averaged.

The specific method for detecting the copy number of the *EPSPS* gene was as follows:
Step 11. Leaves of 100 mg from Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants, Gm chloroplastic ALT-3-01 soybean plants and wild-type soybean plants were respectively taken each, respectively ground into a homogenate in a mortar with liquid nitrogen, and 3 replicates were taken for each sample;
Step 12. Genomic DNAs of the above-mentioned samples were extracted using a DNeasy Plant Mini Kit of Qiagen, and the particular method can refer to the product manual thereof;
Step 13. The concentrations of the genomic DNAs of the above-mentioned samples were detected using NanoDrop 2000 (Thermo Scientific);
Step 14. The concentrations of the genomic DNAs of the above-mentioned samples were adjusted to a consistent concentration value which ranges from 80 to 100 ng/µL;
Step 15. The copy numbers of the samples were identified using the Taqman probe fluorescence quantitative PCR method, wherein samples for which the copy numbers had been identified and known were taken as standards, the samples of the wild type soybean plants were taken as the control, and triple repeats were taken for each sample and averaged; the sequences of fluorescence quantitative PCR primers and a probe were as follows, respectively:
the following primers and probe were used to detect the *EPSPS* gene sequence:
primer 1: CTGGAAGGCGAGGACGTCATCAATA, shown as SEQ ID NO: 20 in the sequence listing;
primer 2: TGGCGGCATTGCCGAAATCGAG, shown as SEQ ID NO: 21 in the sequence listing;
probe 1: ATGCAGGCGATGGGCGCCCGCATCCGTA, shown as SEQ ID NO: 22 in the sequence listing;
PCR reaction system:

| | |
|---|---|
| JumpStart™ Taq ReadyMix™ (Sigma) | 10 µL |
| 50× primer/probe mixture | 1 µL |
| genomic DNA | 3 µL |
| water (ddH₂O) | 6 µL |

The 50× primer/probe mixture comprises 45 µL of each primer at a concentration of 1 mM, 50 µL of the probe at a concentration of 100 µM, and 860 µL of 1× TE buffer, and was stored at 4°C in an amber tube.

### PCR reaction conditions:

| Step | Temperature | Time |
|---|---|---|
| 21 | 95°C | 5 minutes |
| 22 | 95°C | 30 seconds |
| 23 | 60°C | 1 minute |
| 24 | back to step 22, repeated 40 times | |

Data were analyzed using software SDS2. 3 (Applied Biosystems).

It was further confirmed by analyzing the experimental results of the copy number of the *EPSPS* gene that the ALT-1-01 nucleotide sequence, the ALT-2-01 nucleotide sequence and the ALT-3-01 nucleotide sequence had all been incorporated into the chromosomes of the detected soybean plants, and Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants and Gm chloroplastic ALT-3-01 soybean plants all resulted in single copy transgenic soybean plants.

### Example 8. Detection of herbicide tolerance effects of the transgenic soybean plants

### 1. Tribenuron-methyl tolerance

The herbicide tolerance effects of Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants, Gm chloroplastic ALT-3-01 soybean plants and wild-type soybean plants on tribenuron-methyl were detected (at seedling stage), respectively.

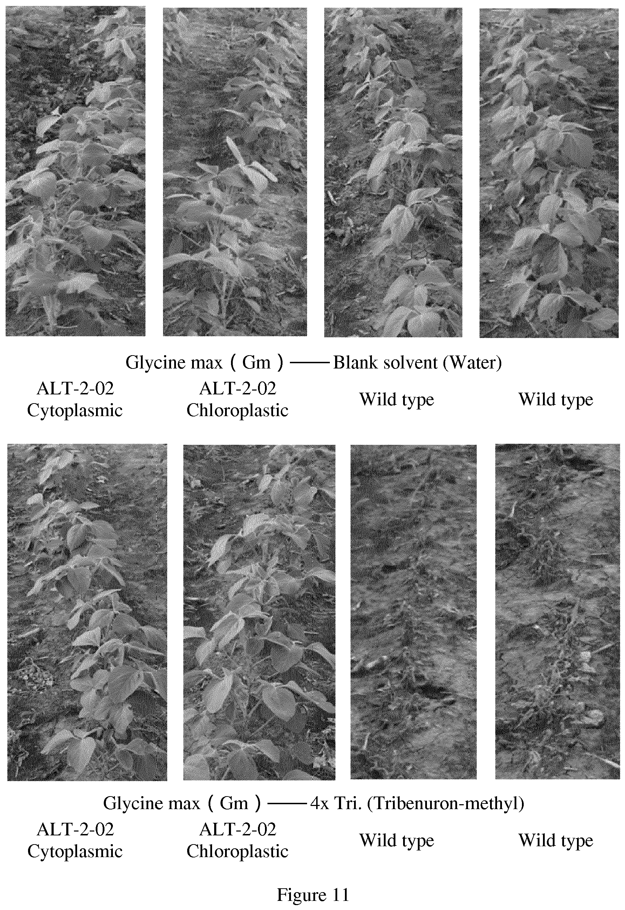

Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants, Gm chloroplastic ALT-3-01 soybean plants and wild-type soybean plants were respectively taken and sprayed with tribenuron-methyl (72 g ai/ha, 4-fold field concentration) and a blank solvent (water). The damage degree caused by the herbicide was measured for each plant according to the leaf curl degree and the growth point damage degree 3 days after spraying (3 DAT), 7 days after spraying (7 DAT), 14 days after spraying (14 DAT) and 21 days after spraying (21 DAT), respectively: considering conditions of leaves being flat as the untreated plants and growth points being intact as having a damage degree of 0%; considering conditions of veins being locally browned, new leaves being malformed and plant growth being slow as having a damage degree of 50%; and considering conditions of veins being purple to whole plant being dead and growth points being browned and dry as having a damage degree of 100%. There were 2 strains in Gm cytoplasmic ALT-1-01 soybean plants in total (S1 and S2), 2 strains in Gm chloroplastic ALT-1-01 soybean plants in total (S3 and S4), 2 strains in Gm cytoplasmic ALT-2-01 soybean plants in total (S5 and S6), 2 strains in Gm chloroplastic ALT-2-01 soybean plants in total (S7 and S8), 2 strains in Gm cytoplasmic ALT-3-01 soybean plants in total (S9 and S10), 2 strains in Gm chloroplastic ALT-3-01 soybean plants in total (S11 and S12), and 1 strain in wild-type soybean plants (CK1) in total; and 10-15 plants were selected from each strain and tested. The results are as shown in Table 3 and Figure 11.

**Table 3. Experimental results of the herbicide tolerance of transgenic soybean T₁ plants**

| **Treatment** | **Soybean genotypes** | **Average damage % 3DAT** | **Average damage % 7DAT** | **Average damage % 14DAT** | **Average damage % 21DAT** |
|---|---|---|---|---|---|
| **Blank solvent (water)** | S1 | 0 | 0 | 0 | 0 |
| | S2 | 0 | 0 | 0 | 0 |
| | S3 | 0 | 0 | 0 | 0 |
| | S4 | 0 | 0 | 0 | 0 |
| | S5 | 0 | 0 | 0 | 0 |
| | S6 | 0 | 0 | 0 | 0 |
| | S7 | 0 | 0 | 0 | 0 |
| | S8 | 0 | 0 | 0 | 0 |
| | S9 | 0 | 0 | 0 | 0 |
| | S10 | 0 | 0 | 0 | 0 |
| | S11 | 0 | 0 | 0 | 0 |
| | S12 | 0 | 0 | 0 | 0 |
| | CK1 | 0 | 0 | 0 | 0 |
| **72 g ai/ha tribenuron-methyl (4x Tri.)** | S1 | 5 | 0 | 0 | 0 |
| | S2 | 4 | 0 | 0 | 0 |
| | S3 | 0 | 0 | 0 | 0 |
| | S4 | 0 | 0 | 0 | 0 |
| | S5 | 6 | 0 | 0 | 0 |
| | S6 | 5 | 0 | 0 | 0 |
| | S7 | 0 | 0 | 0 | 0 |
| | S8 | 0 | 0 | 0 | 0 |
| | S9 | 5 | 0 | 0 | 0 |
| | S10 | 7 | 0 | 0 | 0 |
| | S11 | 0 | 0 | 0 | 0 |
| | S12 | 0 | 0 | 0 | 0 |
| | CK1 | 46 | 87 | 100 | 100 |

For soybean, 72 g ai/ha tribenuron-methyl herbicide is an effective dose distinguishing sensitive plants from plants having an average level of resistance. The results of Table 3 and Figure 11 show that: the thifensulfuron hydrolase (ALT-1, ALT-2 and ALT-3) conferred a high level of tribenuron-methyl herbicide tolerance to the transgenic soybean plants; compared to Gm cytoplasmic ALT-1-01 soybean plants, Gm cytoplasmic ALT-2-01 soybean plants and Gm cytoplasmic ALT-3-01 soybean plants, Gm chloroplastic ALT-1-01 soybean plants, Gm chloroplastic ALT-2-01 soybean plants and Gm chloroplastic ALT-3-01 soybean plants were able to produce a higher tribenuron-methyl herbicide tolerance, suggesting that the thifensulfuron hydrolase (ALT-1, ALT-2 and ALT-3) gene may enhance the tolerance of soybean plants to the tribenuron-methyl herbicide when located in the chloroplast for expression; while the wild-type soybean plants were not tolerant to the tribenuron-methyl herbicide.

### 2. Glyphosate tolerance

The herbicide tolerance effects of Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants, Gm chloroplastic ALT-3-01 soybean plants and wild-type soybean plants on glyphosate were detected (at seedling stage), respectively.

2 strains from Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants, Gm chloroplastic ALT-3-01 soybean plants and wild-type soybean plants were respectively taken each, and 10-15 plants were selected from each strain and tested. The plants above were sprayed with glyphosate (840 g ae/ha, 1-fold field concentration) and a blank solvent (water). The herbicide damage rate was measured for each plant according to the phytotoxicity symptoms 14 days after spraying (14 DAT): herbicide damage rate (%) = ∑(number of damaged plants at the same level × level number)/(total number of plants × highest level). Grading of the phytotoxicity symptoms is as shown in Table 5.

**Table 5. Grading standards of the phytotoxicity degree caused by the glyphosate herbicide to soybeans**

| Phytotoxicity level | Symptom description |
|---|---|
| 1 | growing normally, without any damage symptoms |
| 2 | mild phytotoxicity, less than 10% of phytotoxicity |
| 3 | moderate phytotoxicity, able to recover later |
| 4 | relatively severe phytotoxicity, difficult to recover |
| 5 | severe phytotoxicity, unable to recover |

The results suggested that the glyphosate herbicide damage rates of Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants and Gm chloroplastic ALT-3-01 soybean plants were substantially 0%, whereas the glyphosate herbicide damage rate of wild-type soybean plants (CK1) was up to not less than 90%; thereby, Gm cytoplasmic ALT-1-01 soybean plants, Gm chloroplastic ALT-1-01 soybean plants, Gm cytoplasmic ALT-2-01 soybean plants, Gm chloroplastic ALT-2-01 soybean plants, Gm cytoplasmic ALT-3-01 soybean plants and Gm chloroplastic ALT-3-01 soybean plants were very tolerant to the glyphosate herbicide.

### Example 9. Construction of maize recombinant expression vectors

### 1. Construction of maize recombinant cloning vectors containing ALT nucleotide sequences

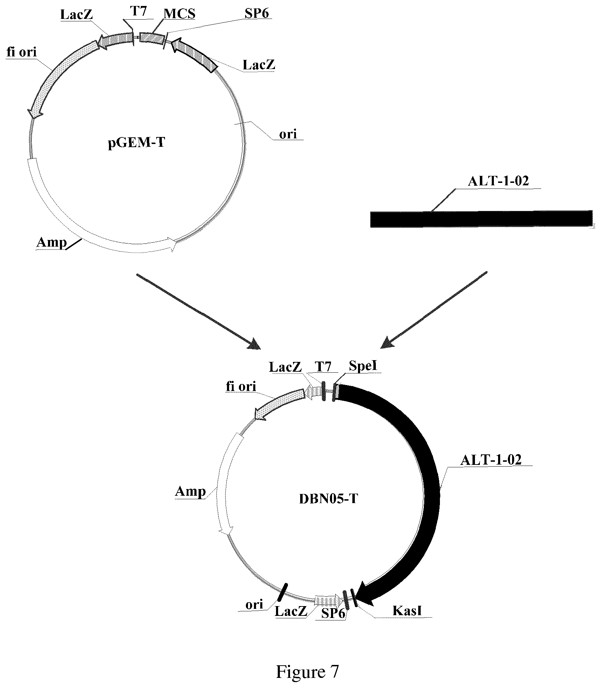

The synthetic ALT-1-02 nucleotide sequence was ligated into cloning vector pGEM-T (Promega, Madison, USA, CAT: A3600), and the operational procedure was carried out according to Promega's pGEM-T vector product instructions, obtaining a recombinant cloning vector DBN05-T, the construction process of which is as shown in Figure 7 (wherein Amp means the ampicillin resistance gene; f1 means the origin of replication of phage f1; LacZ is LacZ initiation codon; SP6 is SP6 RNA polymerase promoter; T7 is T7 RNA polymerase promoter; ALT-1-02 is the ALT-1-02 nucleotide sequence (SEQ ID NO: 3); and MCS is a multiple cloning site).

According to the method in point 1 of Example 2, *Escherichia coli* T1 competent cells were transformed with the recombinant cloning vector DBN05-T using the heat shock method, and the plasmids in the cells were extracted through the alkaline method. After identifying the extracted plasmid by *SpeI* and *Kas*I digestion, positive clones were verified by sequencing. The results showed that the inserted ALT-1-02 nucleotide sequence in the recombinant cloning vector DBN05-T was the nucleotide sequence shown as SEQ ID NO: 3 in the sequence listing, that is, the ALT-1-02 nucleotide sequence was inserted correctly.

According to the above-mentioned method for constructing the recombinant cloning vector DBN05-T, the synthetic ALT-2-02 nucleotide sequence was ligated into a cloning vector pGEM-T, obtaining a recombinant cloning vector DBN06-T, wherein ALT-2-02 was the ALT-2-02 nucleotide sequence (SEQ ID NO: 6). Enzyme digestion and sequencing verified that the ALT-2-02 nucleotide sequence was correctly inserted into the recombinant cloning vector DBN06-T.

According to the above-mentioned method for constructing the recombinant cloning vector DBN05-T, the synthetic ALT-3-02 nucleotide sequence was ligated into a cloning vector pGEM-T, obtaining a recombinant cloning vector DBN07-T, wherein ALT-3-02 was the ALT-3-02 nucleotide sequence (SEQ ID NO: 9). Enzyme digestion and sequencing verified that the ALT-3-02 nucleotide sequence was correctly inserted into the recombinant cloning vector DBN07-T.

### 2. Construction of maize recombinant expression vectors containing ALT nucleotide sequences

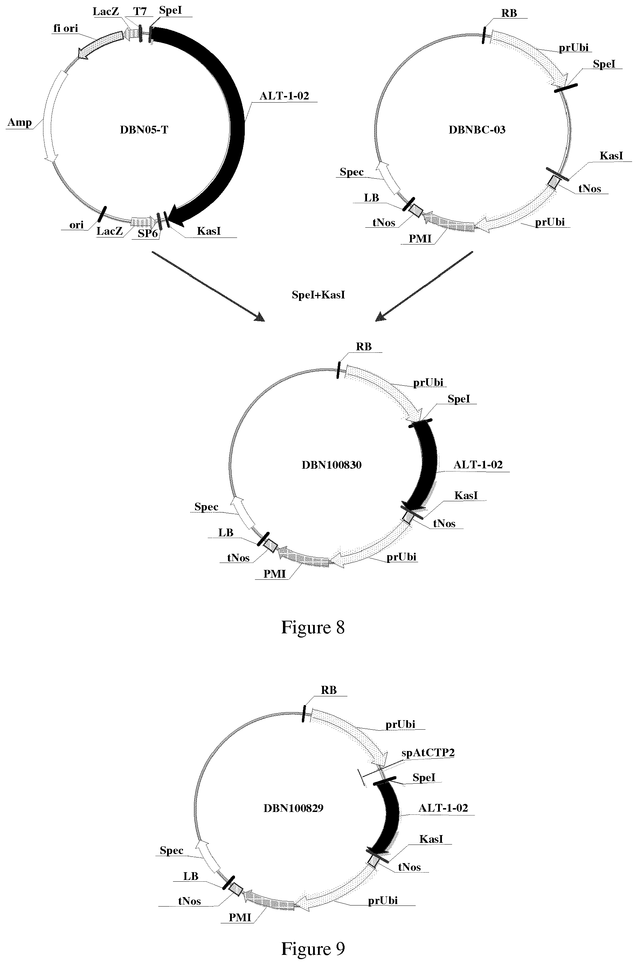

The recombinant cloning vector DBN05-T and an expression vector DBNBC-03 (vector backbone: pCAMBIA2301 (which can be provided by the CAMBIA institution)) were digested with restriction enzymes *Spe*I and *Kas*I*,* respectively; the excised ALT-1-02 nucleotide sequence fragment was inserted between the *Spe*I and *Kas*I sites in the expression vector DBNBC-03; and it is well known to a person skilled in the art to construct a vector using conventional enzyme digestion methods, constructing a recombinant expression vector DBN100830 (located in the cytoplasm), the construction process of which is as shown Figure 8 (Spec: the spectinomycin gene; RB: the right boundary; prUbi: the maize Ubiquitin 1 gene promoter (SEQ ID NO: 23); ALT-1-02: the ALT-1-02 nucleotide sequence (SEQ ID NO: 3); tNos: the terminator of nopaline synthase gene (SEQ ID NO:13); PMI: the phosphomannose isomerase gene (SEQ ID NO: 24); LB: the left boundary).

According to the method in point 2 of Example 2, *Escherichia coli* T1 competent cells were transformed with the recombinant expression vector DBN100830 using the heat shock method, and the plasmids in the cells were extracted through the alkaline method. The extracted plasmid was identified after digesting with restriction enzymes *Spe*I and *Kas*I*,* and positive clones were identified by sequencing. The results showed that the nucleotide sequence between the *SpeI* and *Kas*I sites in the recombinant expression vector DBN100830 was the nucleotide sequence shown as SEQ ID NO: 3 in the sequence listing, i.e., the ALT-1-02 nucleotide sequence.

According to the above-mentioned method for constructing the recombinant expression vector DBN100830, a recombinant expression vector DBN100829 (located in the chloroplast) containing the ALT-1-02 nucleotide sequence was constructed, the vector structure of which is as shown in Figure 9 (vector backbone: pCAMBIA2301 (which can be provided by the CAMBIA institution); Spec: the spectinomycin gene; RB: the right boundary; prUbi: the maize Ubiquitin 1 gene promoter (SEQ ID NO: 23); spAtCTP2: the *Arabidopsis thaliana* chloroplast transit peptide (SEQ ID NO: 17); ALT-1-02: the ALT-1-02 nucleotide sequence (SEQ ID NO: 3); tNos: the terminator of nopaline synthase gene (SEQ ID NO:13); PMI: the phosphomannose isomerase gene (SEQ ID NO: 24); LB: the left boundary). Positive clones were verified by sequencing. The results showed that the inserted ALT-1-02 nucleotide sequence in the recombinant expression vector DBN100829 was the nucleotide sequence shown as SEQ ID NO: 3 in the sequence listing, that is, the ALT-1-02 nucleotide sequence was inserted correctly.

According to the above-mentioned method for constructing the recombinant expression vector DBN100830, the ALT-2-02 nucleotide sequence excised by *SpeI* and *Kas*I from digested recombinant cloning vector DBN06-T was inserted into the expression vector DBNBC-03, obtaining a recombinant expression vector DBN100832. Enzyme digestion and sequencing verified that the nucleotide sequence in the recombinant expression vector DBN100832 contained the nucleotide sequence shown as SEQ ID NO: 6 in the sequence listing, that is, the ALT-2-02 nucleotide sequence was inserted correctly.

According to the above-mentioned method for constructing the recombinant expression vector DBN100829, the ALT-2-02 nucleotide sequence excised by *SpeI* and *Kas*I from digested recombinant cloning vector DBN06-T was inserted into the expression vector DBNBC-03, obtaining a recombinant expression vector DBN100831 (containing spAtCTP2, located in the chloroplast). Enzyme digestion and sequencing verified that the nucleotide sequence in the recombinant expression vector DBN100831 contained the nucleotide sequence shown as SEQ ID NO: 6 in the sequence listing, that is, the ALT-2-02 nucleotide sequence was inserted correctly.

According to the above-mentioned method for constructing the recombinant expression vector DBN100830, the ALT-3-02 nucleotide sequence excised by *SpeI* and *Kas*I from digested recombinant cloning vector DBN07-T was inserted into the expression vector DBNBC-03, obtaining a recombinant expression vector DBN100834. Enzyme digestion and sequencing verified that the nucleotide sequence in the recombinant expression vector DBN100834 contained the nucleotide sequence shown as SEQ ID NO: 9 in the sequence listing, that is, the ALT-3-02 nucleotide sequence was inserted correctly.

According to the above-mentioned method for constructing the recombinant expression vector DBN100829, the ALT-3-02 nucleotide sequence excised by *SpeI* and *Kas*I from digested recombinant cloning vector DBN07-T was inserted into the expression vector DBNBC-03, obtaining a recombinant expression vector DBN100833 (containing spAtCTP2, located in the chloroplast). Enzyme digestion and sequencing verified that the nucleotide sequence in the recombinant expression vector DBN100833 contained the nucleotide sequence shown as SEQ ID NO: 9 in the sequence listing, that is, the ALT-3-02 nucleotide sequence was inserted correctly.

### 3. Transformation of Agrobacterium with the maize recombinant expression vectors

*Agrobacterium* LBA4404 (Invitrogen, Chicago, USA, CAT: 18313-015) was transformed with the recombinant expression vectors DBN100830, DBN100829, DBN100832, DBN100831, DBN100834 and DBN100833 which had been correctly constructed using a liquid nitrogen method, with the following transformation conditions: placing 100 µL of *Agrobacterium* LBA4404, and 3 µL of plasmid DNA (recombinant expression vector) in liquid nitrogen for 10 minutes, warm water bathing at 37°C for 10 minutes; inoculating the transformed *Agrobacterium* LBA4404 into an LB tube, culturing under the conditions of a temperature of 28°C and a rotation speed of 200 rpm for 2 hours, spreading on an LB plate containing 50 mg/L of rifampicin and 50 mg/L of spectinomycin until positive single clones were grown, picking out single clones for culturing and extracting the plasmids thereof, and performing enzyme digestion verification using restriction enzymes. The results showed that the structures of the recombinant expression vectors DBN100830, DBN100829, DBN100832, DBN100831, DBN100834 and DBN100833 were completely correct.

### Example 10. Acquisition and verification of transgenic maize plants

According to the *Agrobacterium* infection method conventionally used, young embryos of a sterile culture of maize variety Zong31 (Z31) were co-cultured with the *Agrobacterium* in point 3 of Example 9, so as to introduce T-DNA (comprising the maize Ubiquitin1 gene promoter sequence, an ALT-1-02 nucleotide sequence, an ALT-2-02 nucleotide sequence , an ALT-3-02 nucleotide sequence, the *Arabidopsis thaliana* chloroplast transit peptide, the PMI gene and the tNos terminator sequence) in the recombinant expression vectors DBN100830, DBN100829, DBN100832, DBN100831, DBN100834 and DBN100833 constructed in point 2 of Example 9 into the maize chromosomes, obtaining maize plants that were transformed with the recombinant expression vector DBN100830 and had an ALT-1-02 nucleotide sequence located in the cytoplasm introduced (Zm cytoplasmic ALT-1-02) and maize plants that were transformed with the recombinant expression vector DBN100829 and had an ALT-1-02 nucleotide sequence located in the chloroplast introduced (Zm chloroplastic ALT-1-02); maize plants that were transformed with the recombinant expression vector DBN100832 and had an ALT-2-02 nucleotide sequence located in the cytoplasm introduced (Zm cytoplasmic ALT-2-02) and maize plants that were transformed with the recombinant expression vector DBN100831 and had an ALT-2-02 nucleotide sequence located in the chloroplast introduced (Zm chloroplastic ALT-2-02); maize plants that were transformed with the recombinant expression vector DBN100834 and had an ALT-3-02 nucleotide sequence located in the cytoplasm introduced (Zm cytoplasmic ALT-3-02) and maize plants that were transformed with the recombinant expression vector DBN100833 and had an ALT-3-02 nucleotide sequence located in the chloroplast introduced (Zm chloroplastic ALT-3-02); meanwhile, wild type maize plants were used as the control.

For the *Agrobacterium-mediated* maize transformation, briefly, immature young embryos were separated from maize, and contacted with an *Agrobacterium* suspension, wherein the *Agrobacterium* can transfer the ALT-1-02 nucleotide sequence, the ALT-2-02 nucleotide sequence and the ALT-3-02 nucleotide sequence to at least one cell of one of young embryos (step 1: infection step). In this step, the young embryos were preferably immersed in an *Agrobacterium* suspension (OD₆₆₀ = 0.4-0.6, an infection culture medium (4.3 g/L of MS salt, MS vitamin, 300 mg/L of casein, 68.5 g/L of sucrose, 36 g/L of glucose, 40 mg/L of acetosyringone (AS), and 1 mg/L of 2,4-dichlorphenoxyacetic acid (2,4-D), pH 5.3) to initiate the inoculation. The young embryos were co-cultured with *Agrobacterium* for a period of time (3 days) (step 2: co-culturing step). Preferably, the young embryos were cultured in a solid culture medium (4.3 g/L of MS salt, MS vitamin, 300 mg/L of casein, 20 g/L of sucrose, 10 g/L of glucose, 100 mg/L of acetosyringone (AS), 1 mg/L of 2,4-dichlorphenoxyacetic acid (2,4-D), and 8 g/L of agar, pH 5.8) after the infection step. After this co-culturing stage, there can be an optional "recovery" step. In the "recovery" step, there may be at least one antibiotic (cephalosporin) known to inhibit the growth of *Agrobacterium* in a recovery culture medium (4.3 g/L of MS salt, MS vitamin, 300 mg/L of casein, 30 g/L of sucrose, 1 mg/L of 2,4-dichlorphenoxyacetic acid (2,4-D), and 3 g/L of phytagel, pH 5.8), without the addition of a selective agent for a plant transformant (step 3: recovery step). Preferably, the young embryos were cultured in a solid culture medium with an antibiotic but without a selective agent, to eliminate *Agrobacterium* and provide a recovery stage for the infected cells. Subsequently, the inoculated young embryos were cultured in a culture medium containing a selective agent (mannose), and growing transformed calli were selected (step 4: selection step). Preferably, the young embryos were cultured in a screening solid culture medium (4.3 g/L of MS salt, MS vitamin, 300 mg/L of casein, 30 g/L of sucrose, 12.5g/L of mannose, 1 mg/L of 2,4-dichlorphenoxyacetic acid (2,4-D), and 3 g/L of phytagel, pH 5.8) with a selective agent, resulting in selective growth of transformed cells. Then, plants were regenerated from the calli (step 5: regeneration step). Preferably, the calli grown in a culture medium containing a selective agent were cultured in solid culture media (MS differentiation culture medium and MS rooting culture medium) to regenerate plants.

Resistant calli screened out were transferred onto the MS differentiation culture medium (4.3 g/L of MS salt, MS vitamin, 300 mg/L of casein, 30 g/L of sucrose, 2 mg/L of 6-benzyladenine, 5g/L of mannose, and 3 g/L of phytagel, pH 5.8), and cultured at 25°C for differentiation. The differentiated seedlings were transferred onto the MS rooting culture medium (2.15 g/L of MS salt, MS vitamin, 300 mg/L of casein, 30 g/L of sucrose, 1 mg/L of indole-3-acetic acid, and 3 g/L of phytagel, pH 5.8), cultured at 25°C to a height of about 10 cm, and transferred to a glasshouse for culturing until fruiting. In the greenhouse, the plants were cultured at 28°C for 16 hours, and then cultured at 20°C for 8 hours every day.

### 2. Verification of the transgenic maize plants using TaqMan

According to the method in point 2 of Example 7 for verifying the transgenic soybean plants using TaqMan, Zm cytoplasmic ALT-1-02 maize plants, Zm chloroplastic ALT-1-02 maize plants, Zm cytoplasmic ALT-2-02 maize plants, Zm chloroplastic ALT-2-02 maize plants, Zm cytoplasmic ALT-3-02 maize plants and Zm chloroplastic ALT-3-02 maize plants were detected and analyzed. The copy number of the *PMI* gene was detected by the Taqman probe fluorescence quantitative PCR method so as to determine the copy number of the ALT gene. Meanwhile, wild type maize plants were used as the control, and detected and analyzed according to the above-mentioned method. Triple repeats were set for the experiments, and averaged.

The following primers and probe were used to detect the *PMI* gene sequence:
primer 3: GCTGTAAGAGCTTACTGAAAAAATTAACA, shown as SEQ ID NO: 25 in the sequence listing;
primer 4: CGATCTGCAGGTCGACGG, shown as SEQ ID NO: 26 in the sequence listing;
probe 2: TCTCTTGCTAAGCTGGGAGCTCGATCC, shown as SEQ ID NO: 27 in the sequence listing.

It was further confirmed by analyzing the experimental results of the copy number of the *PMI* gene that the ALT-1-02 nucleotide sequence, the ALT-2-02 nucleotide sequence and the ALT-3-02 nucleotide sequence had all been incorporated into the chromosomes of the detected maize plants, and Zm cytoplasmic ALT-1-02 maize plants, Zm chloroplastic ALT-1-02 maize plants, Zm cytoplasmic ALT-2-02 maize plants, Zm chloroplastic ALT-2-02 maize plants, Zm cytoplasmic ALT-3-02 maize plants and Zm chloroplastic ALT-3-02 maize plants all resulted in single copy transgenic maize plants.

### Example 11. Detection of herbicide tolerance effects of the transgenic maize plants

The herbicide tolerance effects of Zm cytoplasmic ALT-1-02 maize plants, Zm chloroplastic ALT-1-02 maize plants, Zm cytoplasmic ALT-2-02 maize plants, Zm chloroplastic ALT-2-02 maize plants, Zm cytoplasmic ALT-3-02 maize plants, Zm chloroplastic ALT-3-02 maize plants and wild-type maize plants on tribenuron-methyl were detected (at V3-V4 stage), respectively.

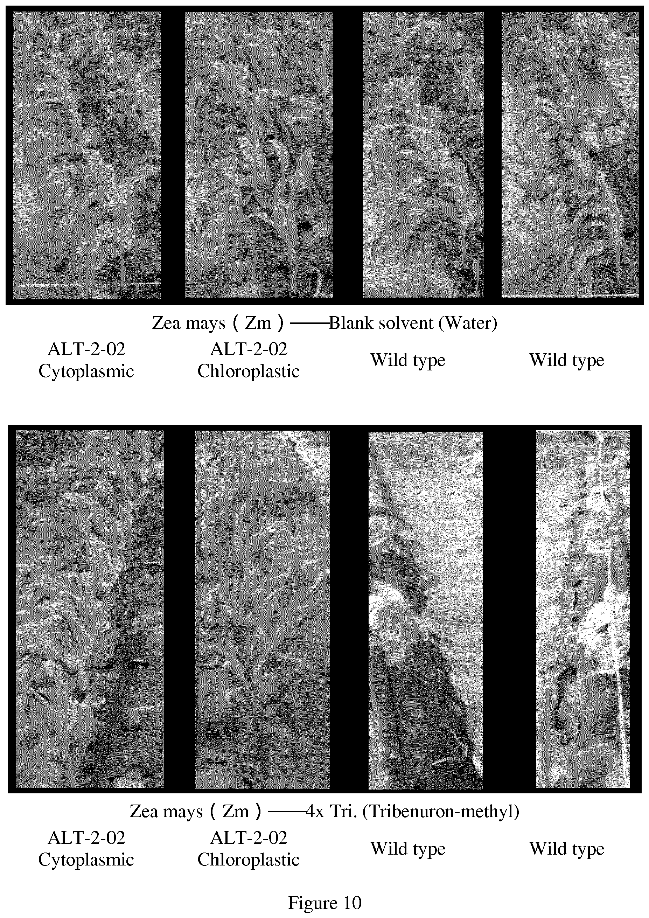

Zm cytoplasmic ALT-1-02 maize plants, Zm chloroplastic ALT-1-02 maize plants, Zm cytoplasmic ALT-2-02 maize plants, Zm chloroplastic ALT-2-02 maize plants, Zm cytoplasmic ALT-3-02 maize plants, Zm chloroplastic ALT-3-02 maize plants and wild-type maize plants were respectively taken and sprayed with tribenuron-methyl (72 g ai/ha, 4-fold field concentration) and a blank solvent (water). The damage degree caused by the herbicide was measured for each plant according to the plant growth status 3 days after spraying (3 DAT), 7 days after spraying (7 DAT), 14 days after spraying (14 DAT) and 21 days after spraying (21 DAT), respectively: considering a growth status equivalent to that of the untreated plants as having a damage degree of 0%; considering conditions of leaves being partially chlorotic and yellow but substantially not affecting the plant normal growth as having a damage degree of 50%; and considering the whole plant being purple and dying as having a damage degree of 100%. There were 2 strains in Zm cytoplasmic ALT-1-02 maize plants in total (S13 and S14), 2 strains in Zm chloroplastic ALT-1-02 maize plants in total (S15 and S16), 2 strains in Zm cytoplasmic ALT-2-02 maize plants in total (S17 and S18), 2 strains in Zm chloroplastic ALT-2-02 maize plants in total (S19 and S20), 2 strains in Zm cytoplasmic ALT-3-02 maize plants in total (S21 and S22), 2 strains in Zm chloroplastic ALT-3-02 maize plants in total (S23 and S24), and 1 strain in wild-type maize plants (CK2) in total; and 10-15 plants were selected from each strain and tested. The results are as shown in Table 4 and Figure 10.

**Table 4. Experimental results of the herbicide tolerance of transgenic maize T1 plants**

| **Treatment** | **Maize genotypes** | **Average damage % 3DAT** | **Average damage % 7DAT** | **Average damage % 14DAT** | **Average damage % 21DAT** |
|---|---|---|---|---|---|
| **Blank solvent (water)** | S13 | 0 | 0 | 0 | 0 |
| | S14 | 0 | 0 | 0 | 0 |
| | S15 | 0 | 0 | 0 | 0 |
| | S16 | 0 | 0 | 0 | 0 |
| | S17 | 0 | 0 | 0 | 0 |
| | S18 | 0 | 0 | 0 | 0 |
| | S19 | 0 | 0 | 0 | 0 |
| | S20 | 0 | 0 | 0 | 0 |
| | S21 | 0 | 0 | 0 | 0 |
| | S22 | 0 | 0 | 0 | 0 |
| | S23 | 0 | 0 | 0 | 0 |
| | S24 | 0 | 0 | 0 | 0 |
| | CK2 | 0 | 0 | 0 | 0 |
| **72 g ai/ha tribenuron-methyl (4x Tri.)** | S13 | 4 | 0 | 0 | 0 |
| | S14 | 5 | 0 | 0 | 0 |
| | S15 | 0 | 0 | 0 | 0 |
| | S16 | 0 | 0 | 0 | 0 |
| | S17 | 6 | 0 | 0 | 0 |
| | S18 | 5 | 0 | 0 | 0 |
| | S19 | 0 | 0 | 0 | 0 |
| | S20 | 0 | 0 | 0 | 0 |
| | S21 | 3 | 0 | 0 | 0 |
| | S22 | 5 | 0 | 0 | 0 |
| | S23 | 0 | 0 | 0 | 0 |
| | S24 | 0 | 0 | 0 | 0 |
| | CK2 | 46 | 86 | 100 | 100 |

For maize, 72 g ai/ha tribenuron-methyl herbicide is an effective dose distinguishing sensitive plants from plants having an average level of resistance. The results of Table 4 and Figure 10 show that: the thifensulfuron hydrolase (ALT-1, ALT-2 and ALT-3) conferred a high level of tribenuron-methyl herbicide tolerance on the transgenic maize plants; compared to Zm cytoplasmic ALT-1-02 maize plants, Zm cytoplasmic ALT-2-02 maize plants and Zm cytoplasmic ALT-3-02 maize plants, Zm chloroplastic ALT-1-02 maize plants, Zm chloroplastic ALT-2-02 maize plants and Zm chloroplastic ALT-3-02 maize plants were able to produce a higher tribenuron-methyl herbicide tolerance, suggesting that the thifensulfuron hydrolase (ALT-1, ALT-2 and ALT-3) gene may enhance the tolerance of maize plants to the tribenuron-methyl herbicide when located in the chloroplast for expression; while the wild-type maize plants were not tolerant to the tribenuron-methyl herbicide.

In summary, the present invention discloses for the first time that a thifensulfuron hydrolase (ALT-1, ALT-2 and ALT-3) can show a high tolerance to a tribenuron-methyl herbicide, *Arabidopsis thaliana* plants, soybean plants and maize plants containing nucleotide sequences encoding the thifensulfuron hydrolase are strongly tolerant to the tribenuron-methyl herbicide and can at least tolerate 1-fold field concentration, and thus the hydrolase has broad application prospects in plants.

Finally, it should be stated that the above embodiments are merely used for illustrating rather than limiting the technical solution of the present invention; and although the present invention has been described in detail with reference to the preferred embodiments, a person skilled in the art should understand that modifications or equivalent substitutions may be made to the technical solution of the present invention without departing from the spirit and scope of the technical solution of the present invention.

## Claims

1. A method for controlling weeds, **characterized in that** the method comprises applying a herbicide containing an effective dose of tribenuron-methyl to a plant growth environment where at least one transgenic plant is present, wherein the transgenic plant comprises a nucleotide sequence encoding a thifensulfuron hydrolase in its genome, and compared to other plants without the nucleotide sequence encoding the thifensulfuron hydrolase, the transgenic plant has reduced plant damage and/or an increased plant yield.

2. The method for controlling weeds according to claim 1, **characterized in that** the effective dose of tribenuron-methyl is 9-144 g ai/ha.

3. The method for controlling weeds according to claim 1 or 2, **characterized in that** the transgenic plant is a monocotyledonous plant or a dicotyledonous plant.

4. The method for controlling weeds according to claim 3, **characterized in that** the transgenic plant is maize, soybean, *Arabidopsis thaliana,* cotton, rape, rice, sorghum, wheat, barley, millet, sugar cane or oat.

5. The method for controlling weeds according to any one of claims 1-4, **characterized in that** the amino acid sequence of the thifensulfuron hydrolase comprises an amino acid sequence as shown in SEQ ID NO: 1, SEQ ID NO: 4 or SEQ ID NO: 7.

6. The method for controlling weeds according to claim 5, **characterized in that** the nucleotide sequence of the thifensulfuron hydrolase comprises:
(a) a nucleotide sequence encoding the amino acid sequence as shown in SEQ ID NO: 1, SEQ ID NO: 4 or SEQ ID NO: 7; or
(b) a nucleotide sequence as shown in SEQ ID NO: 2 or SEQ ID NO: 3; or
(c) a nucleotide sequence as shown in SEQ ID NO: 5 or SEQ ID NO: 6; or
(d) a nucleotide sequence as shown in SEQ ID NO: 8 or SEQ ID NO: 9.

7. The method for controlling weeds according to any of claims 1-6, **characterized in that** the transgenic plant may also comprise at least one second nucleotide different from the nucleotide sequence encoding the thifensulfuron hydrolase.

8. The method for controlling weeds according to claim 7, **characterized in that** the second nucleotide encodes a selectable marker protein, a protein with a synthetic activity, a protein with a decomposing activity, an anti-biostress protein, an anti-nonbiostress protein, a male sterile protein, a protein affecting a plant yield and/or a protein affecting plant quality.

9. The method for controlling weeds according to claim 8, **characterized in that** the second nucleotide encodes 5-enolpyruvylshikimate-3-phosphate synthase, glyphosate oxidoreductase, glyphosate-N-acetyltransferase, glyphosate decarboxylase, glufosinate acetyltransferase, α-ketoglutarate-dependent dioxygenase, dicamba monooxygenase, 4-hydroxyphenylpyruvate dioxygenase, acetolactate synthase, cytochrome-like proteins and/or protoporphyrinogen oxidase.

10. The method for controlling weeds according to any one of claims 1-9, **characterized in that** the herbicide containing an effective dose of tribenuron-methyl also includes glyphosate herbicides, glufosinate herbicides, auxin herbicides, graminicides, pre-emergence selective herbicides and/or post-emergence selective herbicides.

11. A method for controlling glyphosate-tolerant weeds, **characterized in that** the method comprises applying effective doses of a tribenuron-methyl herbicide and a glyphosate herbicide to a field where at least one transgenic plant is planted, wherein the field includes glyphosate-tolerant weeds or seeds thereof, the transgenic plant comprises a nucleotide sequence encoding a thifensulfuron hydrolase and a nucleotide sequence encoding a glyphosate-tolerant protein in its genome, and compared to other plants without the nucleotide sequence encoding the thifensulfuron hydrolase and/or the nucleotide sequence encoding the glyphosate-tolerant protein, the transgenic plant has reduced plant damage and/or an increased plant yield.

12. The method for controlling glyphosate-tolerant weeds according to claim 11, **characterized in that** the effective dose of tribenuron-methyl is 9-144 g ai/ha.

13. The method for controlling glyphosate-tolerant weeds according to claim 11 or 12, **characterized in that** the effective dose of glyphosate is 200-1600 g ae/ha.

14. The method for controlling glyphosate-tolerant weeds according to any of claims 11-13, **characterized in that** the transgenic plant is a monocotyledonous plant or a dicotyledonous plant.

15. The method for controlling glyphosate-tolerant weeds according to claim 14, **characterized in that** the transgenic plant is maize, soybean, *Arabidopsis thaliana,* cotton, rape, rice, sorghum, wheat, barley, millet, sugar cane or oat.

16. The method for controlling glyphosate-tolerant weeds according to any one of claims 11-15, **characterized in that** the amino acid sequence of the thifensulfuron hydrolase comprises an amino acid sequence as shown in SEQ ID NO: 1, SEQ ID NO: 4 or SEQ ID NO: 7.

17. The method for controlling glyphosate-tolerant weeds according to claim 16, **characterized in that** the nucleotide sequence of the thifensulfuron hydrolase comprises:
(a) a nucleotide sequence encoding the amino acid sequence as shown in SEQ ID NO: 1, SEQ ID NO: 4 or SEQ ID NO: 7; or
(b) a nucleotide sequence as shown in SEQ ID NO: 2 or SEQ ID NO: 3; or
(c) a nucleotide sequence as shown in SEQ ID NO: 5 or SEQ ID NO: 6; or
(d) a nucleotide sequence as shown in SEQ ID NO: 8 or SEQ ID NO: 9.

18. The method for controlling glyphosate-tolerant weeds according to any one of claims 11-17, **characterized in that** the glyphosate-tolerant protein includes 5-enolpyruvylshikimate-3-phosphate synthase, glyphosate oxidoreductase, glyphosate-N-acetyltransferase or glyphosate decarboxylase.

19. The method for controlling glyphosate-tolerant weeds according to claim 18, **characterized in that** the amino acid sequence of the glyphosate-tolerant protein comprises an amino acid sequence as shown in SEQ ID NO: 10.

20. The method for controlling glyphosate-tolerant weeds according to claim 19, **characterized in that** the nucleotide sequence of the glyphosate-tolerant protein comprises:
(a) a nucleotide sequence encoding the amino acid sequence as shown in SEQ ID NO: 10; or
(b) a nucleotide sequence as shown in SEQ ID NO: 11.

21. A planting system for controlling weeds growth, **characterized in that** the planting system comprises a tribenuron-methyl herbicide and a plant growth environment where at least one transgenic plant is present, and a herbicide containing an effective dose of tribenuron-methyl is applied to the plant growth environment where at least one transgenic plant is present, wherein the transgenic plant comprises a nucleotide sequence encoding a thifensulfuron hydrolase in its genome, and compared to other plants without the nucleotide sequence encoding the thifensulfuron hydrolase, the transgenic plant has reduced plant damage and/or an increased plant yield.

22. The planting system for controlling weeds growth according to claim 21, **characterized in that** the effective dose of tribenuron-methyl is 9-144 g ai/ha.

23. The planting system for controlling weeds growth according to claim 21 or 22, **characterized in that** the transgenic plant is a monocotyledonous plant or a dicotyledonous plant.

24. The planting system for controlling weeds growth according to claim 3, **characterized in that** the transgenic plant is maize, soybean, *Arabidopsis thaliana,* cotton, rape, rice, sorghum, wheat, barley, millet, sugar cane or oat.

25. The planting system for controlling weeds growth according to any of claims 21-24, **characterized in that** the amino acid sequence of the thifensulfuron hydrolase comprises an amino acid sequence as shown in SEQ ID NO: 1, SEQ ID NO: 4 or SEQ ID NO: 7.

26. The planting system for controlling weeds growth according to claim 25, **characterized in that** the nucleotide sequence of the thifensulfuron hydrolase comprises:
(a) a nucleotide sequence encoding the amino acid sequence as shown in SEQ ID NO: 1, SEQ ID NO: 4 or SEQ ID NO: 7; or
(b) a nucleotide sequence as shown in SEQ ID NO: 2 or SEQ ID NO: 3; or
(c) a nucleotide sequence as shown in SEQ ID NO: 5 or SEQ ID NO: 6; or
(d) a nucleotide sequence as shown in SEQ ID NO: 8 or SEQ ID NO: 9.

27. The planting system for controlling weeds growth according to any of claims 21-26, **characterized in that** the transgenic plant may also comprise at least one second nucleotide different from the nucleotide sequence encoding the thifensulfuron hydrolase.

28. The planting system for controlling weeds growth according to claim 27, **characterized in that** the second nucleotide encodes a selectable marker protein, a protein with a synthetic activity, a protein with a decomposing activity, an anti-biostress protein, an anti-nonbiostress protein, a male sterile protein, a protein affecting a plant yield and/or a protein affecting plant quality.

29. The planting system for controlling weeds growth according to claim 28, **characterized in that** the second nucleotide encodes 5-enolpyruvylshikimate-3-phosphate synthase, glyphosate oxidoreductase, glyphosate-N-acetyltransferase, glyphosate decarboxylase, glufosinate acetyltransferase, α-ketoglutarate-dependent dioxygenase, 4-hydroxyphenylpyruvate dioxygenase, acetolactate synthase, cytochrome-like proteins and/or protoporphyrinogen oxidase.

30. The planting system for controlling weeds growth according to any one of claims 21-29, **characterized in that** the herbicide containing a herbicidally effective dose of tribenuron-methyl also includes glyphosate herbicides, glufosinate herbicides, auxin herbicides, graminicides, pre-emergence selective herbicides and/or post-emergence selective herbicides.

31. A planting system for controlling glyphosate-tolerant weeds, **characterized in that** the planting system comprises a tribenuron-methyl herbicide, a glyphosate herbicide and a field where at least one transgenic plant is planted, and effective doses of the tribenuron-methyl herbicide and the glyphosate herbicide are applied to the field where at least one transgenic plant is planted, wherein the field includes glyphosate-tolerant weeds or seeds thereof, the transgenic plant comprises a nucleotide sequence encoding a thifensulfuron hydrolase and a nucleotide sequence encoding a glyphosate-tolerant protein in its genome, and compared to other plants without the nucleotide sequence encoding the thifensulfuron hydrolase and/or the nucleotide sequence encoding the glyphosate-tolerant protein, the transgenic plant has reduced plant damage and/or an increased plant yield.

32. The planting system for controlling glyphosate-tolerant weeds according to claim 31, **characterized in that** the effective dose of tribenuron-methyl is 9-144 g ai/ha.

33. The planting system for controlling glyphosate-tolerant weeds according to claim 31 or 32, **characterized in that** the effective dose of glyphosate is 200-1600 g ae/ha.

34. The planting system for controlling glyphosate-tolerant weeds according to any one of claims 31-33, **characterized in that** the transgenic plant is a monocotyledonous plant or a dicotyledonous plant.

35. The planting system for controlling glyphosate-tolerant weeds according to claim 34, **characterized in that** the transgenic plant is maize, soybean, *Arabidopsis thaliana,* cotton, rape, rice, sorghum, wheat, barley, millet, sugar cane or oat.

36. The planting system for controlling glyphosate-tolerant weeds according to any one of claims 31-35, **characterized in that** the amino acid sequence of the thifensulfuron hydrolase comprises an amino acid sequence as shown in SEQ ID NO: 1, SEQ ID NO: 4 or SEQ ID NO: 7.

37. The planting system for controlling glyphosate-tolerant weeds according to claim 36, **characterized in that** the nucleotide sequence of the thifensulfuron hydrolase comprises:
(a) a nucleotide sequence encoding the amino acid sequence as shown in SEQ ID NO: 1, SEQ ID NO: 4 or SEQ ID NO: 7; or
(b) a nucleotide sequence as shown in SEQ ID NO: 2 or SEQ ID NO: 3; or
(c) a nucleotide sequence as shown in SEQ ID NO: 5 or SEQ ID NO: 6; or
(d) a nucleotide sequence as shown in SEQ ID NO: 8 or SEQ ID NO: 9.

38. The planting system for controlling glyphosate-tolerant weeds according to any one of claims 31-37, **characterized in that** the glyphosate-tolerant protein includes 5-enolpyruvylshikimate-3-phosphate synthase, glyphosate oxidoreductase, glyphosate-N-acetyltransferase or glyphosate decarboxylase.

39. The planting system for controlling glyphosate-tolerant weeds according to claim 38, **characterized in that** the amino acid sequence of the glyphosate-tolerant protein comprises an amino acid sequence as shown in SEQ ID NO: 10.

40. The planting system for controlling glyphosate-tolerant weeds according to claim 39, **characterized in that** the nucleotide sequence of the glyphosate-tolerant protein comprises:
(a) a nucleotide sequence encoding the amino acid sequence as shown in SEQ ID NO: 10; or
(b) a nucleotide sequence as shown in SEQ ID NO: 11.

41. A method for producing a plant tolerant to a tribenuron-methyl herbicide, **characterized in that** the method comprises introducing a nucleotide sequence encoding a thifensulfuron hydrolase into the genome of a plant, wherein when a herbicide containing an effective dose of tribenuron-methyl is applied to a field where at least the plant is present, the plant has reduced plant damage and/or an increased plant yield compared to other plants without the nucleotide sequence encoding the thifensulfuron hydrolase.

42. A method for cultivating a plant tolerant to a tribenuron-methyl herbicide, **characterized in that** the method comprises:
planting at least one plant propagule, wherein the plant propagule comprises a polynucleotide sequence encoding a thifensulfuron hydrolase in its genome;
growing the plant propagule into a plant;
and applying a herbicide containing an effective dose of tribenuron-methyl to a plant growth environment where at least the plant is included and harvesting the plant having reduced plant damage and/or an increased plant yield compared to other plants without the polynucleotide sequence encoding the thifensulfuron hydrolase.

43. A method for protecting a plant from damage caused by a tribenuron-methyl herbicide, **characterized in that** the method comprises applying a herbicide containing an effective dose of tribenuron-methyl to a plant growth environment where at least one transgenic plant is present, wherein the transgenic plant comprises a nucleotide sequence encoding a thifensulfuron hydrolase in its genome, and compared to other plants without the nucleotide sequence encoding the thifensulfuron hydrolase, the transgenic plant has reduced plant damage and/or an increased plant yield.

44. A method for degrading a tribenuron-methyl herbicide with a thifensulfuron hydrolase, **characterized in that** the method comprises applying a herbicide containing an effective dose of tribenuron-methyl to a plant growth environment where at least one transgenic plant is present, wherein the transgenic plant comprises a nucleotide sequence encoding the thifensulfuron hydrolase in its genome, and compared to other plants without the nucleotide sequence encoding the thifensulfuron hydrolase, the transgenic plant has reduced plant damage and/or an increased plant yield.

45. Use of a thifensulfuron hydrolase to degrade a tribenuron-methyl herbicide.

46. The use of a thifensulfuron hydrolase to degrade a tribenuron-methyl herbicide according to claim 45, **characterized in that** the use of the thifensulfuron hydrolase to degrade a tribenuron-methyl herbicide comprises applying a herbicide containing an effective dose of tribenuron-methyl to a plant growth environment where at least one transgenic plant is present, wherein the transgenic plant comprises a nucleotide sequence encoding the thifensulfuron hydrolase in its genome, and compared to other plants without the nucleotide sequence encoding the thifensulfuron hydrolase, the transgenic plant has reduced plant damage and/or an increased plant yield.

47. The method or use according to any one of claims 41-46, **characterized in that** the amino acid sequence of the thifensulfuron hydrolase comprises an amino acid sequence as shown in SEQ ID NO: 1, SEQ ID NO: 4 or SEQ ID NO: 7.

48. The method or use according to claim 47, **characterized in that** the nucleotide sequence of the thifensulfuron hydrolase comprises:
(a) a nucleotide sequence encoding the amino acid sequence as shown in SEQ ID NO: 1, SEQ ID NO: 4 or SEQ ID NO: 7; or
(b) a nucleotide sequence as shown in SEQ ID NO: 2 or SEQ ID NO: 3; or
(c) a nucleotide sequence as shown in SEQ ID NO: 5 or SEQ ID NO: 6; or
(d) a nucleotide sequence as shown in SEQ ID NO: 8 or SEQ ID NO: 9.


SEQUENCE LISTING

<110>  BEIJING DABEINONG TECHNOLOGY GROUP CO., LTD.
       BEIJING DABEINONG BIOTECHNOLOGY CO., LTD.

<120>  Use of Herbicide-tolerant Protein

<130>  DBNBC93

<160>  27

<170>  PatentIn version 3.5

<210>  1
<211>  398
<212>  PRT
<213>  Methylophilus sp.

<400>  1

Met Glu Thr Asp Lys Lys Thr Gly Thr Ser Arg Arg Ser Phe Val Lys
1               5                   10                  15

Ala Ala Gly Thr Gly Ala Ile Gly Ile Ala Thr Leu Pro Leu Ser Thr
            20                  25                  30

Ala Thr Ala Phe Ala Glu Thr Asp Asn Val Glu Leu Ala Gln Ser Lys
        35                  40                  45

Arg Lys Val Val Leu Ala Glu Gln Gly Ser Phe Tyr Ile Gly Gly Arg
    50                  55                  60

Thr Val Thr Gly Pro Gly Lys Phe Asp Pro Ser Lys Pro Val Ile Pro
65                  70                  75                  80

Tyr Ser Asn Glu Gly Ala Thr Phe Tyr Ile Asn Gln Met Tyr Val Asn
                85                  90                  95

Phe Gln Ala Pro Val Arg Pro Arg Gly Leu Pro Leu Val Phe Trp His
            100                 105                 110

Gly Gly Gly Leu Thr Gly His Ile Trp Glu Ser Thr Pro Asp Gly Arg
        115                 120                 125

Pro Gly Phe Gln Thr Leu Phe Val Gln Asp Arg His Thr Val Tyr Thr
    130                 135                 140

Ile Asp Gln Pro Gly Arg Gly Arg Gly Asn Ile Pro Thr Phe Asn Gly
145                 150                 155                 160

Pro Phe Gly Gln Leu Glu Glu Glu Ser Ile Val Asn Thr Val Thr Gly
                165                 170                 175

Asn Ser Ser Lys Glu Gly Ala Trp Val Arg Asp Arg Leu Gly Pro Ala
        180                 185                 190

Pro Gly Gln Phe Phe Glu Asn Ser Gln Phe Pro Arg Gly Tyr Glu Asp
    195                 200                 205

Asn Tyr Phe Lys Glu Met Gly Phe Ser Pro Ser Ile Ser Ser Asp Glu
    210                 215                 220

Ile Val Asp Ala Val Val Lys Leu Val Thr His Ile Gly Pro Cys Val
225                 230                 235                 240

Leu Val Thr His Ser Ala Ser Gly Val Leu Gly Met Arg Val Ala Thr
                245                 250                 255

His Ala Lys Asn Val Arg Gly Ile Val Ala Tyr Glu Pro Ala Thr Ser
            260                 265                 270

Ile Phe Pro Lys Gly Lys Val Pro Glu Ile Pro Pro Leu Ala Asp Lys
        275                 280                 285

Lys Ser Gln Ile Phe Pro Pro Phe Glu Ile Gln Glu Ser Tyr Phe Lys
    290                 295                 300

Lys Leu Ala Lys Ile Pro Ile Gln Phe Val Phe Gly Asp Asn Ile Pro
305                 310                 315                 320

Lys Asn Pro Lys Ser Ala Tyr Trp Phe Leu Asp Trp Trp Arg Val Thr
                325                 330                 335

Arg Tyr Ala His Ser Leu Ser Leu Glu Ala Ile Asn Lys Leu Gly Gly
            340                 345                 350

Gln Ala Ser Leu Leu Asp Leu Pro Thr Ala Gly Leu Arg Gly Asn Thr
        355                 360                 365

His Phe Pro Phe Thr Asp Arg Asn Asn Val Gln Val Ala Ser Leu Leu
    370                 375                 380

Ser Asp Phe Leu Gly Lys His Gly Leu Asp Gln Asn Glu Ser
385                 390                 395

<210>  2
<211>  1197
<212>  DNA
<213>  Artificial Sequence

<220>

<223>  ALT-1-01 Nucleotide sequence

<400>  2
atggagactg ataagaaaac tggcacatct agaaggtcat ttgttaaggc tgcaggaaca      60

ggtgccattg gaatcgctac ccttccattg tccaccgcca ctgctttcgc agagactgat     120

aacgtggaac tcgcccaatc taagagaaag gtggtgctgg ctgaacaagg gtcattttac     180

atagggggta ggactgttac tggtcctggc aagtttgatc catccaaacc tgtgataccc     240

tacagtaacg aaggagcaac attctatatt aaccaaatgt atgttaactt ccaggcccca     300

gtgagaccta ggggacttcc attggttttc tggcatggag gtggcttgac tggtcacatc     360

tgggagtcta cacctgacgg cagacccggg tttcaaaccc tcttcgttca ggataggcat     420

accgtgtaca ctattgacca acctgggaga ggaaggggta acatcccaac ttttaacgga     480

cctttcggac agttggagga agagagtatt gttaacactg tgacaggaaa ttcttcaaag     540

gaaggtgcct gggtgagaga taggcttggc cctgctcccg ggcaattttt cgagaactct     600

cagtttccta gaggctatga agacaattac tttaaggaga tgggattcag cccatctata     660

tccagtgatg aaattgttga cgctgttgtg aaactcgtga cccatattgg tccttgtgtt     720

ctggtgactc actcagcatc cggcgttctt gggatgagag tggctacaca cgcaaagaat     780

gttaggggaa ttgtggccta tgaaccagct acctcaatct tccccaaggg aaaagttcca     840

gagataccac ctctcgctga taagaaaagc caaatctttc ccccattcga aatacaggag     900

tcttacttta agaaacttgc caagattcca atccaatttg ttttcggaga taacatcccc     960

aagaatccaa aatcagcata ttggttcctg gactggtgga gagtgacaag atacgcacat    1020

agtctcagcc tggaggccat aaacaaattg gggggacaag cttccctttt ggatcttcct    1080

actgcaggat tgagaggtaa tacacacttt cccttcaccg ataggaacaa tgttcaggtg    1140

gcttctctcc tgtcagactt tctgggtaaa cacggtctgg atcaaaatga gagctga       1197


<210>  3
<211>  1197
<212>  DNA
<213>  Artificial Sequence

<220>
<223>  ALT-1-02 Nucleotide sequence

<400>  3
atggagacag acaagaagac cggcacgtcc cgcaggagct tcgtgaaggc tgctggcacc      60

ggggctatcg ggattgctac gctcccactg tcgacagcta ctgccttcgc ggagactgat     120

aacgtggagc tggcgcagag caagaggaag gtggttctgg ctgagcaggg gtcgttctac     180

attggggggc ggactgtgac cgggcccggc aagttcgacc catcgaagcc tgtcattccg     240

tactctaacg agggcgctac gttctacatc aaccagatgt acgtgaattt ccaggctccc     300

gtccgcccaa ggggcctccc actggtgttc tggcacggcg ggggcctgac aggccatatc 360

tgggagtcca ctccagatgg ccgcccaggg ttccagacac tcttcgttca ggacaggcac 420

acagtgtaca ctattgatca gccagggagg ggcaggggga acatccctac cttcaatggc 480

ccattcgggc agctggagga ggagtccatc gtgaacaccg tcacgggcaa ttccagcaag 540

gagggggctt gggtcaggga ccggctcggc ccggccccag ggcagttctt cgagaactct 600

cagttccccc ggggctacga ggataattac ttcaaggaga tgggcttctc accatccatc 660

tcgtctgacg agattgtcga tgccgtggtc aagctcgtta cccacatcgg cccttgcgtt 720

ctggtgacgc atagcgcttc gggcgtcctc gggatgaggg ttgctacaca tgcgaagaac 780

gttcgcggca tcgtggctta cgagccggcc acttccattt tccccaaggg caaggtgcca 840

gagatcccac cactggccga caagaagtca cagatcttcc cacctttcga gattcaggag 900

tcctacttca agaagctcgc taagatcccc attcagttcg tgttcggcga caacattcct 960

aagaatccga agagcgcgta ctggttcctg gattggtggc gcgtcacgcg ctacgcgcac 1020

tctctctcac tggaggctat caacaagctc gggggccagg cctcgctcct ggacctccct 1080

accgctggcc tgagggggaa cacccatttc ccgttcacgg atcggaacaa tgtccaggtt 1140

gcgtccctcc tgagcgattt cctcggcaag cacgggctgg atcagaatga gtcttga 1197

<210> 4
<211> 369
<212> PRT
<213> Artificial Sequence

<220>
<223> ALT-2 Amino acid sequence

<400> 4

Met Glu Thr Asp Asn Val Glu Leu Ala Gln Ser Lys Arg Lys Val Val
1               5                   10                  15

Leu Ala Glu Gln Gly Ser Phe Tyr Ile Gly Gly Arg Thr Val Thr Gly
            20                  25                  30

Pro Gly Lys Phe Asp Pro Ser Lys Pro Val Ile Pro Tyr Ser Asn Glu
        35                  40                  45

Gly Ala Thr Phe Tyr Ile Asn Gln Met Tyr Val Asn Phe Gln Ala Pro
    50                  55                  60

Val Arg Pro Arg Gly Leu Pro Leu Val Phe Trp His Gly Gly Gly Leu
65                  70                  75                  80

Thr Gly His Ile Trp Glu Ser Thr Pro Asp Gly Arg Pro Gly Phe Gln
                85                  90                  95

Thr Leu Phe Val Gln Asp Arg His Thr Val Tyr Thr Ile Asp Gln Pro
                100                 105                 110

Gly Arg Gly Arg Gly Asn Ile Pro Thr Phe Asn Gly Pro Phe Gly Gln
        115                 120                 125

Leu Glu Glu Glu Ser Ile Val Asn Thr Val Thr Gly Asn Ser Ser Lys
    130                 135                 140

Glu Gly Ala Trp Val Arg Asp Arg Leu Gly Pro Ala Pro Gly Gln Phe
145                 150                 155                 160

Phe Glu Asn Ser Gln Phe Pro Arg Gly Tyr Glu Asp Asn Tyr Phe Lys
                165                 170                 175

Glu Met Gly Phe Ser Pro Ser Ile Ser Ser Asp Glu Ile Val Asp Ala
            180                 185                 190

Val Val Lys Leu Val Thr His Ile Gly Pro Cys Val Leu Val Thr His
        195                 200                 205

Ser Ala Ser Gly Val Leu Gly Met Arg Val Ala Thr His Ala Lys Asn
    210                 215                 220

Val Arg Gly Ile Val Ala Tyr Glu Pro Ala Thr Ser Ile Phe Pro Lys
225                 230                 235                 240

Gly Lys Val Pro Glu Ile Pro Pro Leu Ala Asp Lys Lys Ser Gln Ile
                245                 250                 255

Phe Pro Pro Phe Glu Ile Gln Glu Ser Tyr Phe Lys Lys Leu Ala Lys
            260                 265                 270

Ile Pro Ile Gln Phe Val Phe Gly Asp Asn Ile Pro Lys Asn Pro Lys
        275                 280                 285

Ser Ala Tyr Trp Phe Leu Asp Trp Trp Arg Val Thr Arg Tyr Ala His
    290                 295                 300

Ser Leu Ser Leu Glu Ala Ile Asn Lys Leu Gly Gly Gln Ala Ser Leu
305                 310                 315                 320

Leu Asp Leu Pro Thr Ala Gly Leu Arg Gly Asn Thr His Phe Pro Phe
                325                 330                 335

Thr Asp Arg Asn Asn Val Gln Val Ala Ser Leu Leu Ser Asp Phe Leu

340                 345                 350

Gly Lys His Gly Leu Asp Gln Asn Glu Ser Lys Leu Ala Ala Ala Leu
        355                 360                 365

Glu

<210>  5
<211>  1110
<212>  DNA
<213>  Artificial Sequence

<220>
<223>  ALT-2-01 Nucleotide sequence

<400>  5
atggagactg ataacgtgga actcgcccaa tctaagagaa aggtggtgct ggctgaacaa      60

gggtcatttt acataggggg taggactgtt actggtcctg gcaagtttga tccatccaaa     120

cctgtgatac cctacagtaa cgaaggagca acattctata ttaaccaaat gtatgttaac     180

ttccaggccc cagtgagacc taggggactt ccattggttt tctggcatgg aggtggcttg     240

actggtcaca tctgggagtc tacacctgac ggcagacccg ggtttcaaac cctcttcgtt     300

caggataggc ataccgtgta cactattgac caacctggga gaggaagggg taacatccca     360

acttttaacg gacctttcgg acagttggag gaagagagta ttgttaacac tgtgacagga     420

aattcttcaa aggaaggtgc ctgggtgaga gataggcttg gccctgctcc cgggcaattt     480

ttcgagaact ctcagtttcc tagaggctat gaagacaatt actttaagga gatgggattc     540

agcccatcta tatccagtga tgaaattgtt gacgctgttg tgaaactcgt gacccatatt     600

ggtccttgtg ttctggtgac tcactcagca tccggcgttc ttgggatgag agtggctaca     660

cacgcaaaga atgttagggg aattgtggcc tatgaaccag ctacctcaat cttccccaag     720

ggaaaagttc cagagatacc acctctcgct gataagaaaa gccaaatctt tcccccattc     780

gaaatacagg agtcttactt taagaaactt gccaagattc caatccaatt tgttttcgga     840

gataacatcc ccaagaatcc aaaatcagca tattggttcc tggactggtg gagagtgaca     900

agatacgcac atagtctcag cctggaggcc ataaacaaat tgggggacaa agcttccctt     960

ttggatcttc ctactgcagg attgagaggt aatacacact ttcccttcac cgataggaac    1020

aatgttcagg tggcttctct cctgtcagac tttctgggta aacacggtct ggatcaaaat    1080

gagagcaaac tcgccgccgc cctggaatga                                     1110

<210>  6
<211>  1110
<212>  DNA
<213>  Artificial Sequence

<220>
<223>  ALT-2-02 Nucleotide sequence

<400>  6
atggagactg ataacgtgga gctggcgcag agcaagagga aggtggttct ggctgagcag      60

gggtcgttct acattggggg gcggactgtg accgggcccg gcaagttcga cccatcgaag     120

cctgtcattc cgtactctaa cgagggcgct acgttctaca tcaaccagat gtacgtgaat     180

ttccaggctc ccgtccgccc aaggggcctc ccactggtgt tctggcacgg cgggggcctg     240

acaggccata tctgggagtc cactccagat ggccgcccag ggttccagac actcttcgtt     300

caggacaggc acacagtgta cactattgat cagccaggga ggggcagggg gaacatccct     360

accttcaatg gcccattcgg gcagctggag gaggagtcca tcgtgaacac cgtcacgggc     420

aattccagca aggagggggc ttgggtcagg gaccggctcg gcccggcccc agggcagttc     480

ttcgagaact ctcagttccc ccggggctac gaggataatt acttcaagga gatgggcttc     540

tcaccatcca tctcgtctga cgagattgtc gatgccgtgg tcaagctcgt tacccacatc     600

ggcccttgcg ttctggtgac gcatagcgct tcgggcgtcc tcgggatgag ggttgctaca     660

catgcgaaga acgttcgcgg catcgtggct tacgagccgg ccacttccat tttccccaag     720

ggcaaggtgc cagagatccc accactggcc gacaagaagt cacagatctt cccacctttc     780

gagattcagg agtcctactt caagaagctc gctaagatcc ccattcagtt cgtgttcggc     840

gacaacattc ctaagaatcc gaagagcgcg tactggttcc tggattggtg gcgcgtcacg     900

cgctacgcgc actctctctc actggaggct atcaacaagc tcgggggcca ggcctcgctc     960

ctggacctcc ctaccgctgg cctgaggggg aacacccatt tcccgttcac ggatcggaac    1020

aatgtccagg ttgcgtccct cctgagcgat ttcctcggca agcacgggct ggatcagaat    1080

gagtctaagc tcgctgcggc gctggagtga                                    1110


<210>  7
<211>  362
<212>  PRT
<213>  Artificial Sequence

<220>
<223>  ALT-3 Amino acid sequence

<400>  7

Met Glu Thr Asp Asn Val Glu Leu Ala Gln Ser Lys Arg Lys Val Val
1               5                   10                  15


Leu Ala Glu Gln Gly Ser Phe Tyr Ile Gly Gly Arg Thr Val Thr Gly
            20                  25                  30


Pro Gly Lys Phe Asp Pro Ser Lys Pro Val Ile Pro Tyr Ser Asn Glu

35                  40                  45

Gly Ala Thr Phe Tyr Ile Asn Gln Met Tyr Val Asn Phe Gln Ala Pro
    50                  55                  60

Val Arg Pro Arg Gly Leu Pro Leu Val Phe Trp His Gly Gly Gly Leu
65                  70                  75                  80

Thr Gly His Ile Trp Glu Ser Thr Pro Asp Gly Arg Pro Gly Phe Gln
                85                  90                  95

Thr Leu Phe Val Gln Asp Arg His Thr Val Tyr Thr Ile Asp Gln Pro
            100                 105                 110

Gly Arg Gly Arg Gly Asn Ile Pro Thr Phe Asn Gly Pro Phe Gly Gln
        115                 120                 125

Leu Glu Glu Glu Ser Ile Val Asn Thr Val Thr Gly Asn Ser Ser Lys
    130                 135                 140

Glu Gly Ala Trp Val Arg Asp Arg Leu Gly Pro Ala Pro Gly Gln Phe
145                 150                 155                 160

Phe Glu Asn Ser Gln Phe Pro Arg Gly Tyr Glu Asp Asn Tyr Phe Lys
                165                 170                 175

Glu Met Gly Phe Ser Pro Ser Ile Ser Ser Asp Glu Ile Val Asp Ala
            180                 185                 190

Val Val Lys Leu Val Thr His Ile Gly Pro Cys Val Leu Val Thr His
        195                 200                 205

Ser Ala Ser Gly Val Leu Gly Met Arg Val Ala Thr His Ala Lys Asn
    210                 215                 220

Val Arg Gly Ile Val Ala Tyr Glu Pro Ala Thr Ser Ile Phe Pro Lys
225                 230                 235                 240

Gly Lys Val Pro Glu Ile Pro Pro Leu Ala Asp Lys Lys Ser Gln Ile
                245                 250                 255

Phe Pro Pro Phe Glu Ile Gln Glu Ser Tyr Phe Lys Lys Leu Ala Lys
            260                 265                 270

Ile Pro Ile Gln Phe Val Phe Gly Asp Asn Ile Pro Lys Asn Pro Lys
        275                 280                 285

Ser Ala Tyr Trp Phe Leu Asp Trp Trp Arg Val Thr Arg Tyr Ala His
    290                 295                 300

Ser Leu Ser Leu Glu Ala Ile Asn Lys Leu Gly Gly Gln Ala Ser Leu
305                 310                 315                 320

Leu Asp Leu Pro Thr Ala Gly Leu Arg Gly Asn Thr His Phe Pro Phe
                325                 330                 335

Thr Asp Arg Asn Asn Val Gln Val Ala Ser Leu Leu Ser Asp Phe Leu
            340                 345                 350

Gly Lys His Gly Leu Asp Gln Asn Glu Ser
        355                 360

<210>  8
<211>  1089
<212>  DNA
<213>  Artificial Sequence

<220>
<223>  ALT-3-01 Nucleotide sequence

<400>  8
atggagactg ataacgtgga actcgcccaa tctaagagaa aggtggtgct ggctgaacaa      60

gggtcatttt acataggggg taggactgtt actggtcctg gcaagtttga tccatccaaa     120

cctgtgatac cctacagtaa cgaaggagca acattctata ttaaccaaat gtatgttaac     180

ttccaggccc cagtgagacc taggggactt ccattggttt tctggcatgg aggtggcttg     240

actggtcaca tctgggagtc tacacctgac ggcagacccg ggtttcaaac cctcttcgtt     300

caggataggc ataccgtgta cactattgac caacctggga gaggaagggg taacatccca     360

acttttaacg gacctttcgg acagttggag gaagagagta ttgttaacac tgtgacagga     420

aattcttcaa aggaaggtgc ctgggtgaga gataggcttg gccctgctcc cgggcaattt     480

ttcgagaact ctcagtttcc tagaggctat gaagacaatt actttaagga gatgggattc     540

agcccatcta tatccagtga tgaaattgtt gacgctgttg tgaaactcgt gacccatatt     600

ggtccttgtg ttctggtgac tcactcagca tccggcgttc ttgggatgag agtggctaca     660

cacgcaaaga atgttagggg aattgtggcc tatgaaccag ctacctcaat cttccccaag     720

ggaaaagttc cagagatacc acctctcgct gataagaaaa gccaaatctt tcccccattc     780

gaaatacagg agtcttactt taagaaactt gccaagattc caatccaatt tgttttcgga     840

gataacatcc ccaagaatcc aaaatcagca tattggttcc tggactggtg gagagtgaca     900

agatacgcac atagtctcag cctggaggcc ataaacaaat tgggggacaa agcttccctt     960

ttggatcttc ctactgcagg attgagaggt aatacacact ttcccttcac cgataggaac    1020

aatgttcagg tggcttctct cctgtcagac tttctgggta aacacggtct ggatcaaaat 1080

gagagctga 1089

<210> 9
<211> 1089
<212> DNA
<213> Artificial Sequence

<220>
<223> ALT-3-02 Nucleotide sequence

<400> 9
atggagactg ataacgtgga gctggcgcag agcaagagga aggtggttct ggctgagcag 60

gggtcgttct acattggggg gcggactgtg accgggcccg gcaagttcga cccatcgaag 120

cctgtcattc cgtactctaa cgagggcgct acgttctaca tcaaccagat gtacgtgaat 180

ttccaggctc ccgtccgccc aaggggcctc ccactggtgt tctggcacgg cgggggcctg 240

acaggccata tctgggagtc cactccagat ggccgcccag ggttccagac actcttcgtt 300

caggacaggc acacagtgta cactattgat cagccaggga ggggcagggg gaacatccct 360

accttcaatg gcccattcgg gcagctggag gaggagtcca tcgtgaacac cgtcacgggc 420

aattccagca aggagggggc ttgggtcagg gaccggctcg gcccggcccc agggcagttc 480

ttcgagaact ctcagttccc ccggggctac gaggataatt acttcaagga gatgggcttc 540

tcaccatcca tctcgtctga cgagattgtc gatgccgtgg tcaagctcgt tacccacatc 600

ggcccttgcg ttctggtgac gcatagcgct tcgggcgtcc tcgggatgag ggttgctaca 660

catgcgaaga acgttcgcgg catcgtggct tacgagccgg ccacttccat tttccccaag 720

ggcaaggtgc cagagatccc accactggcc gacaagaagt cacagatctt cccacctttc 780

gagattcagg agtcctactt caagaagctc gctaagatcc ccattcagtt cgtgttcggc 840

gacaacattc ctaagaatcc gaagagcgcg tactggttcc tggattggtg gcgcgtcacg 900

cgctacgcgc actctctctc actggaggct atcaacaagc tcgggggcca ggcctcgctc 960

ctggacctcc ctaccgctgg cctgaggggg aacacccatt tcccgttcac ggatcggaac 1020

aatgtccagg ttgcgtccct cctgagcgat ttcctcggca agcacgggct ggatcagaat 1080

gagtcttga 1089

<210> 10
<211> 455
<212> PRT
<213> Artificial Sequence

<220>
<223> Amino acid sequence of Herbicide-tolerant Protein
<400> 10

Met Leu His Gly Ala Ser Ser Arg Pro Ala Thr Ala Arg Lys Ser Ser

1               5                   10                  15

Gly Leu Ser Gly Thr Val Arg Ile Pro Gly Asp Lys Ser Ile Ser His
            20                  25                  30

Arg Ser Phe Met Phe Gly Gly Leu Ala Ser Gly Glu Thr Arg Ile Thr
        35                  40                  45

Gly Leu Leu Glu Gly Glu Asp Val Ile Asn Thr Gly Lys Ala Met Gln
    50                  55                  60

Ala Met Gly Ala Arg Ile Arg Lys Glu Gly Asp Thr Trp Ile Ile Asp
65                  70                  75                  80

Gly Val Gly Asn Gly Gly Leu Leu Ala Pro Glu Ala Pro Leu Asp Phe
                85                  90                  95

Gly Asn Ala Ala Thr Gly Cys Arg Leu Thr Met Gly Leu Val Gly Val
            100                 105                 110

Tyr Asp Phe Asp Ser Thr Phe Ile Gly Asp Ala Ser Leu Thr Lys Arg
        115                 120                 125

Pro Met Gly Arg Val Leu Asn Pro Leu Arg Glu Met Gly Val Gln Val
    130                 135                 140

Lys Ser Glu Asp Gly Asp Arg Leu Pro Val Thr Leu Arg Gly Pro Lys
145                 150                 155                 160

Thr Pro Thr Pro Ile Thr Tyr Arg Val Pro Met Ala Ser Ala Gln Val
                165                 170                 175

Lys Ser Ala Val Leu Leu Ala Gly Leu Asn Thr Pro Gly Ile Thr Thr
            180                 185                 190

Val Ile Glu Pro Ile Met Thr Arg Asp His Thr Glu Lys Met Leu Gln
        195                 200                 205

Gly Phe Gly Ala Asn Leu Thr Val Glu Thr Asp Ala Asp Gly Val Arg
    210                 215                 220

Thr Ile Arg Leu Glu Gly Arg Gly Lys Leu Thr Gly Gln Val Ile Asp
225                 230                 235                 240

Val Pro Gly Asp Pro Ser Ser Thr Ala Phe Pro Leu Val Ala Ala Leu
                245                 250                 255

Leu Val Pro Gly Ser Asp Val Thr Ile Leu Asn Val Leu Met Asn Pro
            260                 265                 270

Thr Arg Thr Gly Leu Ile Leu Thr Leu Gln Glu Met Gly Ala Asp Ile
        275                 280                 285

Glu Val Ile Asn Pro Arg Leu Ala Gly Gly Glu Asp Val Ala Asp Leu
    290                 295                 300

Arg Val Arg Ser Ser Thr Leu Lys Gly Val Thr Val Pro Glu Asp Arg
305                 310                 315                 320

Ala Pro Ser Met Ile Asp Glu Tyr Pro Ile Leu Ala Val Ala Ala Ala
                325                 330                 335

Phe Ala Glu Gly Ala Thr Val Met Asn Gly Leu Glu Glu Leu Arg Val
            340                 345                 350

Lys Glu Ser Asp Arg Leu Ser Ala Val Ala Asn Gly Leu Lys Leu Asn
        355                 360                 365

Gly Val Asp Cys Asp Glu Gly Glu Thr Ser Leu Val Val Arg Gly Arg
    370                 375                 380

Pro Asp Gly Lys Gly Leu Gly Asn Ala Ser Gly Ala Ala Val Ala Thr
385                 390                 395                 400

His Leu Asp His Arg Ile Ala Met Ser Phe Leu Val Met Gly Leu Val
                405                 410                 415

Ser Glu Asn Pro Val Thr Val Asp Asp Ala Thr Met Ile Ala Thr Ser
            420                 425                 430

Phe Pro Glu Phe Met Asp Leu Met Ala Gly Leu Gly Ala Lys Ile Glu
        435                 440                 445

Leu Ser Asp Thr Lys Ala Ala
    450                 455

<210>  11
<211>  1368
<212>  DNA
<213>  Artificial Sequence

<220>
<223>  EPSPS Nucleotide sequence

<400>  11
atgcttcacg gtgcaagcag ccggcccgca accgcccgca aatcctctgg cctttccgga        60

accgtccgca ttcccggcga caagtcgatc tcccaccggt ccttcatgtt cggcggtctc 120

gcgagcggtg aaacgcgcat caccggcctt ctggaaggcg aggacgtcat caatacgggc 180

aaggccatgc aggcgatggg cgcccgcatc cgtaaggaag gcgacacctg gatcatcgat 240

ggcgtcggca atggcggcct cctggcgcct gaggcgccgc tcgatttcgg caatgccgcc 300

acgggctgcc gcctgacgat gggcctcgtc ggggtctacg atttcgacag caccttcatc 360

ggcgacgcct cgctcacaaa gcgcccgatg ggccgcgtgt tgaacccgct gcgcgaaatg 420

ggcgtgcagg tgaaatcgga agacggtgac cgtcttcccg ttaccttgcg cgggccgaag 480

acgccgacgc cgatcaccta ccgcgtgccg atggcctccg cacaggtgaa gtccgccgtg 540

ctgctcgccg gcctcaacac gcccggcatc acgacggtca tcgagccgat catgacgcgc 600

gatcatacgg aaaagatgct gcagggcttt ggcgccaacc ttaccgtcga gacggatgcg 660

gacggcgtgc gcaccatccg cctggaaggc cgcggcaagc tcaccggcca agtcatcgac 720

gtgccgggcg acccgtcctc gacggccttc ccgctggttg cggccctgct tgttccgggc 780

tccgacgtca ccatcctcaa cgtgctgatg aaccccaccc gcaccggcct catcctgacg 840

ctgcaggaaa tgggcgccga catcgaagtc atcaacccgc gccttgccgg cggcgaagac 900

gtggcggacc tgcgcgttcg ctcctccacg ctgaagggcg tcacggtgcc ggaagaccgc 960

gcgccttcga tgatcgacga atatccgatt ctcgctgtcg ccgccgcctt cgcggaaggg 1020

gcgaccgtga tgaacggtct ggaagaactc cgcgtcaagg aaagcgaccg cctctcggcc 1080

gtcgccaatg gcctcaagct caatggcgtg gattgcgatg agggcgagac gtcgctcgtc 1140

gtgcgtggcc gccctgacgg caaggggctc ggcaacgcct cgggcgccgc cgtcgccacc 1200

catctcgatc accgcatcgc catgagcttc ctcgtcatgg gcctcgtgtc ggaaaaccct 1260

gtcacggtgg acgatgccac gatgatcgcc acgagcttcc cggagttcat ggacctgatg 1320

gccgggctgg gcgcgaagat cgaactctcc gatacgaagg ctgcctga 1368

<210> 12
<211> 1322
<212> DNA
<213> Arabidopsis thaliana

<400> 12
gtcgacctgc aggtcaacgg atcaggatat tcttgtttaa gatgttgaac tctatggagg 60

tttgtatgaa ctgatgatct aggaccggat aagttccctt cttcatagcg aacttattca 120

aagaatgttt tgtgtatcat tcttgttaca ttgttattaa tgaaaaaata ttattggtca 180

ttggactgaa cacgagtgtt aaatatggac caggccccaa ataagatcca ttgatatatg 240

aattaaataa caagaataaa tcgagtcacc aaaccacttg ccttttttaa cgagacttgt 300

tcaccaactt gatacaaaag tcattatcct atgcaaatca ataatcatac aaaaatatcc 360

aataacacta aaaaattaaa agaaatggat aatttcacaa tatgttatac gataaagaag 420

ttacttttcc aagaaattca ctgattttat aagcccactt gcattagata aatggcaaaa 480

aaaaacaaaa aggaaaagaa ataaagcacg aagaattcta gaaaatacga aatacgcttc 540

aatgcagtgg gacccacggt tcaattattg ccaattttca gctccaccgt atatttaaaa 600

aataaaacga taatgctaaa aaaatataaa tcgtaacgat cgttaaatct caacggctgg 660

atcttatgac gaccgttaga aattgtggtt gtcgacgagt cagtaataaa cggcgtcaaa 720

gtggttgcag ccggcacaca cgagtcgtgt ttatcaactc aaagcacaaa tacttttcct 780

caacctaaaa ataaggcaat tagccaaaaa caactttgcg tgtaaacaac gctcaataca 840

cgtgtcattt tattattagc tattgcttca ccgccttagc tttctcgtga cctagtcgtc 900

ctcgtctttt cttcttcttc ttctataaaa caatacccaa agcttcttct tcacaattca 960

gatttcaatt tctcaaaatc ttaaaaactt tctctcaatt ctctctaccg tgatcaaggt 1020

aaatttctgt gttccttatt ctctcaaaat cttcgatttt gttttcgttc gatcccaatt 1080

tcgtatatgt tctttggttt agattctgtt aatcttagat cgaagacgat tttctgggtt 1140

tgatcgttag atatcatctt aattctcgat tagggtttca taaatatcat ccgatttgtt 1200

caaataattt gagttttgtc gaataattac tcttcgattt gtgatttcta tctagatctg 1260

gtgttagttt ctagtttgtg cgatcgaatt tgtcgattaa tctgagtttt tctgattaac 1320

ag 1322

<210> 13
<211> 253
<212> DNA
<213> Agrobacterium tumefaciens

<400> 13
gatcgttcaa acatttggca ataaagtttc ttaagattga atcctgttgc cggtcttgcg 60

atgattatca tataatttct gttgaattac gttaagcatg taataattaa catgtaatgc 120

atgacgttat ttatgagatg ggtttttatg attagagtcc cgcaattata catttaatac 180

gcgatagaaa acaaaatata gcgcgcaaac taggataaat tatcgcgcgc ggtgtcatct 240

atgttactag atc 253

<210> 14
<211> 530
<212> DNA
<213> Cauliflower mosaic virus

<400> 14
ccatggagtc aaagattcaa atagaggacc taacagaact cgccgtaaag actggcgaac 60

agttcataca gagtctctta cgactcaatg acaagaagaa aatcttcgtc aacatggtgg 120

agcacgacac gcttgtctac tccaaaaata tcaaagatac agtctcagaa gaccaaaggg 180

caattgagac ttttcaacaa agggtaatat ccggaaacct cctcggattc cattgcccag 240

ctatctgtca ctttattgtg aagatagtgg aaaaggaagg tggctcctac aaatgccatc 300

attgcgataa aggaaaggcc atcgttgaag atgcctctgc cgacagtggt cccaaagatg 360

gacccccacc cacgaggagc atcgtggaaa aagaagacgt tccaaccacg tcttcaaagc 420

aagtggattg atgtgatatc tccactgacg taagggatga cgcacaatcc cactatcctt 480

cgcaagaccc ttcctctata taaggaagtt catttcattt ggagaggaca 530

<210> 15
<211> 552
<212> DNA
<213> Streptomyces viridochromogenes

<400> 15
atgtctccgg agaggagacc agttgagatt aggccagcta cagcagctga tatggccgcg 60

gtttgtgata tcgttaacca ttacattgag acgtctacag tgaactttag gacagagcca 120

caaacaccac aagagtggat tgatgatcta gagaggttgc aagatagata cccttggttg 180

gttgctgagg ttgagggtgt tgtggctggt attgcttacg ctgggccctg gaaggctagg 240

aacgcttacg attggacagt tgagagtact gtttacgtgt cacataggca tcaaaggttg 300

ggcctaggat ccacattgta cacacatttg cttaagtcta tggaggcgca aggttttaag 360

tctgtggttg ctgttatagg ccttccaaac gatccatctg ttaggttgca tgaggctttg 420

ggatacacag cccggggtac attgcgcgca gctggataca agcatggtgg atggcatgat 480

gttggttttt ggcaaaggga ttttgagttg ccagctcctc caaggccagt taggccagtt 540

acccagatct ga 552

<210> 16
<211> 195
<212> DNA
<213> Cauliflower mosaic virus

<400> 16
ctgaaatcac cagtctctct ctacaaatct atctctctct ataataatgt gtgagtagtt 60

cccagataag ggaattaggg ttcttatagg gtttcgctca tgtgttgagc atataagaaa 120

cccttagtat gtatttgtat ttgtaaaata cttctatcaa taaaatttct aattcctaaa 180

accaaaatcc agtgg 195

<210> 17
<211> 228
<212> DNA
<213> Arabidopsis thaliana

<400> 17
atggcgcaag ttagcagaat ctgcaatggt gtgcagaacc catctcttat ctccaatctc 60

tcgaaatcca gtcaacgcaa atctccctta tcggtttctc tgaagacgca gcagcatcca 120

cgagcttatc cgatttcgtc gtcgtgggga ttgaagaaga gtgggatgac gttaattggc 180

tctgagcttc gtcctcttaa ggtcatgtct tctgtttcca cggcgtgc 228

<210> 18
<211> 1534
<212> DNA
<213> Brassica napus

<400> 18
gattatgaca ttgctcgtgg aatgggacag ttatggtatt tttttgtaat aaattgtttc 60

cattgtcatg agattttgag gttaatctat gagacattga atcacttagc attagggatt 120

aagtagtcac aaatcgcatt caagaagctg aagaacacgt tatggtctaa tggttgtgtc 180

tctttattag aaaatgttgg tcagtagcta tatgcactgt ttctgtaaaa ccatgttggt 240

gttgtgttta tttcaagaca catgttgagt ccgttgattc agagcttttg tcttcgaaca 300

caatctagag agcaaatttg ggttcaattt ggatatcaat atgggttcga ttcagataga 360

acaataccct ttgatgtcgg gtttcgattt ggttgagatt catttttatc gggtttggtt 420

cgattttcga attcggttta ttcgccccct catagcatct acattctgca gattaatgta 480

caagttatgg aaaaaaaaat gtggttttcg aattcggttt agtagctaaa cgttgcttgc 540

agtgtagtta tgggaattat gaaacacgac cgaaggtatc aattagaaga acgggtcaac 600

gggtaagtat tgagaaatta ccggagggta aaaataaaca gtattctttt tttttcttaa 660

cgaccgacca aggttaaaaa aagaaaggag gacgagatac aggggcatga ctgtaattgt 720

acataagatc tgatctttaa accctaggtt tccttcgcat cagcaactat aaataattct 780

gagtgccact cttcttcatt cctagatctt tcgccttatc gctttagctg aggtaagcct 840

ttctatacgc atagacgctc tcttttctct tctctcgatc ttcgttgaaa cggtcctcga 900

tacgcatagg atcggttaga atcgttaatc tatcgtctta gatcttcttg attgttgaat 960

tgagcttcta ggatgtattg tatcatgtga tggatagttg attggatctc tttgagtgaa 1020

ctagctagct ttcgatgcgt gtgatttcag tataacagga tccgatgaat tatagctcgc 1080

ttacaattaa tctctgcaga tttattgttt aatcttggat ttgatgctcg ttgttgatag 1140

aggatcgttt atagaactta ttgattctgg aattgagctt gtgtgatgta ttgtatcatg 1200

tgatcgatag ctgatggatc tatttgagtg aactagcgta cgatcttaag atgagtgtgt 1260

attgtgaact gatgattcga gatcagcaaa acaagatctg atgatatctt cgtcttgtat 1320

gcatcttgaa tttcatgatt ttttattaat tatagctcgc ttagctcaaa ggatagagca 1380

ccacaaaatt ttattgtggt agaaatcggt tcgattccga tagcagctta ctgtgatgaa 1440

tgattttgag atttggtatt tgatatatgt ctactgtgtt gaatgatcgt ttatgcattg 1500

tttaatcgct gcagatttgc attgacaagt agcc 1534

<210> 19
<211> 643
<212> DNA
<213> Pisum sativum

<400> 19
agctttcgtt cgtatcatcg gtttcgacaa cgttcgtcaa gttcaatgca tcagtttcat 60

tgcgcacaca ccagaatcct actgagtttg agtattatgg cattgggaaa actgtttttc 120

ttgtaccatt tgttgtgctt gtaatttact gtgtttttta ttcggttttc gctatcgaac 180

tgtgaaatgg aaatggatgg agaagagtta atgaatgata tggtcctttt gttcattctc 240

aaattaatat tatttgtttt ttctcttatt tgttgtgtgt tgaatttgaa attataagag 300

atatgcaaac attttgtttt gagtaaaaat gtgtcaaatc gtggcctcta atgaccgaag 360

ttaatatgag gagtaaaaca cttgtagttg taccattatg cttattcact aggcaacaaa 420

tatattttca gacctagaaa agctgcaaat gttactgaat acaagtatgt cctcttgtgt 480

tttagacatt tatgaacttt cctttatgta attttccaga atccttgtca gattctaatc 540

attgctttat aattatagtt atactcatgg atttgtagtt gagtatgaaa atatttttta 600

atgcatttta tgacttgcca attgattgac aacatgcatc aat 643

<210> 20
<211> 25
<212> DNA
<213> Artificial Sequence

<220>
<223> Primer 1

<400> 20
ctggaaggcg aggacgtcat caata 25

<210> 21
<211> 22
<212> DNA
<213> Artificial Sequence

<220>
<223> Primer 2

<400> 21
tggcggcatt gccgaaatcg ag 22

<210> 22
<211> 28
<212> DNA
<213> Artificial Sequence

<220>

<223>  Probe 1

<400>  22
atgcaggcga tgggcgcccg catccgta                                          28


<210>  23
<211>  1992
<212>  DNA
<213>  Zea mays

<400>  23
ctgcagtgca gcgtgacccg gtcgtgcccc tctctagaga taatgagcat tgcatgtcta      60

agttataaaa aattaccaca tatttttttt gtcacacttg tttgaagtgc agtttatcta     120

tctttataca tatatttaaa ctttactcta cgaataatat aatctatagt actacaataa     180

tatcagtgtt ttagagaatc atataaatga acagttagac atggtctaaa ggacaattga     240

gtattttgac aacaggactc tacagtttta tctttttagt gtgcatgtgt tctccttttt     300

ttttgcaaat agcttcacct atataatact tcatccattt tattagtaca tccatttagg     360

gtttagggtt aatggttttt atagactaat ttttttagta catctatttt attctatttt     420

agcctctaaa ttaagaaaac taaaactcta ttttagtttt tttatttaat aatttagata     480

taaaatagaa taaaataaag tgactaaaaa ttaaacaaat accctttaag aaattaaaaa     540

aactaaggaa acatttttct tgtttcgagt agataatgcc agcctgttaa acgccgtcga     600

cgagtctaac ggacaccaac cagcgaacca gcagcgtcgc gtcgggccaa gcgaagcaga     660

cggcacggca tctctgtcgc tgcctctgga cccctctcga gagttccgct ccaccgttgg     720

acttgctccg ctgtcggcat ccagaaattg cgtggcggag cggcagacgt gagccggcac     780

ggcaggcggc ctcctcctcc tctcacggca cggcagctac gggggattcc tttcccaccg     840

ctccttcgct ttcccttcct cgcccgccgt aataaataga caccccctcc acaccctctt     900

tccccaacct cgtgttgttc ggagcgcaca cacacacaac cagatctccc ccaaatccac     960

ccgtcggcac ctccgcttca aggtacgccg ctcgtcctcc cccccccccc ctctctacct    1020

tctctagatc ggcgttccgg tccatggtta gggcccggta gttctacttc tgttcatgtt    1080

tgtgttagat ccgtgtttgt gttagatccg tgctgctagc gttcgtacac ggatgcgacc    1140

tgtacgtcag acacgttctg attgctaact tgccagtgtt tctctttggg gaatcctggg    1200

atggctctag ccgttccgca gacgggatcg atttcatgat tttttttgtt tcgttgcata    1260

gggtttggtt tgcccttttc ctttatttca atatatgccg tgcacttgtt tgtcgggtca    1320

tcttttcatg cttttttttg tcttggttgt gatgatgtgg tctggttggg cggtcgttct    1380

agatcggagt agaattctgt ttcaaactac ctggtggatt tattaatttt ggatctgtat    1440

gtgtgtgcca tacatattca tagttacgaa ttgaagatga tggatggaaa tatcgatcta    1500

ggataggtat acatgttgat gcgggtttta ctgatgcata tacagagatg ctttttgttc    1560

gcttggttgt gatgatgtgg tgtggttggg cggtcgttca ttcgttctag atcggagtag 1620

aatactgttt caaactacct ggtgtattta ttaattttgg aactgtatgt gtgtgtcata 1680

catcttcata gttacgagtt taagatggat ggaaatatcg atctaggata ggtatacatg 1740

ttgatgtggg ttttactgat gcatatacat gatggcatat gcagcatcta ttcatatgct 1800

ctaaccttga gtacctatct attataataa acaagtatgt tttataatta ttttgatctt 1860

gatatacttg gatgatggca tatgcagcag ctatatgtgg atttttttag ccctgccttc 1920

atacgctatt tatttgcttg gtactgtttc ttttgtcgat gctcaccctg ttgtttggtg 1980

ttacttctgc ag 1992

<210> 24
<211> 1176
<212> DNA
<213> Escherichia coli

<400> 24
atgcaaaaac tcattaactc agtgcaaaac tatgcctggg gcagcaaaac ggcgttgact 60

gaactttatg gtatggaaaa tccgtccagc cagccgatgg ccgagctgtg gatgggcgca 120

catccgaaaa gcagttcacg agtgcagaat gccgccggag atatcgtttc actgcgtgat 180

gtgattgaga gtgataaatc gactctgctc ggagaggccg ttgccaaacg ctttggcgaa 240

ctgcctttcc tgttcaaagt attatgcgca gcacagccac tctccattca ggttcatcca 300

aacaaacaca attctgaaat cggttttgcc aaagaaaatg ccgcaggtat cccgatggat 360

gccgccgagc gtaactataa agatcctaac cacaagccgg agctggtttt tgcgctgacg 420

cctttccttg cgatgaacgc gtttcgtgaa ttttccgaga ttgtctccct actccagccg 480

gtcgcaggtg cacatccggc gattgctcac tttttacaac agcctgatgc cgaacgttta 540

agcgaactgt tcgccagcct gttgaatatg cagggtgaag aaaaatcccg cgcgctggcg 600

attttaaaat cggccctcga tagccagcag ggtgaaccgt ggcaaacgat tcgtttaatt 660

tctgaatttt acccggaaga cagcggtctg ttctccccgc tattgctgaa tgtggtgaaa 720

ttgaaccctg gcgaagcgat gttcctgttc gctgaaacac cgcacgctta cctgcaaggc 780

gtggcgctgg aagtgatggc aaactccgat aacgtgctgc gtgcgggtct gacgcctaaa 840

tacattgata ttccggaact ggttgccaat gtgaaattcg aagccaaacc ggctaaccag 900

ttgttgaccc agccggtgaa acaaggtgca gaactggact tcccgattcc agtggatgat 960

tttgccttct cgctgcatga ccttagtgat aaagaaacca ccattagcca gcagagtgcc 1020

gccattttgt tctgcgtcga aggcgatgca acgttgtgga aaggttctca gcagttacag 1080

cttaaaccgg gtgaatcagc gtttattgcc gccaacgaat caccggtgac tgtcaaaggc 1140

cacggccgtt tagcgcgtgt ttacaacaag ctgtaa 1176

<210>  25
<211>  29
<212>  DNA
<213>  Artificial Sequence

<220>
<223>  Òýîï3

<400>  25
gctgtaagag cttactgaaa aaattaaca                                         29


<210>  26
<211>  18
<212>  DNA
<213>  Artificial Sequence

<220>
<223>  Primer 4

<400>  26
cgatctgcag gtcgacgg                                                     18


<210>  27
<211>  27
<212>  DNA
<213>  Artificial Sequence

<220>
<223>  Probe 2

<400>  27
tctcttgcta agctgggagc tcgatcc                                           27